# EUROPEAN PATENT SPECIFICATION

(11) **EP 3 319 936 B1**
(45) Date of publication and mention of the grant of the patent: **17.12.2025**
(21) Application number: 15773571.3
(22) Date of filing: 12.07.2015
(51) Int. Cl.: C07C 243/28, C07D 207/452, C07D 403/14, C07C 311/55, C07D 213/71, C07C 275/50, C07K 5/065, A61K 45/06, A61P 31/00, A61P 35/00

(54) **BRIDGE LINKERS FOR CONJUGATION OF CELL-BINDING MOLECULES**
BRÜCKENLINKER ZUR KONJUGATION VON ZELLBINDENDEN MOLEKÜLEN
LIEUX DE PONTAGE POUR LA CONJUGAISON DES MOLÉCULES DE LIAISON CELLULAIRE

(43) Date of publication of application: 16.05.2018
(60) Divisional application: 25214104.9
(73) Proprietor: Hangzhou Dac Biotech Co., Ltd., Hangzhou City, Zhejiang Province 310018 (CN)
(72) Inventor: ZHAO, Robert Yongxin, Lexington, MA 02421 (US)
(74) Representative: Haseltine Lake Kempner LLP
(86) International application number: PCT/IB2015/055264
(87) International publication number: WO 2015/151081

(56) References cited:
- WO-A1-2014/080251
- WO-A1-2014/114207
- WO-A1-2015/028850
- US-A- 5 316 888
- DLRR, S. ET AL.: "Neue Synthesen und Reaktionen ungesåttigter Heterotitanacyclen", JOURNAL OF ORGANOMETALLIC CHEMISTRY, vol. 458, no. 1-2, 5 October 1993 (1993-10-05), pages 89 - 96, XP055451777, ISSN: 0022-328X
- BARTON, D. H. R. ET AL.: "An unusual reaction between the esters of N-hydroxy-2- thiopyridone and activated azo compounds", TETRAHEDRON, vol. 44, no. 24, 31 December 1988 (1988-12-31), pages 7385 - 7392, XP026596495, ISSN: 0040-4020
- SUNDERMEYER, W. ET AL.: "Perfluoralkyl- und Fluorcarbonyl-Hydrazine", JOURNAL OF FLUORINE CHEMISTRY, vol. 34, no. 2, 31 December 1986 (1986-12-31), pages 251 - 254, XP026730936, ISSN: 0022-1139
- SPRENGER, G. H. ET AL.: "Photochemistry of perfluoroalkyl and perfluoroacyl N- chloramines. Reactions of N-chloramines and N-chlorimines in the presence of mercury", JOURNAL OF THE AMERICAN CHEMICAL SOCIETY, vol. 96, no. 6, 20 March 1974 (1974-03-20), pages 1770 - 1775, XP055451778, ISSN: 0002-7863

## Description

### FIELD OF THE INVENTION

The present disclosure relates to cell-binding molecule-linker-drug conjugates. The present disclosure also relates to the preparation of novel linkers used for the specific conjugation of compounds, in particular, cytotoxic agents to a cell-binding molecule, through bridge linking pairs of thiols on a cell-binding molecule. The present disclosure also relates to methods of making cell-binding agent-drug (cytotoxic agent) conjugates in a specific manner comprising either modification of drugs with these linkers first, followed by reaction with prepared cell-binding agents; or modification of cell-binding agents with these linkers first, followed by reaction with drugs.

### BACKGROUND OF THE INVENTION

Traditional chemotherapy is often accompanied by systemic toxicity to the patient. Monoclonal antibody which can offer an alternative tumor-selective treatment approach via discriminating antigens on cancer cells, is normally not sufficiently potent to be therapeutically active by itself. Antibody-drug conjugates (ADCs), which by their name have the exquisite targeting ability of antibodies in combination with the cytotoxic action of anticancer agents, enable to target and deliver drugs to cancer cells leaving normal cells largely unaffected, thus improving the therapeutic index of chemotherapeutic agents. since US FDA approvals of Adcetris (brentuximab vedotin) in 2011 and Kadcyla (ado-trastuzumab emtansine) in 2013, the applications of antibody-drug conjugate (ADC) as a promise targeted treatment of cancers have been exploded and almost every major pharmaceutical and biotech company has adopted this approach (Chari, R. et al, Angew. Chem., Int. Ed. 2014, 53, 3796-3827; Sievers, E. L. et al. Annu Rev Med. 2013, 64, 15-29; Mehr-ling, T. Future Oncol, 2015, 11, 549). Antibody-drug conjugates are now a drug class with a robust pipeline and a flurry of deal-making among pharmaceutical companies. With more than 50 ADCs currently in various stages of clinical trials according to www.clinictrails.gov, and even more in preclinical and/or are ready to enter first-in-human trials, the market is rife with eager anticipation of more ADC drugs to be approved by regulators.

The first-generation ADCs, including Kadcyla and Adcetris, are produced through nonselective conjugation of native lysine amines or interchain cysteine thiols on an antibody respectively to a cytotoxic drug. Since there are over 50 surface-exposed lysines and 8 hinge cysteine residues in IgG1 antibodies, this nonselective conjugation results in randomly cross-linkage of cytotoxic drugs to practically all areas of the antibody molecule, particularly having a diverse population of ADCs with a wide distribution of drugs per antibody (DAR) (Wang, L., et al. 2005 Protein Sci. 14, 2436; Hamblett, K. J., et al. 2004 Clin. Cancer Res. 10, 7063). Thus some of the undesired ADC subpopulation could lead to shorter circulation half-life, lower efficacy, potentially increased off-target toxicity and a wide range of in vivo pharmacokinetic (PK) properties (Hamblett, K. J. et al, Clin. Cancer Res. 2004, 10, 7063-7070; Adem, Y. T. et al, Bioconjugate Chem. 2014, 25, 656-664 ; Boylan, N. J. Bioconjugate Chem., 2013, 24, 1008-1016; Strop, P., et al 2013 Chem. Biol. 20, 161-167). In addition, with this classical conjugation, the batch-to-batch consistency in ADC production can be challenging and may require diligent manufacturing capabilities (Wakankar, A. mAbs, 2011, 3, 161-172).

Therefore, biotechnology companies and academic institutions are intensely focusing on establishing novel reliable methods for site-specific ADC conjugation. So far, there are several approaches developed in recent years for site selective ADC preparation (Pan-owski, S, 2014, mAbs 6, 34). They include incorporation of unpaired cysteines, e.g. engineered reactive cysteine residues, called THIOMAB from Genentech (Junutula, J. R., et al 2010 Clin. Cancer Res. 16, 4769; Junutula, J. R., et al 2008 Nat Biotechnol. 26, 925-32; US Patents 8,309,300; 7,855,275; 7,521,541; 7,723,485, WO2008/141044), genetically introduced glutamine tag with Streptoverticillium mobaraense transglutaminase (mTG) (Strop, P., Bioconjugate Chem., 2014, 25, 855-862; Strop, P., et al., 2013, Chem. Biol. 20, 161-167; US Patent 8,871,908 for Rinat-Pfizer) or with Microbial transglutaminase (MTGase) (Dennler, P., et al, 2014, Bioconjug. Chem. 25, 569-578. US pat appl 20130189287 for Innate Pharma; US Pat 7,893,019 for Bio-Ker S.r.l. (IT)), incorporation of thiolfucose (Dennler, P., et al, 2014 Bioconjugate Chemistry 25, 569; Okeley, N. M., et al 2013 Bioconjugate Chem. 24, 1650), incorporation of unnatural amino acids through mutagenesis (Axup, J.Y., et al., 2012, Proc. Natl. Acad. Sci. 109, 16101-16106; Zimmer-man, E.S., et al., 2014, Bioconjug. Chem. 25, 351-361; Wu, P., et al, 2009 Proc. Natl. Acad. Sci. 106, 3000-3005; Rabuka, D., et al, 2012 Nat. Protoc. 7, 1052-67; US Pattent 8,778,631 and US Pat Appl. 20100184135, WO2010/081110 for Sutro Biopharma; WO2006/069246, 2007/059312, US Patents 7,332,571, 7,696,312, and 7,638,299 for Am-brx; WO2007/130453, US patents 7,632,492 and 7,829,659 for Allozyne), Incorporation of selenocysteine into antibodies (Hofer, T., et al 2009, Biochemistry 48, 12047-12057; US Patent 8,916,159 for US National Cancer Institute), Convertion of cysteines located in the CXPXR consensus sequence to formylglycine (FGly) with formylglycine generating enzyme (FGE) (Drake, P.M., et al., 2014, Bioconjug. Chem. 25, 1331-1341. Carrico; Isaac S. et al 7,985,783; 8,097,701; 8,349,910, and US Pat Appl 20140141025, 20100210543 for Redwood Bioscience), and through glycoengineeringly introduction of sialic acid with the use of galactosyl- and sialytransferases (Zhou, Q., et al 2014, Bioconjug. Chem., 25, 510-520, US Pat Appl 20140294867 for Sanofi-Genzyme). These above methods have produced nearly homogeneous product profiles, but they are required antibody-engineering processes and reoptimization of cell culture conditions. Moreover, expression yields for genetic encoding of an unnatural amino acid were typically not promisingly high enough (Tian, F., et al, 2014, Proc. Natl. Acad. Sci. U. S. A. 111, 1766-71) which has a significant impact on the cost of goods of the ADC. In addition, it has been known that ADCs obtained by conjugation to cysteine side chains often display limited stability in circulation, leading to premature disconnection of the cytotoxic payload before the tumor site is reached (Junutula, J. R., et al 2008, Nat. Biotechnol. 26, 925-32).

The disulfide bond structures of the four subclasses of IgG antibodies were known in the 1960s (Milstein C. Biochem J 1966, 101:338 - 351; Pink JR, Milstein C. Nature 1967, 214:92-94; Frangione B, Milstein C. Nature 1967, 216:939 - 941; Pink JR, Milstein C. Nature 1967, 216:941 -942; Frangione B, et al. Biochem J 1968, 106,15 - 21; Frangione B, Milstein C. J Mol Biol 1968; 33:893 - 906; Edelman GM, et al. Proc Natl Acad Sci USA 1969; 63:78 -85; Frangione B, et al. Nature 196, 221:145 -148, Spiegelberg, H. L. et al Biochemistry, 1975, 10, 2157-63 ). Disulfide bond structure is critical for the structure, stability, and biological functions of IgG molecules. Among the four subclasses of IgG antibodies, IgG₁, IgG₂, IgG₃ and IgG₄, each IgG contains a total of 12 intra-chain disulfide bonds; each disulfide bond is associated with an individual IgG domain. The two heavy chains are connected in the hinge region by a variable number of disulfide bonds: 2 for IgG₁ and IgG₄, 4 for IgG₂ and 11 for IgG₃. The light chain of the IgG₁ is connected to the heavy chain by a disulfide bond between the last cysteine residue of the light chain and the fifth cysteine residue of the heavy chain. But, for IgG₂, IgG₃ and IgG₄, the light chain is linked to the heavy chain by a disulfide bond between the last cysteine residue of the light chain and the third cysteine residue of the heavy chain (Liu, H. and May, K., 2012, mAbs 4, 17-23). On the ranks of the susceptibility of disulfide bonds in human IgG1 antibodies by experimental reduction, differential alkylation, and LC-MS analysis (Liu, H, et al Anal. Chem., 2010, 82, 5219-5226), inter chain disulfide bonds are more susceptible to reduction than intra chain disulfide bonds, and the disulfide bonds between the light chain and heavy chain were more susceptible than disulfide bonds between the two heavy chains. The upper disulfide bond of the two inter heavy chain disulfide bonds was more susceptible than the lower one. Furthermore, disulfide bonds in the CH2 domain were the most susceptible to reduction. Disulfide bonds in VL, CL, VH, and CH1 domains had similar and moderate susceptibility, while disulfide bonds in the CH3 domain were the least susceptible to reduction (Liu, H, et al Anal. Chem., 2010, 82, 5219-5226).

Based on the more susceptibility of inter chain disulfide bonds in human IgG1 antibodies, several institutions and companies adopted the chemically specific conjugation strategy through rebridging reduced interchain disulfide bonds of a native antibody, such as, using bromo or dibromo-maleimides, called next generation maleimides (NGMs) (Schumacher, F.F., et al 2014, Org. Biomol. Chem. 12, 7261-7269; UCL Cancer Inst.), applying bis-alkylating reagents via a three-carbon bridge (Badescu, G., et al., 2014, Bioconjug. Chem. 25, 1124-1136., WO2013/190272, WO2014/064424 for PolyTherics Ltd), with di-substituted heteroaryl bridge (US Pat Appl. 2015/0105539 for Concortis Biosystem), or through di-maleimide as a bridge (WO2014/114207). We have also used bromo maleimide and dibromomaleimide linkers to conjugate both drugs and antibodies for a quite while (WO2014/009774, PCT/IB2012/053554). However, these above bridge linkers were designed in the way to conjugate only one cytotoxic agent to a pair of disulfide bonds, and therefore at most of time they only produced ADCs at DAR less than 2 (drugs per antibody), due to limited numbers (about only two pairs) of reduced disulfide bonds are more accessible for conjugation.

As one of the major issues for ADCs is the limited numbers or amount of cytotoxic compounds that ultimately reaches the tumor, and the favorable DAR over 3 is much important factor for improvement of ADC therapeutical index (Epenetos, A. A. et al, Cancer Res., 1986, 46, 3183-3191; Chari, R. V. Acc. Chem. Res., 2008, 41, 98-107, Zhao, R. Y. et al, 2011, J. Med. Chem. 54, 3606-3623), we therefore disclose novel disulfide bridge linkers of this invention that are able to conjugate two or more drugs per linker for achieving higher DARs (≥4) via re-bridging the susceptible a pair of thiols on the inter chain of IgG antibody that are reduced by TCEP and DTT. And the other reduced disulfide bonds that are inaccessibly reached by the bridge linkers, due to the big size of bridge linker containing two cytotoxic agents, can be recoupled (regenerated) by an oxide, e.g. dehy-droascorbic acid (DHAA) or Cu (II), at the end of conjugation. In a word, these bridge linkers of the invention can make homogeneous production of specific ADCs in a simple way.

WO2014080251 discloses cell binding agent-drug conjugates comprising hydrophilic linkers, and methods of using such linkers; WO2015028850 provides cytotoxic agents pyrrolo[2,1,c][1,4]benzodiazepine (PBD) derivatives, their conjugates with a cell binding agent, and their preparation and therapeutic uses in the targeted treatment of cancers, autoimmune disorders and infectious diseases.

### SUMMARY OF THE INVENTION

The present invention provides a product that is:
(A) a cell-binding molecule-linker-drug conjugate represented as 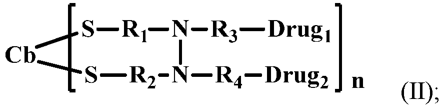 or
(B) a compound of formula (IV) 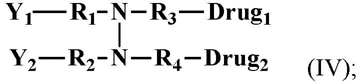 wherein:
   Y₁ and Y₂ are selected from the structures displayed below: 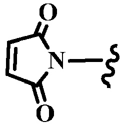 maleimide; 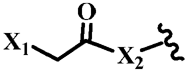 haloacetyl; and 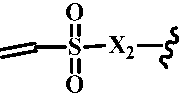 ethenesulfonyl; wherein X₁ is F, Cl, Br, I or Lv; X₂ is O, NH, N(R₁), or CH₂; Lv is selected from nitrophenol; N-hydroxysuccinimide (NHS); phenol; dinitrophenol; pentafluorophenol; tetrafluorophenol; difluorophenol; monofluorophenol; pentachlorophenol; triflate; imidazole; dichlorophenol; tetrachlorophenol; 1-hydroxybenzotriazole; tosylate; mesylate; 2-ethyl-5-phenylisoxazolium-3'-sulfonate, or anhydrides; or an intermediate molecule generated with a condensation reagent for peptide coupling reactions or for Mitsobonu reactions,
      R₁, R₂, R₃ and R₄ are the same or different, and are absent, linear alkyl having from 1-8 carbon atoms, branched or cyclic alkyl having from 3 to 8 carbon atoms, linear, branched or cyclic alkenyl or alkynyl, or 1~8 carbon atoms of esters, ether, amide, or polyethyleneoxy unit of formula (OCH₂CH₂)ₚ, wherein p is an integer from 0 to 1000, or combination thereof; and
      additionally, R₁, R₂, R₃ and R₄ are respectively a chain of atoms selected from C, N, O, S, Si, and P, which covalently connects to Y₁ or Y₂ and Z₁ or Z₂, wherein the atoms used in forming the R₁ R₂, R₃ and R₄ may be combined in all chemically relevant ways, such as forming alkylate, alkylene, alkenylene, and alkynylene, ethers, polyoxyalkylene, esters, amines, imines, polyamines, hydrazines, hydrazones, amides, ureas, semicarbazides, carbazides, alkoxyamines, alkoxylamines, urethanes, amino acids, peptides, acyloxylamines, hydroxamic acids, or a combination thereof;
      Cb represents a cell-binding agent;
      Drug₁ and Drug₂ represent the same or different drugs/cytotoxic agents, linked to the cell-binding agent via the bridge linker through analkylene, alkenylene, alkynylene, ether, polyoxyalkylene, ester, amine, imine, polyamine, hydrazine, hydrazone, amide, urea, semicarbazide, carbazide, alkoxyamine, urethanes, amino acid, peptide, acyloxylamine, hydroxamic acid, disulfide, thioether, thioester, carbamate, carbonate, heterocyclic ring, heteroaromatic, or alkoxime bond; and
   n is an integer from 1 to 20

The present disclosure provides linkers containing a hydrazine to link two drugs to a cell-binding agent (e.g., an antibody). The preferred formula of the cell-binding molecule-linker-drug conjugates can be represented as: 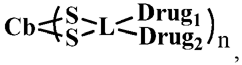 wherein Cb is a cell-binding agent, L is a linker containing a hydrazine group, Drug1 and Drug2 are a drug molecule, n is an integer from 1 to 20, and two S (sulfur) elements from Cb bridgely link to L, which covalently connects two or more drugs (per bridge linker L). The advantages in applying the linker in the cell molecule-drug conjugate are: a). Retaining the stability of the conjugates by covalently cross-linking (re-bridging) the pairs of reduced di-sulfur bonds of the cell-binding agents, particularly of antibodies; b). Enabling conjugation of the cytotoxic agents/drugs to specific sites of a cell-binding molecule, e.g. the inter chain sites of IgG antibodies, resulting in homogeneous production of ADC.

Described herein is a linker represented by Formula (I)

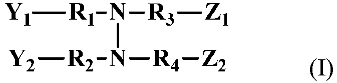

Wherein:
Y₁ and Y₂ are the same or different a functional group that enables reaction with a pair of sulfur atoms of a cell-binding agent; Y₁ andY₂ can react to a pair of sulfur atoms to form disulfide, thioether, or thioester bonds. The function groups for Y₁ and Y₂ aremaleimide, haloacetyl or ethenesulfonyl (i.e., as defined herein).
Z₁ and Z₂ are the same or different a function group that enables to react with a cytotoxic drug. The functional group Z₁ or Z₂ can react to a cytotoxic drug to form a disulfide, ether, ester, thioether, thioester, peptide, hydrazone, carbamate, carbonate, amine (secondary, tertiary, or quaternary), imine, cycloheteroalkyl, heteroaromatic, alkoxime or amide bond;
R₁ R₂, R₃ and R₄ are the same or different, and are absent, linear alkyl having from 1-8 carbon atoms, branched or cyclic alkyl having from 3 to 8 carbon atoms, linear, branched or cyclic alkenyl or alkynyl, or 1~8 carbon atoms of esters, ether, amide, or polyethyleneoxy unit of formula (OCH₂CH₂)ₚ, wherein p is an integer from 0 to about 1000, or combination thereof.

Additionally R₁, R₂, R₃ and R₄ are respectively a chain of atoms selected from C, N, O, S, Si, and P, preferably having 0~500 atoms, which covalently connects to Y₁ or Y₂ and Z₁ or Z₂. The atoms used in forming the R₁, R₂, R₃ and R₄ may be combined in all chemically relevant ways, such as forming alkylate, alkylene, alkenylene, and alkynylene, ethers, polyoxyalkylene, esters, amines, imines, polyamines, hydrazines, hydrazones, amides, ureas, semicarbazides, carbazides, alkoxyamines, alkoxylamines, urethanes, amino acids, peptides, acyloxylamines, hydroxamic acids, or combination thereof.

In an aspect, this invention provides a cell-binding agent-drug conjugate of Formula (II), in which the cell-binding agent, Cb, and the drug, Drug1 and Drug2, have reacted at the ends of the bridge linker: 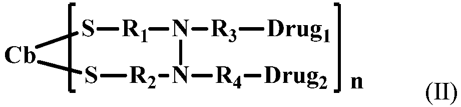 wherein:
Cb represents a cell-binding agent, preferred an antibody;

Inside the bracket (parentheses) are the linker-drug components that are conjugated to a cell-binding molecule via a pair of thiol atoms. The conjugatable thiol atoms can generally be generated from reduction of pairs of disulfide bonds on the cell-binding molecule with TCEP or DTT reagents.

Drug₁ and Drug₂ represent the same or different cytotoxic agents, which linked to the cell-binding agent via the bridge linker by a disulfide, thioether, thioester, peptide, hydrazone, ether, ester, carbamate, carbonate, cycloheteroalkyane, heteroaromatic, alkoxime, amide, alkylene, alkenylene, alkynylene or aromatic bond;
n is 1 ~ 20; R₁ R₂, R₃ and R₄ are described the same previously in Formula (I).

Also disclosed herein is a modified cell-binding agent of Formula (III), in which the cell-binding agent, Cb, has reacted with the bridge linker, which has Z₁ and Z₂, the function groups capable of reacting with a drug:

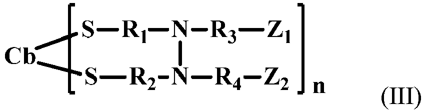

Wherein Cb, Z₁, Z₂, R₁, R₂, R₃ and R₄ are defined the same as in Formula (I) and (II).

In a further aspect, the present invention provides a modified drug of Formula (IV), in which the drug, Drug₁ and Drug₂, have reacted with the linker of Formula (I), which still has Y₁ and Y₂ group capable of reacting with a pair of sulfur atoms of the cell-binding agent:

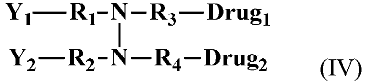

Wherein Y₁, Y₂, Drug₁, Drug₂, R₁, R₂ R₃ and R₄ are defined the same as in Formula (I) and (II).

The present disclosure further relates to a method of making a cell-binding molecule-drug conjugate of Formula (II), wherein the drugs, Drug₁ and Drug₂ are linked to a cell-binding agent via the bridge linker.

The present disclosure also relates to a method of making a modified cell-binding molecule of Formula (III), wherein the cell-binding molecule is reacted with the bridge linker of Formula (I).

The present disclosure also relates to a method of making a modified drug of formula (IV), wherein the drug is reacted with the bridge linker of Formula (I).

### BRIEF DESCRIPTION OF THE DRAWINGS

Figure 1 shows the synthesis of a bridge linker and the application of this linker in the conjugation of an antibody with drugs.
Figure 2 shows the synthesis of a bridge linker containing polyethylene glycols and the application of this linker in the conjugation of drugs to an antibody via amide and thioether linkage.
Figure 3 shows the synthesis of a bridge linker containing polyethylene glycols and the application of this linker in the conjugation of drugs to an antibody via amide and thioether linkage.
Figure 4 shows the synthesis of a bridge linker and the application of this linker in the conjugation of drugs to an antibody via amide and thioether linkage.
Figure 5 shows the synthesis of a bridge linker containing peptides and the application of this linker in the conjugation of drugs to an antibody via amide and thioether linkage.
Figure 6 shows the synthesis of a bridge linker containing polyethylene glycols and the application of this linker in the conjugation of drugs to an antibody via amide and thioether linkage.
Figure 7 shows the synthesis of bridge linkers (one containing polyethylene glycols), and the application in the conjugation of two or four drugs per linker to an antibody via amide, oxime or hydrazone linkage to drugs, and thioether linkage to antibodies.
Figure 8 shows the synthesis of an antibody-maytansinoid conjugate and an antibody-MMAF conjugate via the bridge-linker.
Figure 9 shows the synthesis an antibody-maytansinoid conjugate via the bridge-linker, wherein four maytansinoids are linked to one bridge linker.
Figure 10 shows the synthesis of the conjugates of cell-binding molecule-tubulysin analogs via the bridge-linker.
Figure 11 shows the synthesis of a conjugate containing four tubulysin analogs per bridge linker, and the synthesis of a conjugate of antibody-maytansinoids containing hinder disulfide linkage.

### DETAILED DESCRIPTION OF THE INVENTION

### DEFINITIONS

"Alkyl" refers to an aliphatic hydrocarbon group which may be straight or branched having 1 to 8 carbon atoms in the chain. "Branched" means that one or more lower C numbers of alkyl groups such as methyl, ethyl or propyl are attached to a linear alkyl chain. Exemplary alkyl groups include methyl, ethyl, n-propyl, i-propyl, n-butyl, t-butyl, n-pentyl, 3-pentyl, octyl, nonyl, decyl, cyclopentyl, cyclohexyl, 2,2-dimethylbutyl, 2,3-dimethylbutyl, 2,2-dimethylpentyl, 2,3-dimethylpentyl, 3,3-dimethylpentyl, 2,3,4-trimethylpentyl, 3-methyl-hexyl, 2,2-dimethylhexyl, 2,4-dimethylhexyl, 2,5-dimethylhexyl, 3,5-dimethylhexyl, 2,4-dimethylpentyl, 2-methylheptyl, 3-methylheptyl, n-heptyl, isoheptyl, n-octyl, and isooctyl. A C₁-C₈ alkyl group can be unsubstituted or substituted with one or more groups including, but not limited to, -C₁-C₈ alkyl,-O-(C₁-C₈ alkyl), -aryl, -C(O)R', - OC(O)R', -C(O)OR', -C(O)NH₂, -C(O)NHR', -C(O)N(R')₂, -NHC(O)R', -SR', -S(O)₂R', - S(O)R', -OH, -halogen, -N₃, -NH₂, -NH(R'), -N(R') ₂ and -CN; where each R' is independently selected from -C₁-C₈ alkyl and aryl."Halogen" refers to fluorine, chlorine, bromine or iodine atom; preferably fluorine and chlorine atom.

"Heteroalkyl" refers to C₂-C₈ alkyl in which one to four carbon atoms are independently replaced with a heteroatom from the group consisting of O, S and N.

"Carbocycle" refers to a saturated or unsaturated ring having 3 to 8 carbon atoms as a monocycle or 7 to 13 carbon atoms as a bicycle. Monocyclic carbocycles have 3 to 6 ring atoms, more typically 5 or 6 ring atoms. Bicyclic carbocycles have 7 to 12 ring atoms, arranged as a bicycle [4,5], [5,5], [5,6] or [6,6] system, or 9 or 10 ring atoms arranged as a bicycle [5,6] or [6,6] system. Representative C₃-C₈ carbocycles include, but are not limited to, -cyclopropyl, -cyclobutyl, -cyclopentyl, -cyclopentadienyl, -cyclohexyl, -cyclohexenyl, -1,3-cyclohexadienyl, -1,4-cyclohexadienyl, -cycloheptyl, -1,3-cycloheptadienyl, -1,3,5-cycloheptatrienyl, -cyclooctyl, and -cyclooctadienyl.

A "C₃-C₈ carbocycle" refers to a 3-, 4-, 5-, 6-, 7- or 8-membered saturated or unsaturated nonaromatic carbocyclic ring. A C₃-C₈ carbocycle group can be unsubstituted or substituted with one or more groups including, but not limited to, -C₁-C₈ alkyl,-O-(C₁-C₈ alkyl), -aryl, -C(O)R', -OC(O)R', -C(O)OR', -C(O)NH₂, -C(O)NHR', -C(O)N(R')₂, - NHC(O)R', -SR', -S(O)R',-S(O)₂R', -OH, -halogen, -N₃, -NH₂, -NH(R'), -N(R') ₂ and -CN; where each R' is independently selected from -C₁-C₈ alkyl and aryl.

"Alkenyl" refers to an aliphatic hydrocarbon group containing a carbon-carbon double bond which may be straight or branched having 2 to 8 carbon atoms in the chain. Exemplary alkenyl groups include ethenyl, propenyl, n-butenyl, i-butenyl, 3-methylbut-2-enyl, n-pentenyl, hexylenyl, heptenyl, octenyl.

"Alkynyl" refers to an aliphatic hydrocarbon group containing a carbon-carbon triple bond which may be straight or branched having 2 to 8 carbon atoms in the chain. Exemplary alkynyl groups include ethynyl, propynyl, *n*-butynyl, 2-butynyl, 3-methylbutynyl, 5-pentynyl, *n*-pentynyl, hexylynyl, heptynyl, and octynyl.

"Alkylene" refers to a saturated, branched or straight chain or cyclic hydrocarbon radical of 1-18 carbon atoms, and having two monovalent radical centers derived by the removal of two hydrogen atoms from the same or two different carbon atoms of a parent alkane. Typical alkylene radicals include, but are not limited to: methylene (-CH₂-), 1,2-ethyl (-CH₂CH₂-), 1,3-propyl (-CH₂CH₂CH₂-), 1,4-butyl (-CH₂CH₂CH₂CH₂-), and the like.

"Alkenylene" refers to an unsaturated, branched or straight chain or cyclic hydrocarbon radical of 2-18 carbon atoms, and having two monovalent radical centers derived by the removal of two hydrogen atoms from the same or two different carbon atoms of a parent alkene. Typical alkenylene radicals include, but are not limited to: 1,2-ethylene (-CH=CH-).

"Alkynylene" refers to an unsaturated, branched or straight chain or cyclic hydrocarbon radical of 2-18 carbon atoms, and having two monovalent radical centers derived by the removal of two hydrogen atoms from the same or two different carbon atoms of a parent alkyne. Typical alkynylene radicals include, but are not limited to: acetylene, propargyl and 4-pentynyl.

"Aryl" or Ar refers to an aromatic or hetero aromatic group, composed of one or several rings, comprising three to fourteen carbon atoms, preferentially six to ten carbon atoms. The term of "hetero aromatic group" refers one or several carbon on aromatic group, preferentially one, two, three or four carbon atoms are replaced by O, N, Si, Se, P or S, preferentially by O, S, and N. The term aryl or Ar also refers to an aromatic group, wherein one or several H atoms are replaced independently by -R', -halogen, -OR', or - SR', -NR'R'', -N=NR', -N=R', -NR'R'',-NO₂, -S(O)R', -S(O)₂R', -S(O)₂OR', - OS(O)₂OR', -PR'R'', -P(O)R'R'', -P(OR')(OR''), -P(O)(OR')(OR") or - OP(O)(OR')(OR'') wherein R', R'' are independently H, alkyl, alkenyl, alkynyl, heteroalkyl, aryl, arylalkyl, carbonyl, or pharmaceutical salts.

"Heterocycle" refers to a ring system in which one to four of the ring carbon atoms are independently replaced with a heteroatom from the group of O, N, S, Se, B, Si and P. Preferable heteroatoms are O, N and S. Heterocycles are also described in The Handbook of Chemistry and Physics, 78th Edition, CRC Press, Inc., 1997-1998, p. 225 to 226. Preferred nonaromatic heterocyclic include, but are not limited to epoxy, aziridinyl, thiiranyl, pyrrolidinyl, pyrazolidinyl, imidazolidinyl, oxiranyl, tetrahydrofuranyl, dioxolanyl, tetrahydropyranyl, dioxanyl, dioxolanyl, piperidyl, piperazinyl, morpholinyl, pyranyl, imidazolinyl, pyrrolinyl, pyrazolinyl, thiazolidinyl, tetrahydrothiopyranyl, dithianyl, thiomorpholinyl, dihydropyranyl, tetrahydropyranyl, dihydropyranyl, tetrahydropyridyl, dihydropyridyl, tetrahydropyrimidinyl, dihydrothiopyranyl, azepanyl, as well as the fused systems resulting from the condensation with a phenyl group.

The term "heteroaryl" or aromatic heterocycles refers to a 5 to 14, preferably 5 to 10 membered aromatic hetero, mono-, bi- or multicyclic ring. Examples include pyrrolyl, pyridyl, pyrazolyl, thienyl, pyrimidinyl, pyrazinyl, tetrazolyl, indolyl, quinolinyl, purinyl, imidazolyl, thienyl, thiazolyl, benzothiazolyl, furanyl, benzofuranyl, 1,2,4-thiadiazolyl, isothiazolyl, triazolyl, tetrazolyl, isoquinolyl, benzothienyl, isobenzofuryl, pyrazolyl, carbazolyl, benzimidazolyl, isoxazolyl, pyridyl-N-oxide, as well as the fused systems resulting from the condensation with a phenyl group.

"Alkyl", "cycloalkyl", "alkenyl", "alkynyl", "aryl", "heteroaryl", "heterocyclic" and the like refer also to the corresponding "alkylene", "cycloalkylene", "alkenylene", "alkynylene", "arylene", "heteroarylene", "heterocyclene" and the likes which are formed by the removal of two hydrogen atoms.

"Arylalkyl" refers to an acyclic alkyl radical in which one of the hydrogen atoms bonded to a carbon atom, typically a terminal or sp³ carbon atom, is replaced with an aryl radical. Typical arylalkyl groups include, but are not limited to, benzyl, 2-phenylethan-1-yl, 2-phenylethen-1-yl, naphthylmethyl, 2-naphthylethan-1-yl, 2-naphthylethen-1-yl, naphthobenzyl, 2-naphthophenylethan-1-yl and the like.

"Heteroarylalkyl" refers to an acyclic alkyl radical in which one of the hydrogen atoms bonded to a carbon atom, typically a terminal or sp³ carbon atom, is replaced with a heteroaryl radical. Typical heteroarylalkyl groups include, but are not limited to, 2-benzimidazolylmethyl, 2-furylethyl and the like.

Examples of a "hydroxyl protecting group" include, but are not limited to, methoxymethyl ether, 2-methoxyethoxymethyl ether, tetrahydropyranyl ether, benzyl ether, *p-*methoxybenzyl ether, trimethylsilyl ether, triethylsilyl ether, triisopropylsilyl ether, *t*-butyldimethylsilyl ether, triphenylmethylsilyl ether, acetate ester, substituted acetate esters, pivaloate, benzoate, methanesulfonate and p-toluenesulfonate.

"Leaving group" refers to a functional group that can be substituted by another functional group. Such leaving groups are well known in the art, and examples include, but are not limited to, a halide (e.g., chloride, bromide, and iodide), methanesulfonyl (mesyl), *p-*toluenesulfonyl (tosyl), trifluoromethylsulfonyl (triflate), and trifluoromethylsulfonate.

The following abbreviations may be used herein and have the indicated definitions: Boc, *tert*-butoxy carbonyl; BroP, bromotrispyrrolidinophosphonium hexafluorophosphate; CDI, 1,1'-carbonyldiimidazole; DCC, dicyclohexylcarbodiimide; DCE, 1,2-dichloroethane; DCM, dichloromethane; DIAD, diisopropylazodicarboxylate; DIBAL-H, diisobutylalumi-nium hydride; DIPEA, diisopropylethylamine; DEPC, diethyl phosphorocyanidate; DMA, N,N-dimethyl acetamide; DMAP, 4-(*N, N*-dimethylamino)pyridine; DMF, *N,N*-dimethyl-formamide; DMSO, dimethylsulfoxide; DTT, dithiothreitol; EDC, 1-(3-dimethylamino-propyl)-3-ethylcarbodiimide hydrochloride; ESI-MS, electrospray mass spectrometry; HATU, O-(7-azabenzotriazol-1-yl)-*N*, *N, N', N*'-tetramethyluronium hexafluorophosphate; HOBt, 1-hydroxybenzotriazole; HPLC, high pressure liquid chromatography; NHS, N-Hydroxysuccinimide; MMP, 4-methylmorpholine; PAB, *p*-aminobenzyl; PBS, phosphate-buffered saline (pH 7.0~7.5); PEG, polyethylene glycol; SEC, size-exclusion chromatography; TCEP, tris(2-carboxyethyl)phosphine; TFA, trifluoroacetic acid; THF, tetrahydro-furan; Val, valine.

"Pharmaceutically" or "pharmaceutically acceptable" refer to molecular entities and compositions that do not produce an adverse, allergic or other untoward reaction when administered to an animal, or a human, as appropriate.

"Pharmaceutically acceptable solvate" or "solvate" refer to an association of one or more solvent molecules and a disclosed compound. Examples of solvents that form pharmaceutically acceptable solvates include, but are not limited to, water, isopropanol, ethanol, methanol, DMSO, ethyl acetate, acetic acid and ethanolamine.

"Pharmaceutically acceptable excipient" includes any carriers, diluents, adjuvants, or vehicles, such as preserving or antioxidant agents, fillers, disintegrating agents, wetting agents, emulsifying agents, suspending agents, solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions as suitable therapeutic combinations.

As used herein, "pharmaceutical salts" refer to derivatives of the disclosed compounds wherein the parent compound is modified by making acid or base salts thereof. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, tartaric, citric, methanesulfonic, benzenesulfonic, glucuronic, glutamic, benzoic, salicylic, toluenesulfonic, oxalic, fumaric, maleic, lactic and the like. Further addition salts include ammonium salts such as tromethamine, meglumine, epolamine, etc., metal salts such as sodium, potassium, calcium, zinc or magnesium.

The pharmaceutical salts of the present invention can be synthesized from the parent compound which contains a basic or acidic moiety by conventional chemical methods. Generally, such salts can be prepared via reaction the free acidic or basic forms of these compounds with a stoichiometric amount of the appropriate base or acid in water or in an organic solvent, or in a mixture of the two. Generally, non-aqueous media like ether, ethyl acetate, ethanol, isopropanol, or acetonitrile are preferred. Lists of suitable salts are found in Remington's Pharmaceutical Sciences, 17th ed., Mack Publishing Company, Easton, PA, 1985, p. 1418.

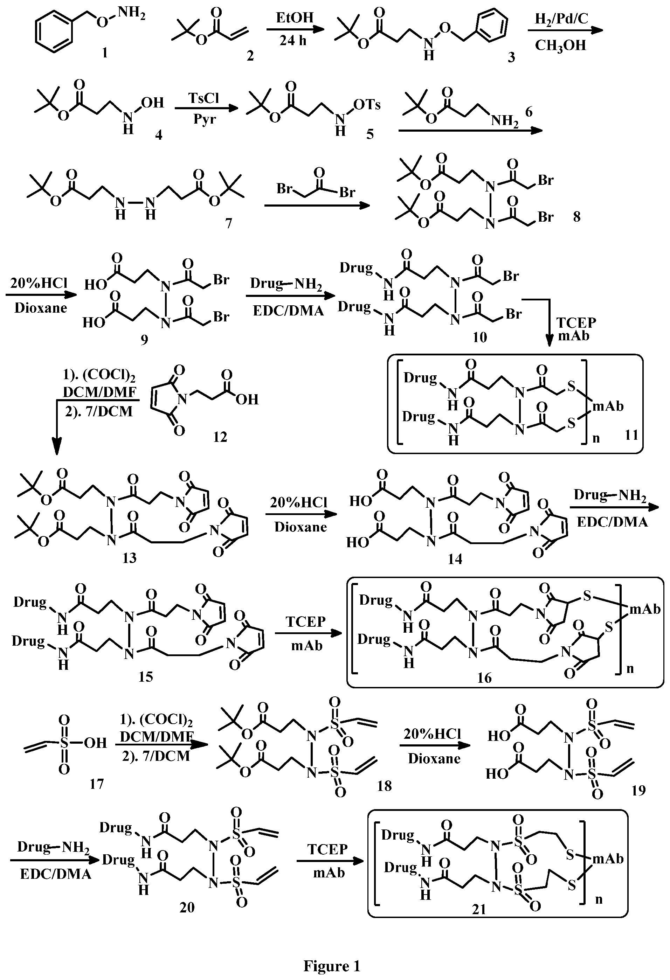

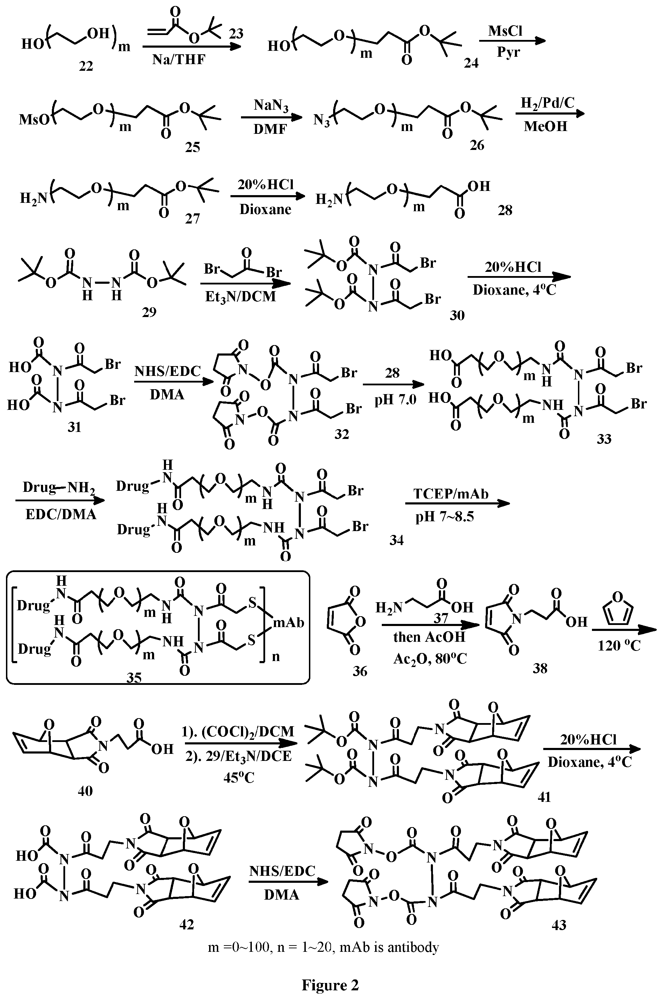

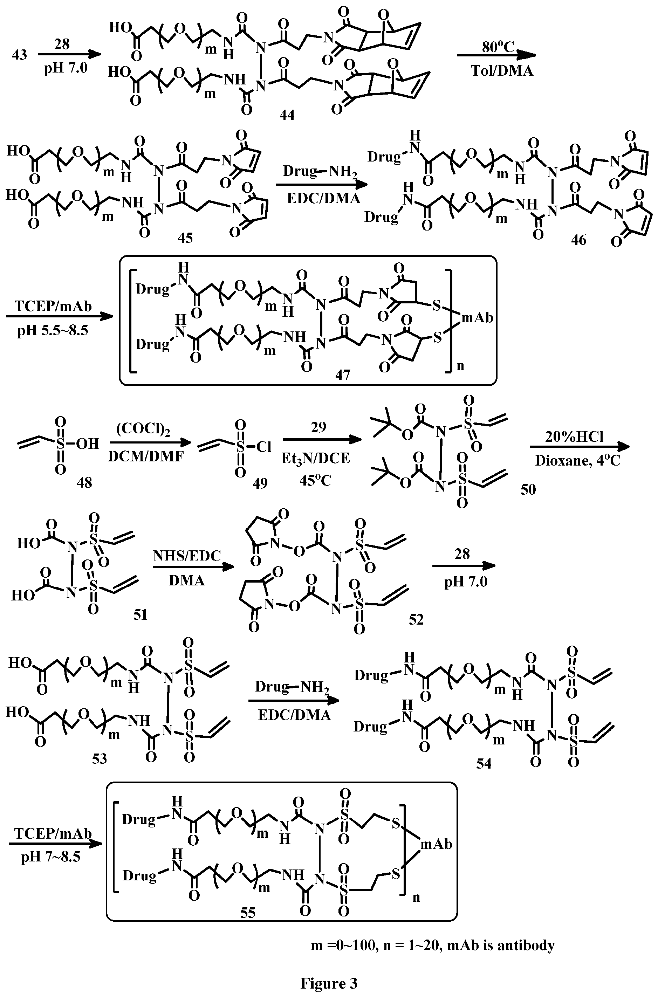

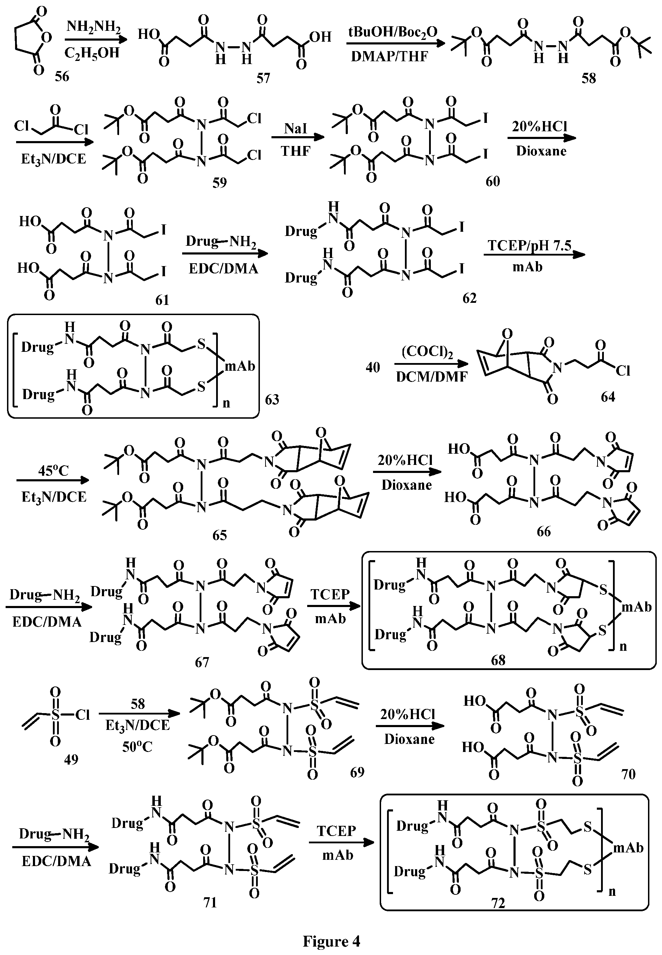

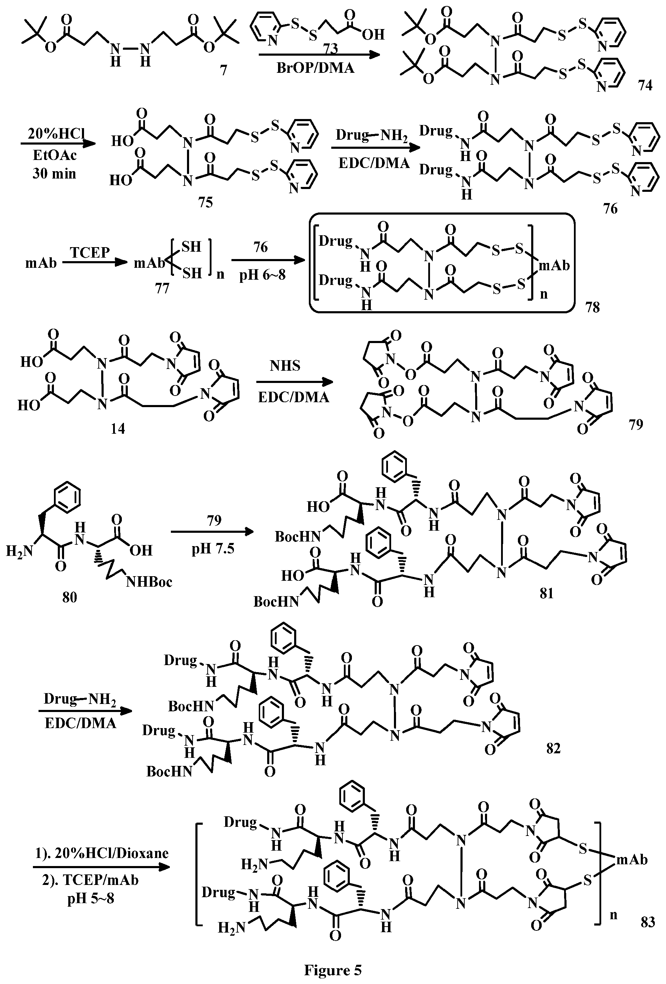

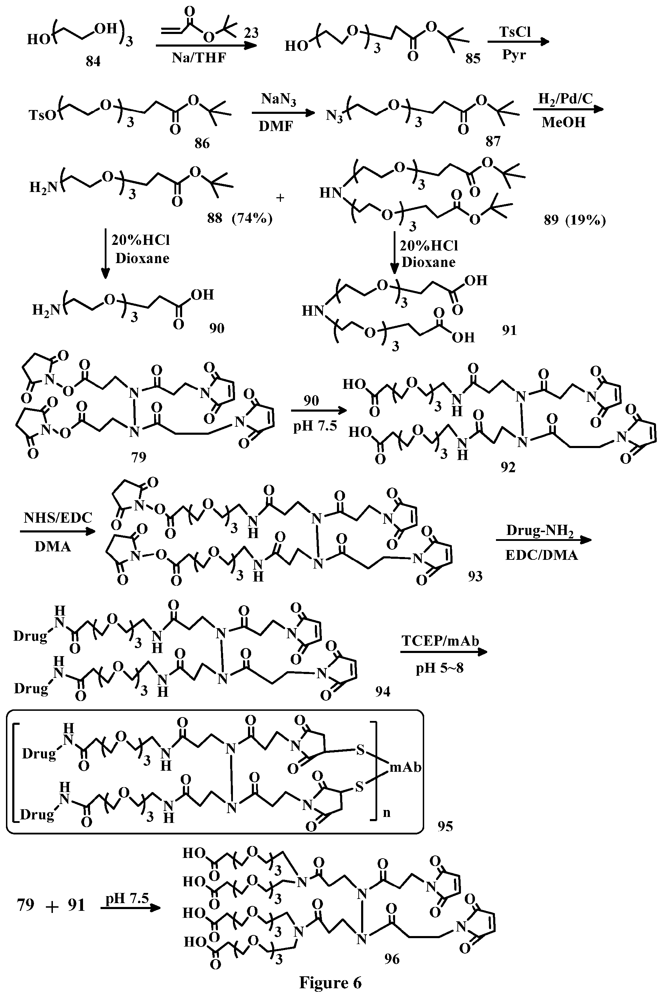

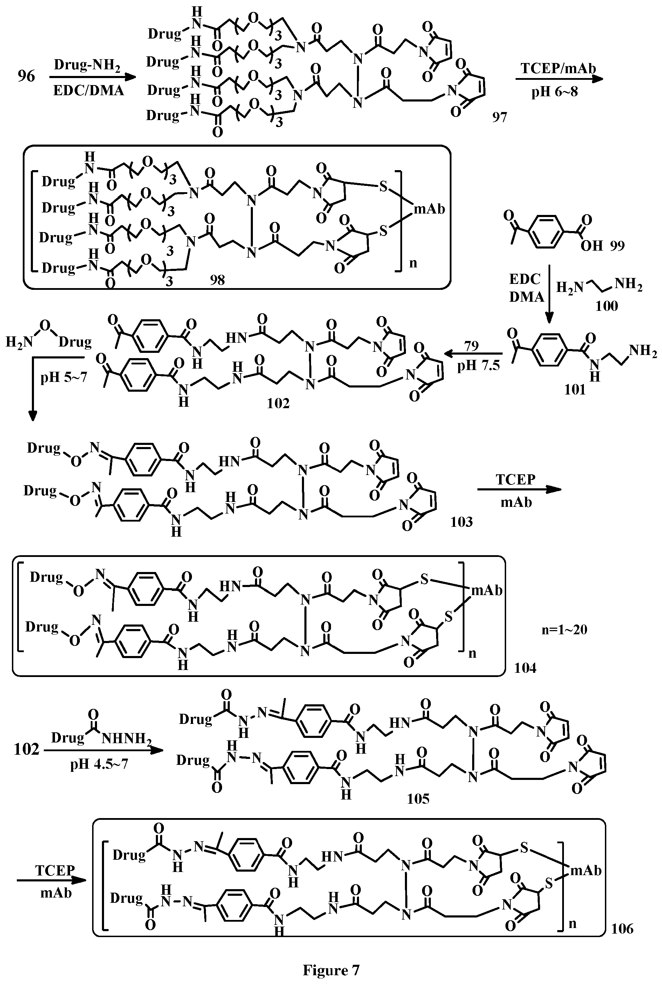

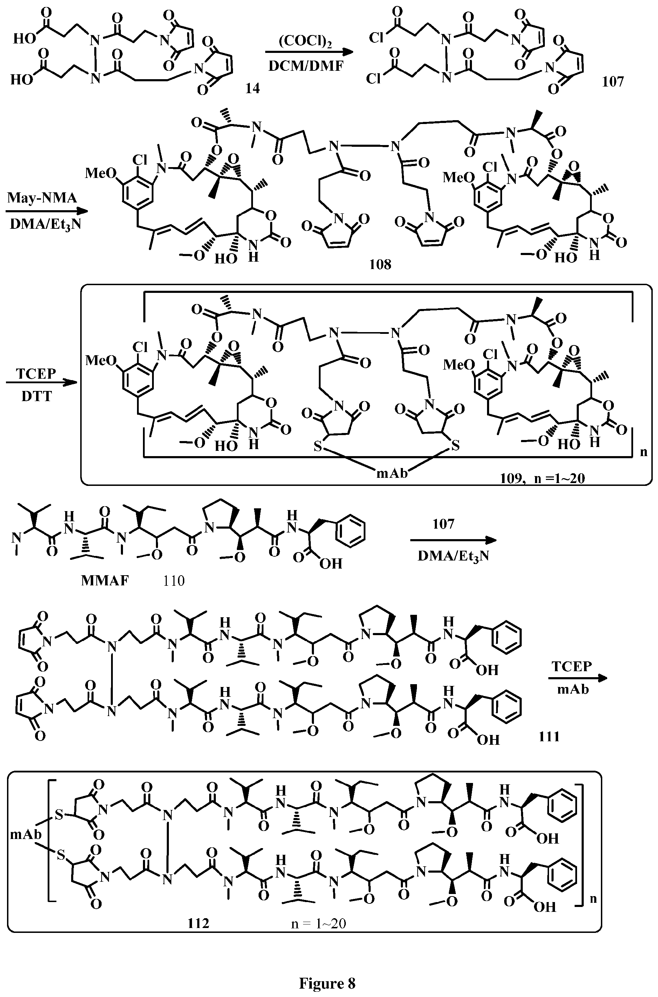

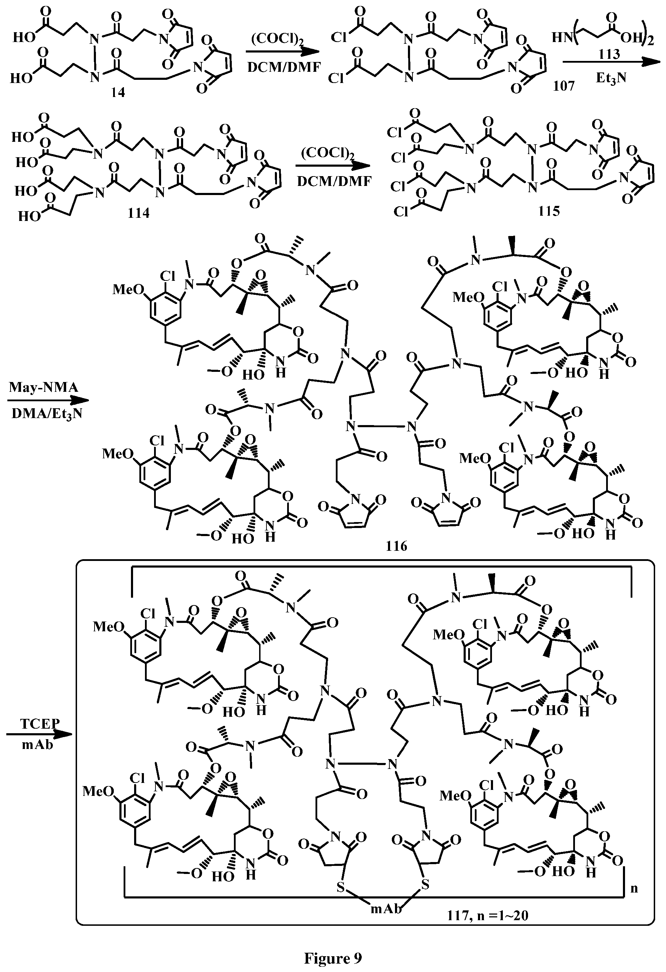

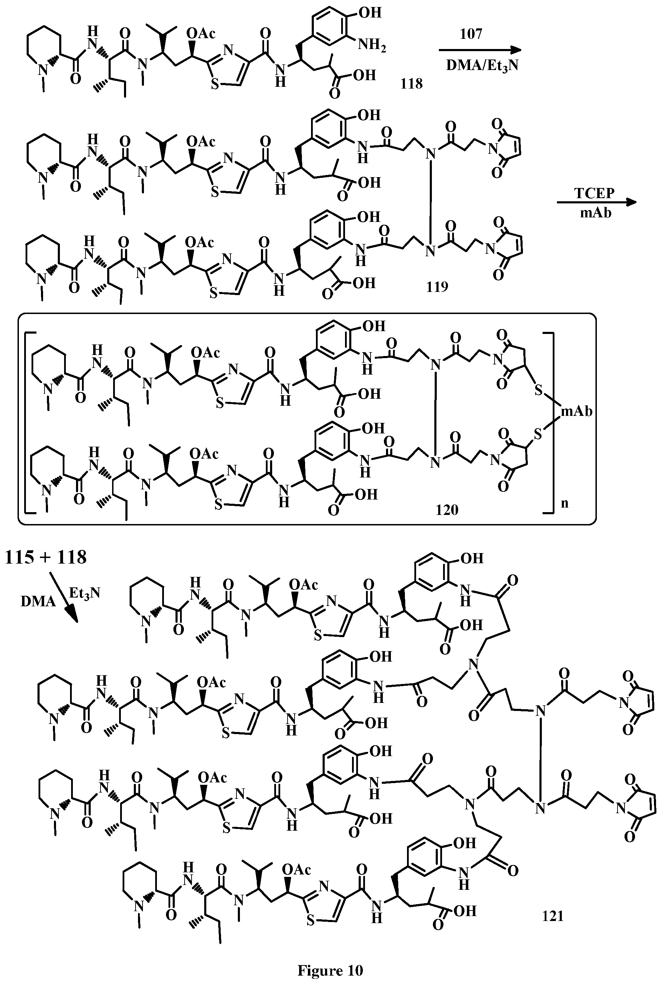

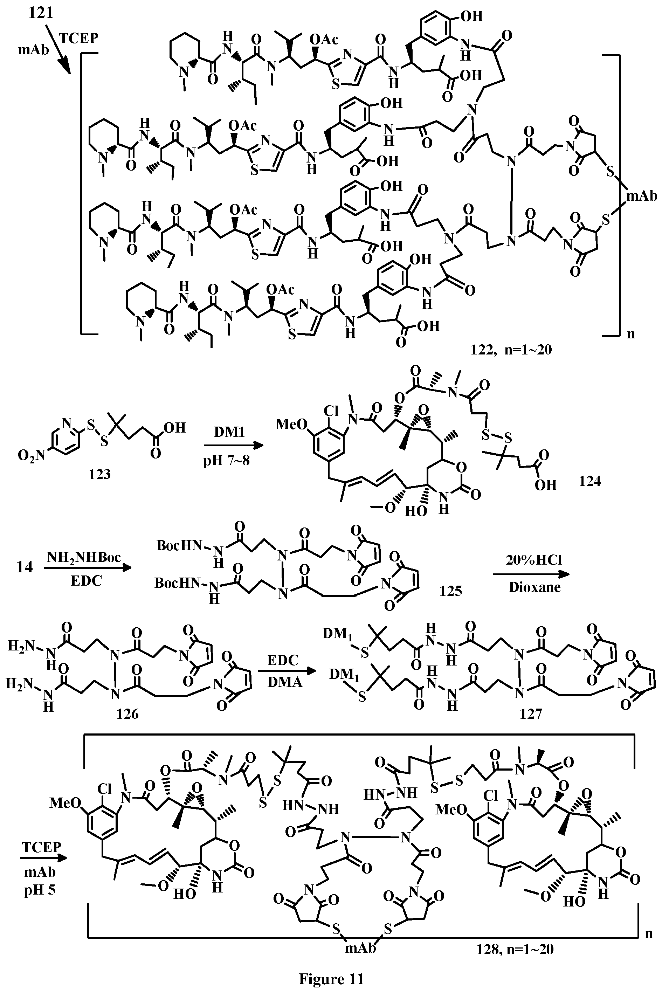

The novel conjugates disclosed herein use the bridge linkers. Examples of some suitable linkers and their synthesis are shown in Figures 1 to 11.

### THE BRIDGE LINKERS

The synthetic routes to produce bridge linkers as well as the preparation of the conjugates of drugs to a cell binding molecules of the present invention are shown in Figures 1~11. The bridge linkers possess three elements: a) Two groups, are the same or independently maleimide haloacetyl, or ethenesulfonyl, groups, capable of reaction with a pair of thiol atoms on a cell-binding agent; b) The middle bridge is hydrazine, linked to the function groups; and c) Two groups, are the same or independently, such as but not limited to, a disulfide, maleimide, haloacetyl, aldehyde, ketone, azide, amine, alkoxyamine and hydrazide, capable of reaction with a drug. The bridge linkers containing hydrazine can be introduced by direct condensation of hydrazine with an acid, an acid halide or acid anhydride, following by introduction of the function groups capable of reaction with a cell-binding agent and drugs. The synthesis of these bridge linkers is exampled in the Figures 1~11 and in the experimental section.

Preferably, the bridge linkers are compounds of the Formula (I) below:

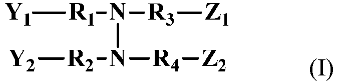

Wherein:
Y₁ and Y₂ are the same or different a functional group that enables reaction with a pair of sulfur atoms of a cell-binding agent, to form disulfide, thioether, or thioester bonds, wherein the function groups for Y₁ and Y₂ are, maleimide, haloacetyl, ethenesulfonyl, groups, as the structures displayed below: 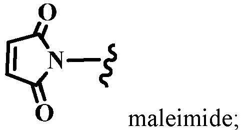 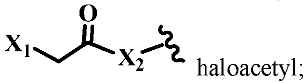 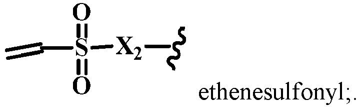 Wherein X₁ is F, Cl, Br, I or Lv; X₂ is O, NH, N(R₁), or CH₂; R₅ is R₁, aromatic, heteroaromatic, or aromatic group wherein one or several H atoms are replaced independently by - R₁, -halogen, -OR₁, -SR₁, -NR₁R₂, - NO₂, -S(O)R₁, -S(O)₂R₁, or -COOR₁; Lv is selected from nitrophenol; N-hydroxysuccinimide (NHS); phenol; dinitrophenol; pentafluorophenol; tetrafluorophenol; difluorophenol; monofluorophenol; pentachlorophenol; triflate; imidazole; dichlorophenol; tetrachlorophenol; 1-hydroxybenzotriazole; tosylate; mesylate; 2-ethyl-5-phenylisoxazolium-3'-sulfonate, anhydrides formed its self, or formed with the other anhydride, e.g. acetyl anhydride, formyl anhydride; or a intermediate molecule generated with a condensation reagent for peptide coupling reactions, or for Mitsunobu reactions.
Z₁ and Z₂ are the same or different a function group that enables to react with a cytotoxic drug. The functional group Z₁ or Z₂ can react to a cytotoxic drug to form a disulfide, ether, ester, thioether, thioester, peptide, hydrazone, carbamate, carbonate, amine (secondary, tertiary, or quaternary), imine, cycloheteroalkyane, heteroaromatic, alkoxime or amide bond;
R₁, R₂, R₃ and R₄ are the same or different, and are absent, linear alkyl having from 1~8 carbon atoms, branched or cyclic alkyl having from 3 to 8 carbon atoms, linear, branched or cyclic alkenyl or alkynyl, or 1~8 carbon atoms of esters, ether, amide, or polyethyleneoxy unit of formula (OCH₂CH₂)ₚ, wherein p is an integer from 0 to about 1000, or combination thereof, and
additionally R₁, R₂, R₃ and R₄ are respectively a chain of atoms selected from C, N, O, S, Si, and P, preferably having 0~500 atoms, which covalently connects to Y₁ or Y₂ and Z₁ or Z₂. The atoms used in forming the R₁, R₂, R₃ and R₄ may be combined in all chemically relevant ways, such as forming alkylate, alkylene, alkenylene, and alkynylene, ethers, polyoxyalkylene, esters, amines, imines, polyamines, hydrazines, hydrazones, amides, ureas, semicarbazides, carbazides, alkoxyamines, alkoxylamines, urethanes, amino acids, peptides, acyloxylamines, hydroxamic acids, or combination thereof.

Examples of the functional group, Z₁ and Z₂, which enable linkage of a cytotoxic drug, include groups that enable linkage via a disulfide, thioether, thioester, peptide, hydrazone, ester, carbamate, carbonate, alkoxime or an amide bond. Such functional groups include, but are not limited to, thiol, disulfide, amino, carboxy, aldehydes, ketone, maleimido, haloacetyl, hydrazines, alkoxyamino, and/or hydroxy.

Examples of the functional group, Z₁ and Z₂, that enable reaction with the terminal of amine of a drug/ cytotoxic agent can be, but not limited to, N-hydroxysuccinimide esters; p-nitrophenyl esters; dinitrophenyl esters; pentafluorophenyl esters; or esters formed with tetrafluorophenol, difluorophenol, monofluorophenol, pentachlorophenol, triflate, imidazole, dichlorophenol, tetrachlorophenol, 1-hydroxybenzotriazole, tosylate, mesylate, 2-ethyl-5-phenylisoxazolium-3'-sulfonate; or an anhydride formed with, e.g. acetyl anhydride, formyl anhydride; or a intermediate molecule generated with a condensation reagent for peptide coupling reactions, or for Mitsunobu reactions. With the terminal of thiol can be, as but not limited to, pyridyldisulfides; nitropyridyldisulfides; maleimides; haloacetates and carboxylic acid chlorides. With the terminal of ketone or aldehyde can be, as but not limited to, amines; alkoxyamines; hydrazines; or acyloxylamine; With the terminal of azide can be, as but not limited to, alkyne.

In preferred embodiments, the functional groups, Z₁ and Z₂, react to Drug1 and Drug2 through condensation of an acid and an amine to form an amide bond. The condensation reagents are, but not limited: EDC (N-(3-Dimethylaminopropyl)-N'-ethylcarbodiimide), DCC (Dicyclohexyl-carbodiimide), N,N'-Diisopropylcarbodiimide (DIC), N-Cyclohexyl-N'-(2-morpholinoethyl)carbodiimide metho-p-toluenesulfonate (CMC,or CME-CDI), 1,1'-Carbonyldiimidazole (CDI),TBTU (O-(Benzotriazol-1-yl)-N,N,N',N'-tetramethyluronium tetrafluoroborate), N,N,N',N'-Tetramethyl-O-(1H-benzotriazol-1-yl)uronium hexafluoro-phosphate (HBTU), (Benzotriazol-1-yloxy)tris(dimethylamino)phosphonium hexafluoro-phosphate (BOP), (Benzotriazol-1-yloxy)tripyrrolidinophosphonium hexafluorophosphate (PyBOP), Diethyl cyanophosphonate (DEPC), Chloro-N,N,N',N'-tetramethylformami-din-ium hexafluorophosphate, 1-[Bis(dimethylamino)methylene]-1H-1,2,3-triazolo[4,5-b]pyridinium 3-oxid hexafluorophosphate (HATU), 1-[(Dimethylamino)(morpholino) methylene]-1H-[1,2,3]triazolo[4,5-b]pyridine-1-ium 3-oxide hexafluorophosphate (HDMA), 2-Chloro-1,3-dimethylimidazolidinium hexafluorophosphate (CIP), Chlorotripyrrolidinophosphonium hexafluorophosphate (PyCloP), Fluoro-N,N,N',N'-bis(tetra-methylene)formamidinium hexafluorophosphate (BTFFH), N,N,N',N'-Tetramethyl-S-(1-oxido-2-pyridyl)thiuronium hexafluorophosphate, O-(2-Oxo-1(2H)pyridyl)-N,N,N',N'-tetra-methyluronium tetrafluoroborate (TPTU), S-(1-Oxido-2-pyridyl)-N,N,N',N'-tetramethyl-thiuronium tetrafluoroborate, O-[(Ethoxycarbonyl) cyano-methylenamino]-N,N,N',N'-tetramethyluronium hexafluorophosphate (HOTU), (1-Cyano-2-ethoxy-2-oxoethylidenaminooxy) dimethylaminomorpholinocarbenium hexafluorophosphate (COMU), O-(Benzotriazol-1-yl)-N,N,N',N'-bis(tetramethylene)uronium hexafluoro-phosphate (HBPyU), N-Benzyl-N'-cyclohexylcarbodiimide (with, or without polymer-bound), Dipyrrolidino(N-succinimidyloxy)carbenium hexafluoro-phosphate (HSPyU), Chlorodipyrrolidinocarbenium hexafluorophosphate (PyClU), 2-Chloro-1,3-dimethyl-imidazolidinium tetrafluoroborate(CIB), (Benzotriazol-1-yloxy) dipiperidinocarbenium hexafluorophosphate (HBPipU), O-(6-Chlorobenzotriazol-1-yl)-N,N,N',N'-tetramethyl-uronium tetrafluoroborate (TCTU), Bromotris(dimethylamino)-phosphonium hexafluoro-phosphate (BroP), Propylphosphonic anhydride (PPACA, T3P^{®}), 2-Morpholinoethyl isocyanide (MEI), N,N,N',N'-Tetramethyl-O-(N-succinimidyl)uronium hexafluoro-phosphate (HSTU), 2-Bromo-1-ethyl-pyridinium tetrafluoroborate (BEP), O-[(Ethoxycarbonyl)cyanomethyle-namino]-N,N,N',N'-tetramethyluronium tetrafluoroborate (TOTU), 4-(4,6-Dimethoxy-1,3,5-triazin-2-yl)-4-methylmorpholinium chloride (MMTM, DMTMM), N,N,N',N'-Tetramethyl-O-(N-succinimidyl)uronium tetrafluoroborate (TSTU), O-(3,4-Dihydro-4-oxo-1,2,3-benzotriazin-3-yl)-N,N,N',N'-tetramethyluronium tetrafluoro-borate (TDBTU), 1,1'-(Azodicarbonyl)dipiperidine (ADD), Di-(4-chlorobenzyl) azodicar-boxylate (DCAD), Di-tert-butyl azodicarboxylate (DBAD), Diisopropyl azodicarboxylate (DIAD), Diethyl azodicarboxylate (DEAD).

In preferred embodiments, R₁, R₂, R₃, and R₄, are linear alkyl having from 1-8 carbon atoms, or containing dipeptides, or tripeptides, or polyethyleneoxy unit of formula (OCH₂CH₂)ₚ, p = 1~100. In addition, R₁, R₂, R₃, and R₄ can be cleavable by a protease.

The detail examples of the synthesis of the bridge linkers are shown in the figures 1~11. Normally the bridge substituent of hydrazine can be condensated with linker components R₁, R₂, R₃, and R₄ containing function groups capable to react to drug compounds and thiol molecules.

### CELL-BINDING AGENT-DRUG CONJUGATES

The conjugates of the present invention can be represented by the following formula, 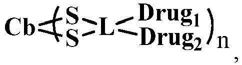 wherein Cb is a cell-binding agent, L is a linker, Drug1 and Drug2 are a drug molecule, n is an integer from 1 to 20, and two S (sulfur) elements from Cb bridgely link to L, which covalently connects two or more drugs (per bridge linker L), wherein the conjugates of the invention have the following Formula (II): 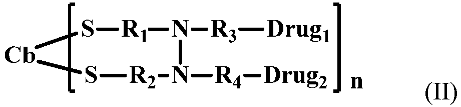 wherein:
Cb represents a cell-binding agent, preferably an antibody;
Drug₁ and Drug₂ represent the same or different drugs/cytotoxic agents, linked to the cell-binding agent via the bridge linker through analkylene, alkenylene, alkynylene, ether, polyoxyalkylene, ester, amine, imine, polyamine, hydrazine, hydrazone, amide, urea, semicarbazide, carbazide, alkoxyamine, urethanes, amino acid, peptide, acyloxylamine, hydroxamic acid, disulfide, thioether, thioester, carbamate, carbonate, heterocyclic ring, heteroaromatic, or alkoxime bond, or combination thereof.

Inside the bracket (parentheses) are the linker-drug components that are conjugated to a cell-binding molecule via a pair of thiol atoms. The conjugatable thiol atoms can generally be generated from reduction of pairs of disulfide bonds on the cell-binding molecule with TCEP or DTT reagents.

n is 1 ~ 20; R₁ R₂, R₃ and R₄ are described the same previously in Formula (I).

As described in more detail below, Drug₁ and Drug₂ can be any of many small molecule drugs, including, but not limited to, tubulysins, calicheamicins, auristatins, maytansinoids, CC-1065 analogs, morpholinos doxorubicins, taxanes, cryptophycins, epothilones, and benzodiazepine dimers (e.g., dimmers of pyrrolobenzodiazepine (PBD) or tomaymycin), indolinobenzodiazepines, imidazobenzothiadiazepines, or oxazolidinobenzodiaze-pines).

To synthesize the conjugate, the cell-binding agent can be first modified with the bridge linkers of the present invention through reduction of disulfide bonds of the cell-binding molecule. The yielded a pair of free thiols can react to the bridge linker of Formula (I) at pH 5~9 aqueous media with or without addition of 0~30% of water mixable (miscible) organic solvents, such as DMA, DMF, ethanol, methanol, acetone, acetonitrile, THF, isopropanol, dioxane, propylene glycol, or ethylene diol, to introduce the reactive groups of Z₁ and Z₂, whose reactive groups can be a disulfide, maleimido, haloacetyl, azide, 1-yne, ketone, aldehyde, alkoxyamino, or hydrazide. Then the reactive group of a cytotoxic agent reacts to the modified cell-binding molecule accordingly. For example, synthesis of the cell-binding agent-drug conjugates linked via disulfide bonds is achieved by a disulfide exchange between the disulfide bond in the modified cell-binding agent and a drug containing a free thiol group. Synthesis of the cell-binding agent-drug conjugates linked via thioether is achieved by reaction of the maleimido or haloacetyl or ethylsulfonyl modified cell-binding agent and a drug containing a free thiol group. Synthesis of conjugates bearing an acid labile hydrazone can be achieved by reaction of a carbonyl group with the hydrazide moiety in the linker, by methods known in the art (see, for example, P. Hamann et al., Hinman, L. M., et al, Cancer Res. 53, 3336-334, 1993; B. Laguzza et al., J. Med. Chem., 32; 548-555, 1959; P. Trail et al., Cancer Res., 57; 100-105, 1997). Synthesis of conjugates bearing triazole linkage can be achieved by reaction of a 1-yne group of the drug with the azido moiety in the linker, through the click chemistry (Huisgen cycloaddition) (Lutz, J-F. et al, 2008, Adv. Drug Del. Rev. 60, 958-970; Sletten, E. M. et al 2011, Acc Chem. Research 44, 666-676).

Alternatively, the drug can react with the bridge linkers of the present disclosure that have conjugated to a cell-binding molecule to give a modified cell-binding molecule linker of Formula (III) bearing functionalities. For example, a thiol-containing drug can react with the modified cell-binding molecule bridge linker of Formula (III) bearing a maleimdo, or a haloacetyl, or an ethylsulfonyl substituent at pH 5.0~9.0 in aqueous buffer to give a cell-binding molecule-drug conjugate via a thioether linkage. A thiol-containing drug can undergo disulfide exchange with a modified bridge linker of Formula (III) bearing a pyridyldithio moiety to give a conjugate a disulfide bond linkage. A drug bearing a hydroxyl group or a thiol group can be reacted with a modified bridge linker of Formula (III) bearing a halogen, particularly the alpha halide of carboxylates, in the presence of a mild base, e.g. pH 7.5~9.5, to give a modified drug bearing an ether or thiol ether link. A hydroxyl group containing drug can be condensed with a bridge cross linker of Formula (I) bearing a carboxyl group, in the presence of a dehydrating agent, such as EDC or DCC, to give ester linkage, then the subject drug modified bridge linker undergoes the conjugation with a cell-binding molecule. A drug containing an amino group can condensate with a carboxyl ester of NHS, imidazole, nitrophenol; N-hydroxysuccinimide (NHS); phenol; dinitrophenol; pentafluorophenol; tetrafluorophenol; difluorophenol; monofluorophenol; pentachlorophenol; triflate; imidazole; dichlorophenol; tetrachlorophenol; 1-hydroxybenzotriazole; tosylate; mesylate; 2-ethyl-5-phenylisoxazolium-3'-sulfonate on the cell-binding molecule-bridge linker of Formula (III) to give a conjugate via amide bond linkage.

The conjugate may be purified by standard biochemical means, such as gel filtration on a Sephadex G25 or Sephacryl S300 column, adsorption chromatography, and ion exchange or by dialysis. In some cases, a small molecule as a cell-binding agent (e.g. folic acid, melanocyte stimulating hormone, EGF etc) conjugated with a small molecular drugs can be purified by chromatography such as by HPLC, medium pressure column chromatography or ion exchange chromatography.

### MODIFIED CELL-BINDING AGENTS/MOLECULES

The cell-binding agent modified by reaction with linkers of the present disclosure are preferably represented by the Formula (III) 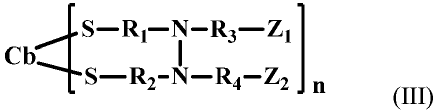 Wherein Cb, Z₁, Z₂, n, R₁, R₂, R₃ and R₄ are defined the same as in Formula (I) and (II).

In preferred embodiments, Z₁ and Z₂ are a disulfide substituent, maleimido, haloacetyl, alkoxyamine, azido, ketone, aldehyde, hydrazine group, an *N*-hydroxysuccinimide ester, or a carboxyl ester formed with phenol; dinitrophenol; pentafluorophenol; tetrafluorophenol; difluorophenol; monofluorophenol; pentachlorophenol; triflate; imidazole; dichlorophenol; tetrachlorophenol; 1-hydroxybenzotriazole; tosylate; mesylate; 2-ethyl-5-phenyl-isoxa-zolium-3'-sulfonate. Z₁ and Z₂ can then react with a cytotoxic agent through thioether, hydrazone, amide, alkoxime, carbamate, ester, ether or disulfide bond. The modified cell-binding agent can be prepared via a reaction of the cell-binding agent with the bridge linkers of Formula (I) as described in Formula (II) above.

In order to achieve a higher conjugation yield for the reaction of the function groups Y₁ and Y₂ on the bridge linkers of Formula (I) with a pair of free thiols on the cell-binding molecule, a small percentage of organic co-solvent may be required to add to the reaction mixture, as well added in the solution after the reaction to maintain solubility of the Formula (III) in aqueous solution. To modify the cell-binding agents, the cross-linking reagent (bridge linker) of Formula (I) can be first dissolved in a polar organic solvent, which is miscible with water, for example different alcohols, such as methanol, ethanol, and propanol, acetone, acetonitrile, tetrahydrofuran (THF), 1,4-dioxane, dimethyl formamide (DMF), dimethyl acetamide (DMA), or dimethylsulfoxide (DMSO), at a high concentration, for example 1-500 mM. Meanwhile, the cell-binding molecule, such as antibody dissolved in an aqueous buffer pH 5~9.5, preferably pH 6~8.5, at 1~35 mg/ml concentration is treated with 1~20 equivalent of TCEP or DTT for 20 min to 12 hour. After the reduction, DTT can be removed by SEC chromatographic purification. TCEP can be optionally removed by SEC chromatography too, or can be staying in the reaction mixture for the next step reaction without purification. Furthermore, the reduction of antibodies or the other cell-binding agents with TCEP can be performed in the presence of a bridge linker of Formula (I), for which the conjugation reaction can be achieved simultaneously along with the TCEP reduction. After conjugation reaction, the over reduced free thiols on the cell-binding molecules can be oxidized with DHAA or Cu (II) to regenerate the disulfide bonds, or the free thiols can be capped with a thiol-reactive molecule, such as N-ethyl maleimide, sodium iodoacetate, sodium bromoacetate, bromoacetic acid methyl ester.

The aqueous solutions for the modification of cell-binding agents are buffered between pH 6 and 9, preferably between 6.5 and 7.5 and can contain any non-nucleophilic buffer salts useful for these pH ranges. Typical buffers include phosphate, triethanolamine HCl, HEPES, and MOPS buffers, which can contain additional components, such as cyclodextrins, sucrose and salts, for examples, NaCl and KCl. After the addition of the bridge linker of Formula (I) into the solution containing the reduced cell-binding molecules, the reaction mixture is incubated at a temperature of from 4 °C to 45 °C, preferably at ambient temperature. The progress of the reaction can be monitored by measuring the decrease in the absorption at 254 nm, or increase in the absorption at 280 nm, or the other changes at an appropriate wavelength. After the reaction is complete, isolation of the modified cell-binding agent can be performed in a routine way, using for example gel filtration chromatography, or adsorptive chromatography.

The extent of modification can be assessed by measuring the absorbance of the nitropyridine thione, dinitropyridine dithione, pyridine thione, carboxamidopyridine dithione and dicarboxamidopyridine dithione group released via UV spectra. For the conjugation without a chromophore group, the modification or conjugation reaction can be monitored by LC-MS, preferably by UPLC-QTOF mass spectrometry, or Capilary electrophoresis-mass spectrometry (CE-MS). The bridge cross-linkers described herein have diverse functional groups that can react with any drugs, preferably cytotoxic agents that possess a suitable substituent. For examples, the modified cell-binding molecules bearing an amino or hydroxyl substituent can react with drugs bearing an N-hydroxysuccinimide (NHS) ester, the modified cell-binding molecules bearing a thiol substituent can react with drugs bearing a maleimido or haloacetyl group. Additionally, the modified cell-binding molecules bearing a carbonyl (ketone or aldehyde) substituent can react with drugs bearing a hydrazide or an alkoxyamine. One skilled in the art can readily determine which linker to use based on the known reactivity of the available functional group on the linkers.

### MODIFIED CYTOTOXIC DRUGS

The cytotoxic drugs modified by reaction with cross-linkers of the present invention are preferably represented by the Formula (IV):

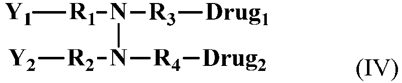

Wherein Y₁, Y₂, Drug₁, Drug₂, R₁, R₂ R₃ and R₄ are defined the same as in Formula (I) and (II).

In preferred embodiments, Y₁ and Y₂ are a a maleimido; a haloacetyl; an ethenesulfonyl (i.e., as defined herein).

The modified drugs can be prepared via reaction of the drug with the linkers of the Formula (I) to give a modified drug of Formula (IV) bearing functionality of Y₁ and Y₂ groups capable of reacting with a pair of thiol groups of a cell-binding agent. But for drugs containing a thiol, or the drugs undergoing to link a cell-binding molecule via the bridge linkers through thioether, thioester or disulfide bond, it is therefore preferred that the Drug₁ or Drug₂ may be synthesized to connect to R₃, or R₄ in a piece of components via the linkage of thioether, thioester or disulfide bond first. Then the synthesized R₃-Drug₁ or R₄-Drug₂ component is assembled to a hydrazine group to form the bridge linker modified drugs of Formula (IV).

For examples of the synthesis, a thiol-containing drug can be reacted with the linker of components R₃ or R₄ bearing a maleimdo substituent at neutral pH in aqueous buffer to give a R₃-Drug₁ or R₄-Drug₂ compartment bearing thioether linkage, and following by condensation with a compartment of hydrazine group containing function Y₁ and Y₂ groups to give a modified drug of Formula (IV) bearing thioether linkage. A drug bearing a hydroxyl group can be reacted with a linker component R₃ or R₄ bearing a halogen, or a tosylate, or a mesylate, in the presence of a mild base, to give a R₃-Drug₁ or R₄-Drug₂ compartment bearing ether linkage, and following by condensation with a compartment of hydrazine group containing function groups Y₁ and Y₂ to give a modified drug of Formula (IV) bearing thioether linkage. A hydroxyl group containing drug can be condensed with a linker of Formula (I) bearing a carboxyl group, in the presence of a dehydrating agent, such as EDC or dicyclohexylcarbodiimide (DCC), to give a modified drug of Formula (IV) via ester linkage. A drug bearing a thiol group can also react the linker of components R₃ or R₄ bearing a maleimido or a vinylsulfonyl, or a haloacetyl group, give a R₃-Drug₁ or R₄-Drug₂ compartment bearing thioether linkage, and following by condensation with a compartment of hydrazine group containing function groups Y₁ and Y₂ to give a modified drug of Formula (IV) bearing thioether linkage. An amino group containing drug can similarly undergo condensation with a carboxyl group on the bridge linker of Formula (I) to give a modified drug of Formula (IV) bearing amide bonds. The modified drug can be purified by standard methods such as column chromatography over silica gel or alumina, crystallization, preparatory thin layer chromatography, ion exchange chromatography, or HPLC.

### CELL-BINDING AGENTS

The cell-binding molecule that comprises the conjugates and the modified cell-binding agents of the present invention may be of any kind presently known, or that become known, molecule that binds to, complexes with, or reacts with a moiety of a cell population sought to be therapeutically or otherwise biologically modified.

The cell binding agents include, but are not limited to, large molecular weight proteins such as, for example, full-length antibodies (polyclonal antibodies, monoclonal antibodies, dimers, multimers, multispecific antibodies (e.g., bispecific antibodies); single chain antibodies; fragments of antibodies such as Fab, Fab', F(ab')₂, Fᵥ, [Parham, J. Immunol. 131, 2895-2902 (1983)], fragments produced by a Fab expression library, anti-idiotypic (anti-Id) antibodies, CDR's, diabody, triabody, and epitope-binding fragments of any of the above which immuno-specifically bind to cancer cell antigens, viral antigens, microbial antigens or a protein generated by the immune system that is capable of recognizing, binding to a specific antigen or exhibiting the desired biological activity (Miller et al (2003) J. of Immunology 170:4854-4861); interferons (such as type I, II, III); peptides; lymphokines such as IL-2, IL-3, IL-4, IL-5, IL-6, IL-10, GM-CSF, interferon-gamma (IFN-γ); hormones such as insulin, TRH (thyrotropin releasing hormones), MSH (melanocyte-stimulating hormone), steroid hormones, such as androgens and estrogens, melanocyte-stimulating hormone (MSH); growth factors and colony-stimulating factors such as epidermal growth factors (EGF), granulocyte-macrophage colony-stimulating factor (GM-CSF), transforming growth factors (TGF), such as TGFα, TGFβ, insulin and insulin like growth factors (IGF-I, IGF-II) G-CSF, M-CSF and GM-CSF [Burgess, Immunology Today, 5, 155-158 (1984)]; vaccinia growth factors (VGF); fibroblast growth factors (FGFs); smaller molecular weight proteins, poly-peptide, peptides and peptide hormones, such as bombesin, gastrin, gastrin-releasing peptide; platelet-derived growth factors; interleukin and cytokines, such as interleukin-2 (IL-2), interleukin-6 (IL-6), leukemia inhibitory factors, granulocyte-macrophage colony-stimulating factor (GM-CSF); vitamins, such as folate; apoproteins and glycoproteins, such as transferrin [O'Keefe et al, 260 J. Biol. Chem. 932-937 (1985)]; sugar-binding proteins or lipoproteins, such as lectins; cell nutrient-transport molecules; and small molecular inhibitors, such as prostate-specific membrane antigen (PSMA) inhibitors and small molecular tyrosine kinase inhibitors (TKI), non-peptides or any other cell binding molecule or substance, such as bioactive polymers (Dhar, et al, Proc. Natl. Acad. Sci. 2008, 105, 17356-61); bioactive dendrimers (Lee, et al, Nat. Biotechnol. 2005, 23, 1517-26; Almutairi, et al; Proc. Natl. Acad. Sci. 2009, 106, 685-90); nanoparticles (Liong, et al, ACS Nano, 2008, 19, 1309-12; Medarova, et al, Nat. Med. 2007, 13, 372-7; Javier, et al, Bioconjugate Chem. 2008, 19, 1309-12); liposomes (Medinai, et al, Curr. Phar. Des. 2004, 10, 2981-9); viral capsides (Flenniken, et al, Viruses Nanotechnol. 2009, 327, 71-93).

In general, a monoclonal antibody is preferred as a cell-surface binding agent if an appropriate one is available. And the antibody may be murine, human, humanized, chimeric, or derived from other species.

Production of antibodies used in the present invention involves *in vivo* or *in vitro* procedures or combinations thereof. Methods for producing polyclonal anti-receptor peptide antibodies are well-known in the art, such as in U.S. Pat. No. 4,493,795 (to Nestor et al). A monoclonal antibody is typically made by fusing myeloma cells with the spleen cells from a mouse that has been immunized with the desired antigen (Köhler, G.; Milstein, C. (1975). Nature 256: 495-497). The detailed procedures are described in "Antibodies--A Laboratory Manual", Harlow and Lane, eds., Cold Spring Harbor Laboratory Press, New York (1988). Particularly monoclonal antibodies are produced by immunizing mice, rats, hamsters or any other mammal with the antigen of interest such as the intact target cell, antigens isolated from the target cell, whole virus, attenuated whole virus, and viral proteins. Splenocytes are typically fused with myeloma cells using polyethylene glycol (PEG) 6000. Fused hybrids are selected by their sensitivity to HAT (hypoxanthine-aminopterin-thymine). Hybridomas producing a monoclonal antibody useful in practicing this invention are identified by their ability to immunoreact specified receptors or inhibit receptor activity on target cells.

A monoclonal antibody used in the present invention can be produced by initiating a monoclonal hybridoma culture comprising a nutrient medium containing a hybridoma that secretes antibody molecules of the appropriate antigen specificity. The culture is maintained under conditions and for a time period sufficient for the hybridoma to secrete the antibody molecules into the medium. The antibody-containing medium is then collected. The antibody molecules can then be further isolated by well-known techniques, such as using protein-A affinity chromatography; anion, cation, hydrophobic, or size exclusive chromatographies (particularly by affinity for the specific antigen after protein A, and sizing column chromatography); centrifugation, differential solubility, or by any other standard technique for the purification of proteins.

Media useful for the preparation of these compositions are both well-known in the art and commercially available and include synthetic culture media. An exemplary synthetic medium is Dulbecco's minimal essential medium (DMEM; Dulbecco et al., Virol. 8, 396 (1959)) supplemented with 4.5 gm/l glucose, 0~20 mM glutamine, 0~20% fetal calf serum, several ppm amount of heavy metals, such as Cu, Mn, Fe, or Zn, etc, or/and the heavy metals added in their salt forms, and with an anti-foaming agent, such as polyoxyethylene-polyoxypropylene block copolymer.

In addition, antibody-producing cell lines can also be created by techniques other than fusion, such as direct transformation of B lymphocytes with oncogenic DNA, or transfection with an oncovirus, such as Epstein-Barr virus (EBV, also called human herpesvirus 4 (HHV-4)) or Kaposi's sarcoma-associated herpesvirus (KSHV). See, U.S. Pat. Nos. 4,341,761; 4,399,121; 4,427,783; 4,444,887; 4,451,570; 4,466,917; 4,472,500; 4,491,632; 4,493,890. A monoclonal antibody may also be produced via an anti-receptor peptide or peptides containing the carboxyl terminal as described well-known in the art. See Niman et al., Proc. Natl. Acad. Sci. USA, 80: 4949-4953 (1983); Geysen et al., Proc. Natl. Acad. Sci. USA, 82: 178-182 (1985); Lei et al. Biochemistry 34(20): 6675-6688, (1995). Typically, the anti-receptor peptide or a peptide analog is used either alone or conjugated to an immunogenic carrier, as the immunogen for producing anti-receptor peptide monoclonal antibodies.

There are also a number of other well-known techniques for making monoclonal antibodies as binding molecules in this invention. Particularly useful are methods of making fully human antibodies. One method is phage display technology which can be used to select a range of human antibodies binding specifically to the antigen using methods of affinity enrichment. Phage display has been thoroughly described in the literature and the construction and screening of phage display libraries are well known in the art, see, e.g., Dente et al, Gene. 148(1):7-13 (1994); Little et al, Biotechnol Adv. 12(3):539-55 (1994); Clackson et al., Nature 352:264-628 (1991); Huse et al., Science 246:1275-1281 (1989).

Monoclonal antibodies derived by hybridoma technique from another species than human, such as mouse, can be humanized to avoid human anti-mouse antibodies when infused into humans. Among the more common methods of humanization of antibodies are complementarity-determining region grafting and resurfacing. These methods have been extensively described, see e.g. U.S. Pat. Nos. 5,859,205 and 6,797,492; Liu et al, Immunol Rev. 222:9-27 (2008); Almagro et al, Front Biosci. 13: 1619-33 (2008); Lazar et al, Mol Immunol. 44(8):1986-98 (2007); Li et al, Proc. Natl. Acad. Sci. U S A. 103(10):3557-62 (2006). Fully human antibodies can also be prepared by immunizing transgenic mice, rabbits, monkeys, or other mammals, carrying large portions of the human immunoglobulin heavy and light chains, with an immunogen. Examples of such mice are: the Xenomouse. (Abgenix/Amgen), the HuMAb-Mouse (Medarex/BMS), the VelociMouse (Regeneron), see also U.S. Pat. No. 6,596,541 , 6,207,418, No. 6,150,584, No. 6,111,166, No. 6,075,181, No. 5,922,545, Nos. 5,661,016, 5,545,806, 5,436,149 and 5,569,825. In human therapy, murine variable regions and human constant regions can also be fused to construct called "chimeric antibodies" that are considerably less immunogenic in man than murine mAbs (Kipriyanov et al, Mol Biotechnol. 26:39-60 (2004); Houdebine, Curr Opin Biotechnol. 13:625-9 (2002)). In addition, site-directed mutagenesis in the variable region of an antibody can result in an antibody with higher affinity and specificity for its antigen (Brannigan et al, Nat Rev Mol Cell Biol. 3:964-70, (2002)); Adams et al, J Immunol Methods. 231:249-60 (1999)) and exchanging constant regions of a mAb can improve its ability to mediate effector functions of binding and cytotoxicity.

Antibodies immunospecific for a malignant cell antigen can also be obtained commercially or produced by any method known to one of skill in the art such as, e.g., chemical synthesis or recombinant expression techniques. The nucleotide sequence encoding antibodies immunospecific for a malignant cell antigen can be obtained commercially, e.g., from the GenBank database or a database like it, the literature publications, or by routine cloning and sequencing.

Apart from an antibody, a peptide or protein that bind/block/target or in some other way interact with the epitopes or corresponding receptors on a targeted cell can be used as a binding molecule. These peptides or proteins could be any random peptide or proteins that have an affinity for the epitopes or corresponding receptors and they don't necessarily have to be of the immunoglobulin family. These peptides can be isolated by similar techniques as for phage display antibodies (Szardenings, J Recept Signal Transduct Res. 2003; 23(4):307-49). The use of peptides from such random peptide libraries can be similar to antibodies and antibody fragments. The binding molecules of peptides or proteins may be conjugated on or linked to a large molecules or materials, such as, but is not limited, an albumin, a polymer, a liposome, a nano particle, a dendrimer, as long as such attachment permits the peptide or protein to retain its antigen binding specificity.

Examples of antibodies used for conjugation of drugs via the bridge linkers of this prevention for use in treating cancer, autoimmune disease, and/or infectious disease include, but are not limited to, 3F8 (anti-GD2), Abagovomab (anti CA-125), Abciximab (anti CD41 (integrin alpha-IIb), Adalimumab (anti-TNF-α), Adecatumumab (anti-EpCAM, CD326), Afelimomab (anti-TNF-α); Afutuzumab (anti-CD20), Alacizumab pegol (anti-VEGFR2), ALD518 (anti-IL-6), Alemtuzumab (Campath, MabCampath, anti-CD52), Altumomab (anti-CEA), Anatumomab (anti-TAG-72), Anrukinzumab (IMA-638, anti-IL-13), Apolizumab (anti-HLA-DR), Arcitumomab (anti-CEA), Aselizumab (anti-L-selectin (CD62L), Atlizumab (tocilizumab, Actemra, RoActemra, anti-IL-6 receptor), Atorolimumab (anti-Rhesus factor), Bapineuzumab (anti-beta amyloid), Basiliximab (Simulect, antiCD25 (α chain of IL-2 receptor), Bavituximab (anti-phosphatidylserine), Bectumomab (LymphoScan, anti-CD22), Belimumab (Benlysta, LymphoStat-B, anti-BAFF), Benralizumab (anti-CD125), Bertilimumab (anti-CCL11 (eotaxin-1)), Besilesomab (Scintimun, anti-CEA-related antigen), Bevacizumab (Avastin, anti-VEGF-A), Biciromab (FibriScint, anti-fibrin II beta chain), Bivatuzumab (anti-CD44 v6), Blinatumomab (BiTE, anti-CD19), Brentuximab (cAC10, anti-CD30 TNFRSF8), Briakinumab (anti-IL-12, IL-23) Canakinumab (Ilaris, anti-IL-1), Cantuzumab (C242, anti-CanAg), Capromab, Catumaxomab (Removab, anti-EpCAM, anti-CD3), CC49 (anti-TAG-72), Cedelizumab (anti-CD4), Certolizumab pegol (Cimzia anti-TNF-α), Cetuximab (Erbitux, IMC-C225, anti-EGFR), Citatuzumab bogatox (anti-EpCAM), Cixutumumab (anti-IGF-1), Clenoliximab (anti-CD4), Clivatuzumab (anti-MUC1), Conatumumab (anti-TRAIL-R2), CR6261 (anti-Influenza A hemagglutinin), Dacetuzumab (anti-CD40), Daclizumab (Zenapax, anti-CD25 (α chain of IL-2 receptor)), Daratumumab (anti-CD38 (cyclic ADP ribose hydrolase), Denosumab (Prolia, anti-RANKL), Detumomab (anti-B-lymphoma cell), Dorlimomab, Dorlixizumab, Ecromeximab (anti-GD3 ganglioside), Eculizumab (Soliris, anti-C5), Edobacomab (anti-endotoxin), Edrecolomab (Panorex, MAb17-1A, anti-EpCAM), Efalizumab (Raptiva, anti-LFA-1 (CD11a), Efungumab (Mycograb, anti-Hsp90), Elotuzumab (anti-SLAMF7), Elsilimomab (anti-IL-6), Enlimomab pegol (anti-ICAM-1 (CD54)), Epitumomab (anti-episialin), Epratuzumab (anti-CD22), Erlizumab (anti-ITGB2 (CD18)), Ertumaxomab (Rexomun, anti-HER2/neu, CD3), Etaracizumab (Abegrin, anti-integrin αᵥβ₃), Exbivirumab ( anti-hepatitis B surface antigen), Fanolesomab (NeutroSpec, anti-CD15), Faralimomab (anti-interferon receptor), Farletuzumab (anti-folate receptor 1), Felvizumab (anti-respiratory syncytial virus), Fezakinumab (anti-IL-22), Figitumumab (anti-IGF-1 receptor), Fontolizumab (anti-IFN-γ), Foravirumab (anti-rabies virus glycoprotein), Fresolimumab (anti-TGF-β), Galiximab (anti-CD80), Gantenerumab (anti- beta amyloid), Gavilimomab (anti-CD147 (basigin)), Gemtuzumab (anti-CD33), Girentuximab (anti-carbonic anhydrase 9), Glembatumumab (CR011, anti-GPNMB), Golimumab (Sim-poni, anti-TNF-α), Gomiliximab (anti-CD23 (IgE receptor)), Ibalizumab (anti-CD4), Ibritumomab (anti-CD20), Igovomab (Indimacis-125, anti-CA-125), Imciromab (Myoscint, anti-cardiac myosin), Infliximab (Remicade, anti-TNF-α), Intetumumab (anti-CD51), Inolimomab (anti-CD25 (α chain of IL-2 receptor)), Inotuzumab (anti-CD22), Ipilimumab (anti-CD152), Iratumumab (anti- CD30 (TNFRSF8)), Keliximab (anti-CD4), Labetuzumab (CEA-Cide, anti-CEA), Lebrikizumab (anti- IL-13), Lemalesomab (anti-NCA-90 (granulocyte antigen)), Lerdelimumab (anti-TGF beta 2), Lexatumumab (anti-TRAIL-R2), Libivirumab (anti-hepatitis B surface antigen), Lintuzumab (anti-CD33), Lucatumumab (anti-CD40), Lumiliximab (anti- CD23 (IgE receptor), Mapatumumab (anti-TRAIL-R1), Maslimomab (anti- T-cell receptor), Matuzumab (anti-EGFR), Mepolizumab (Bosatria, anti-IL-5), Metelimumab (anti-TGF beta 1), Milatuzumab (anti-CD74), Minretumomab (anti-TAG-72), Mitumomab (BEC-2, anti-GD3 ganglioside), Morolimumab (anti-Rhesus factor), Motavizumab (Numax, anti-respiratory syncytial virus), Muromonab-CD3 (Orthoclone OKT3, anti-CD3), Nacolomab (anti-C242), Naptumomab (anti-5T4), Natalizumab (Tysabri, anti-integrin α₄), Nebacumab (anti-endotoxin), Necitumumab (anti-EGFR), Nerelimomab (anti-TNF-α), Nimotuzumab (Theracim, Theraloc, anti-EGFR), Nofetumomab, Ocrelizumab (anti-CD20), Odulimomab (Afolimomab, anti-LFA-1 (CD11a)), Ofatumumab (Arzerra, anti-CD20), Olaratumab (anti-PDGF-R α), Omalizumab (Xolair, anti-IgE Fc region), Oportuzumab (anti-EpCAM), Oregovomab (OvaRex, anti-CA-125), Otelixizumab (anti-CD3), Pagibaximab (anti-lipoteichoic acid), Palivizumab (Synagis, Abbosynagis, anti-respiratory syncytial virus), Panitumumab (Vectibix, ABX-EGF, anti-EGFR), Panobacumab (anti*-Pseudomonas aeruginosa*)*,* Pascolizumab (anti-IL-4), Pemtumomab (Theragyn, anti-MUC1), Pertuzumab (Omnitarg, 2C4, anti-HER2/neu), Pexelizumab (anti-C5), Pintumomab (anti-adenocarcinoma antigen), Priliximab (anti-CD4), Pritumumab (anti-vimentin), PRO 140 (anti-CCR5), Racotumomab (1E10, anti-(N-glycolylneuraminic acid (NeuGc, NGNA)-gangliosides GM3)), Rafivirumab (anti-rabies virus glycoprotein), Ramucirumab (anti-VEGFR2), Ranibizumab (Lucentis, anti-VEGF-A), Raxibacumab (anti-anthrax toxin, protective antigen), Regavirumab (anti-cytomegalovirus glycoprotein B), Reslizumab (anti-IL-5), Rilotumumab (anti-HGF), Rituximab (MabThera, Rituxanmab, anti-CD20), Robatumumab (anti-IGF-1 receptor), Rontalizumab (anti-IFN-α), Rovelizumab (LeukArrest, anti-CD11, CD18), Ruplizumab (Antova, anti-CD154 (CD40L)), Satumomab (anti-TAG-72), Sevirumab (anti-cytomegalovirus), Sibrotuzumab (anti-FAP), Sifalimumab (anti-IFN-α), Siltuximab (anti-IL-6), Siplizumab (anti-CD2), (Smart) MI95 (anti-CD33), Solanezumab (anti-beta amyloid), Sonepcizumab (anti-sphingosine-1-phosphate), Sontuzumab (anti-episialin), Stamulumab (anti-myostatin), Sulesomab (LeukoScan, (anti-NCA-90 (granulocyte antigen), Tacatuzumab (anti-alpha-fetoprotein), Tadocizumab (anti-integrin α_{IIb}β₃), Talizumab (anti-IgE), Tanezumab (anti-NGF), Taplitumomab (anti-CD19), Tefibazumab (Aurexis, (anti-clumping factor A), Telimomab, Tenatumomab (anti-tenascin C), Teneliximab (anti-CD40), Teplizumab (anti-CD3), TGN1412 (anti-CD28), Ticilimumab (Tremelimumab, (anti-CTLA-4), Tigatuzumab (anti-TRAIL-R2), TNX-650 (anti-IL-13), Tocilizumab (Atlizumab, Actemra, RoActemra, (anti-IL-6 receptor), Toralizumab (anti-CD154 (CD40L)), Tositumomab (anti-CD20), Trastuzumab (Herceptin, (anti-HER2/neu), Tremelimumab (anti-CTLA-4), Tucotuzumab celmoleukin (anti-EpCAM), Tuvirumab (anti-hepatitis B virus), Urtoxazumab (anti- *Escherichia coli*)*,* Ustekinumab (Stelara, anti-IL-12, IL-23), Vapaliximab (anti-AOC3 (VAP-1)), Vedolizumab, (anti-integrin α₄β₇), Veltuzumab (anti-CD20), Vepalimomab (anti-AOC3 (VAP-1), Visilizumab (Nuvion, anti-CD3), Vitaxin (anti-vascular integrin avb3), Volociximab (anti-integrin α₅β₁), Votumumab (HumaSPECT, anti-tumor antigen CTAA16.88), Zalutumumab (HuMax-EGFr, (anti-EGFR), Zanolimumab (HuMax-CD4, anti-CD4), Ziralimumab (anti-CD147 (basigin)), Zolimomab (anti-CD5), Etanercept (Enbrel^{®}), Alefacept (Amevive^{®}), Abatacept (Orencia^{®}), Rilonacept (Arcalyst), 14F7 [anti-IRP-2 (Iron Regulatory Protein 2)], 14G2a (anti-GD2 ganglioside, from Nat. Cancer Inst. for melanoma and solid tumors), J591 (anti-PSMA, Weill Cornell Medical School for prostate cancers), 225.28S [anti-HMW-MAA (High molecular weight-melanoma-associated antigen), Sorin Radiofarmaci S.R.L. (Milan, Italy) for melanoma], COL-1 (anti-CEACAM3, CGM1, from Nat. Cancer Inst. USA for colorectal and gastric cancers), CYT-356 (Oncoltad^{®}, for prostate cancers), HNK20 (OraVax Inc. for respiratory syncytial virus), ImmuRAIT (from Immunomedics for NHL), Lym-1 (anti-HLA-DR10, Peregrine Pharm. for Cancers), MAK-195F [anti-TNF (tumor necrosis factor; TNFA, TNF-alpha; TNFSF2), from Abbott / Knoll for Sepsis toxic shock], MEDI-500 [T10B9, anti-CD3, TRαβ (T cell receptor alpha/beta), complex, from MedImmune Inc for Graft-versus-host disease], RING SCAN [ anti-TAG 72 (tumour associated glycoprotein 72), from Neoprobe Corp. for Breast, Colon and Rectal cancers], Avicidin (anti-EPCAM (epithelial cell adhesion molecule), anti-TACSTD1 (Tumor-associated calcium signal transducer 1), anti-GA733-2 (gastrointestinal tumor-associated protein 2), anti-EGP-2 (epithelial glycoprotein 2); anti-KSA; KS1/4 antigen; M4S; tumor antigen 17-1A; CD326, from NeoRx Corp. for Colon, Ovarian, Prostate cancers and NHL]; LymphoCide (Immunomedics, NJ), Smart ID10 (Protein Design Labs), Oncolym (Techniclone Inc, CA), Allomune (BioTransplant, CA), anti-VEGF (Genentech, CA); CEAcide (Immunomedics, NJ), IMC-1C11 (ImClone Systems, NJ) and Cetuximab (ImClone, NJ) .

Other antibodies as cell binding molecules/ligands include, but are not limited to, are antibodies against the following antigens: Aminopeptidase N (CD13), Annexin A1, B7-H3 (CD276, various cancers), CA125 (ovarian), CA15-3 (carcinomas), CA19-9 (carcinomas), L6 (carcinomas), Lewis Y (carcinomas), Lewis X (carcinomas), alpha fetoprotein (carcinomas), CA242 (colorectal), placental alkaline phosphatase (carcinomas), prostate specific antigen (prostate), prostatic acid phosphatase (prostate), epidermal growth factor (carcinomas), CD2 (Hodgkin's disease, NHL lymphoma, multiple myeloma), CD3 epsilon (T cell lymphoma, lung, breast, gastric, ovarian cancers, autoimmune diseases, malignant ascites), CD19 (B cell malignancies), CD20 (non-Hodgkin's lymphoma), CD22 (leukemia, lymphoma, multiple myeloma, SLE), CD30 (Hodgkin's lymphoma), CD33 (leukemia, autoimmune diseases), CD38 (multiple myeloma), CD40 (lymphoma, multiple myeloma, leukemia (CLL)), CD51 (Metastatic melanoma, sarcoma), CD52 (leukemia), CD56 (small cell lung cancers, ovarian cancer, Merkel cell carcinoma, and the liquid tumor, multiple myeloma), CD66e (cancers), CD70 (metastatic renal cell carcinoma and non-Hodgkin lymphoma), CD74 (multiple myeloma), CD80 (lymphoma), CD98 (cancers), mucin (carcinomas), CD221 (solid tumors), CD227 (breast, ovarian cancers), CD262 (NSCLC and other cancers), CD309 (ovarian cancers), CD326 (solid tumors), CEACAM3 (colorectal, gastric cancers), CEACAM5 (carcinoembryonic antigen; CEA, CD66e) (breast, colorectal and lung cancers), DLL4 (delta-like-4), EGFR (Epidermal Growth Factor Receptor, various cancers), CTLA4 (melanoma), CXCR4 (CD184, Heme-oncology, solid tumors), Endoglin (CD105, solid tumors), EPCAM (epithelial cell adhesion molecule, bladder, head, neck, colon, NHL prostate, and ovarian cancers), ERBB2 (Epidermal Growth Factor Receptor 2; lung, breast, prostate cancers), FCGR1 (autoimmune diseases), FOLR (folate receptor, ovarian cancers), GD2 ganglioside (cancers), G-28 (a cell surface antigen glyvolipid, melanoma), GD3 idiotype (cancers), Heat shock proteins (cancers), HER1 (lung, stomach cancers), HER2 (breast, lung and ovarian cancers), HLA-DR10 (NHL), HLA-DRB (NHL, B cell leukemia), human chorionic gonadotropin (carcinoma), IGF1R (insulin-like growth factor 1 receptor, solid tumors, blood cancers), IL-2 receptor (interleukin 2 receptor, T-cell leukemia and lymphomas), IL-6R (interleukin 6 receptor, multiple myeloma, RA, Castleman's disease, IL6 dependent tumors), Integrins (αvβ3, α5β1, α6β4, αllβ3, α5β5, αvβ5, for various cancers), MAGE-1 (carcinomas), MAGE-2 (carcinomas), MAGE-3 (carcinomas), MAGE 4 (carcinomas), anti-transferrin receptor (carcinomas), p97 (melanoma), MS4A1 (membrane-spanning 4-domains subfamily A member 1, Non-Hodgkin's B cell lymphoma, leukemia), MUC1 or MUC1-KLH (breast, ovarian, cervix, bronchus and gastrointestinal cancer), MUC16 (CA125) (Ovarian cancers), CEA (colorectal), gp100 (melanoma), MART1 (melanoma), MPG (melanoma), MS4A1 (membrane-spanning 4-domains subfamily A, small cell lung cancers, NHL), Nucleolin, Neu oncogene product (carcinomas), P21 (carcinomas), Paratope of anti-(N-glycolylneuraminic acid, Breast, Melanoma cancers), PLAP-like testicular alkaline phosphatase (ovarian, testicular cancers), PSMA (prostate tumors), PSA (prostate), ROBO4, TAG 72 (tumour associated glycoprotein 72, AML, gastric, colorectal, ovarian cancers), T cell transmembrane protein (cancers), Tie (CD202b), TNFRSF10B (tumor necrosis factor receptor superfamily member 10B, cancers), TNFRSF13B (tumor necrosis factor receptor superfamily member 13B, multiple myeloma, NHL, other cancers, RA and SLE), TPBG (trophoblast glycoprotein, Renal cell carcinoma), TRAIL-R1 (Tumor necrosis apoprosis Inducing ligand Receptor 1,lymphoma, NHL, colorectal, lung cancers), VCAM-1 (CD106, Melanoma), VEGF, VEGF-A, VEGF-2 (CD309) (various cancers). Some other tumor associated antigens recognized by antibodies have been reviewed (Gerber, et al, mAbs 1:3, 247-253 (2009); No-vellino et al, Cancer Immunol Immunother. 54(3), 187-207 (2005). Franke, et al, Cancer Biother Radiopharm. 2000, 15, 459-76).

The cell-binding agents, more preferred antibodies, can be any agents that are able to against tumor cells, virus infected cells, microorganism infected cells, parasite infected cells, autoimmune cells, activated cells, myeloid cells, activated T-cells, B cells, or melanocytes. More specifically the cell binding agents can be any agent/molecule that is able to against any one of the following antigens or receptors: CD3, CD4, CD5, CD6, CD7, CD8, CD9, CD10, CD11a, CD11b, CD11c, CD12w, CD14, CD15, CD16, CDw17, CD18, CD19, CD20, CD21, CD22, CD23, CD24, CD25, CD26, CD27, CD28, CD29, CD30, CD31, CD32, CD33, CD34, CD35, CD36, CD37, CD38, CD39, CD40, CD41, CD42, CD43, CD44, CD45, CD46, CD47, CD48, CD49b, CD49c, CD51, CD52, CD53, CD54, CD55, CD56, CD58, CD59, CD61, CD62E, CD62L, CD62P, CD63, CD66, CD68, CD69, CD70, CD72, CD74, CD79, CD79a, CD79b, CD80, CD81, CD82, CD83, CD86, CD87, CD88, CD89, CD90, CD91, CD95, CD96, CD98, CD100, CD103, CD105, CD106, CD109, CD117, CD120, CD125, CD126, CD127, CD133, CD134, CD135, CD138, CD141, CD142, CD143, CD144, CD147, CD151, CD147, CD152, CD154, CD156, CD158, CD163, CD166, .CD168, CD174, CD180, CD184, CDw186, CD194, CD195, CD200, CD200a, CD200b, CD209, CD221, CD227, CD235a, CD240, CD262, CD271, CD274, CD276 (B7-H3), CD303, CD304, CD309, CD326, 4-1BB, 5AC, 5T4 (Trophoblast glycoprotein, TPBG, 5T4, Wnt-Activated Inhibitory Factor 1 or WAIF1), Adenocarcinoma antigen, AGS-5, AGS-22M6, Activin receptor-like kinase 1, AFP, AKAP-4, ALK, Alpha intergrin, Alpha v beta6, Amino-peptidase N, Amyloid beta, Androgen receptor, Angiopoietin 2, Angiopoietin 3, Annexin A1, Anthrax toxin protective antigen, Anti-transferrin receptor, AOC3 (VAP-1), B7-H3, Bacillus anthracis anthrax, BAFF (B-cell activating factor), B-lymphoma cell, ber-abl, Bombesin, BORIS, C5, C242 antigen, CA125 (carbohydrate antigen 125, MUC16), CA-IX (or CAIX, carbonic anhydrase 9), CALLA, CanAg, Canis lupus familiaris IL31, Carbonic anhydrase IX, Cardiac myosin, CCL11(C-C motif chemokine 11), CCR4 (C-C chemokine receptor type 4, CD194), CCR5, CD3E (epsilon), CEA (Carcinoembryonic antigen), CEACAM3, CEACAM5 (carcinoembryonic antigen), CFD (Factor D), Ch4D5, Cholecystokinin 2 (CCK2R), CLDN18 (Claudin-18), Clumping factor A, CRIPTO, FCSF1R (Colony stimulating factor 1 receptor, CD115), CSF2 (colony stimulating factor 2, Granulocyte-macrophage colony-stimulating factor (GM-CSF)), CTLA4 (cytotoxic T-lymphocyte-associated protein 4), CTAA16.88 tumor antigen, CXCR4 (CD184), C-X-C chemokine receptor type 4, cyclic ADP ribose hydrolase, Cyclin B1, CYP1B1, Cytomegalovirus, Cytomegalovirus glycoprotein B, Dabigatran, DLL4 (delta-like-ligand 4), DPP4 (Dipeptidyl-peptidase 4), DR5 (Death receptor 5), E. coli shiga toxin type-1, E. coli shiga toxin type-2, ED-B, EGFL7 (EGF-like domain-containing protein 7), EGFR, EGFRII, EGFRvIII, Endoglin (CD105), Endothelin B receptor, Endotoxin, EpCAM (epithelial cell adhesion molecule), EphA2, Episialin, ERBB2 (Epidermal Growth Factor Receptor 2), ERBB3, ERG (TMPRSS2 ETS fusion gene), Escherichia coli, ETV6-AML, FAP (Fibroblast activation protein alpha), FCGR1, alpha-Fetoprotein, Fibrin II, beta chain, Fibronectin extra domain-B, FOLR (folate receptor), Folate receptor alpha, Folate hydrolase, Fos-related antigen 1.F protein of respiratory syncytial virus, Frizzled receptor, Fucosyl GM1, GD2 ganglioside, G-28 (a cell surface antigen glyvolipid), GD3 idiotype, GloboH, Glypican 3, N-glycolylneuraminic acid, GM3, GMCSF receptor α-chain, Growth differentiation factor 8, GP100, GPNMB (Transmem-brane glycoprotein NMB), GUCY2C (Guanylate cyclase 2C, guanylyl cyclase C(GC-C), intestinal Guanylate cyclase, Guanylate cyclase-C receptor, Heat-stable entero-toxin receptor (hSTAR)), Heat shock proteins, Hemagglutinin, Hepatitis B surface antigen, Hepatitis B virus, HER1 (human epidermal growth factor receptor 1), HER2, HER2/neu, HER3 (ERBB-3), IgG4, HGF/SF (Hepatocyte growth factor/scatter factor), HHGFR, HIV-1, Histone complex, HLA-DR (human leukocyte antigen), HLA-DR10, HLA-DRB , HMWMAA, Human chorionic gonadotropin, HNGF, Human scatter factor receptor kinase, HPV E6/E7, Hsp90, hTERT, ICAM-1 (Intercellular Adhesion Molecule 1), Idiotype, IGF1R (IGF-1, insulin-like growth factor 1 receptor), IGHE, IFN-y, Influeza hemag-glutinin, IgE, IgE Fc region, IGHE, IL-1, IL-2 receptor (interleukin 2 receptor), IL-4, IL-5, IL-6, IL-6R (interleukin 6 receptor), IL-9, IL-10, IL-12, IL-13, IL-17, IL-17A, IL-20, IL-22, IL-23, IL31RA, ILGF2 (Insulin-like growth factor 2), Integrins (α4, α_{IIb}β₃, αvβ3, α₄β₇, α5β1, α6β4, α7β7, αllβ3, α5β5, αvβ5), Interferon gamma-induced protein, ITGA2, ITGB2, KIR2D, LCK, Le, Legumain, Lewis-Y antigen, LFA-1(Lymphocyte function-associated antigen 1, CD11a), LHRH, LINGO-1, Lipoteichoic acid, LIV1A, LMP2, LTA, MAD-CT-1, MAD-CT-2, MAGE-1, MAGE-2, MAGE-3, MAGE A1, MAGE A3, MAGE 4, MART1, MCP-1, MIF (Macrophage migration inhibitory factor, or glycosylation-inhibiting factor (GIF)), MS4A1 (membrane-spanning 4-domains subfamily A member 1), MSLN (mesothelin), MUC1(Mucin 1, cell surface associated (MUC1) or polymorphic epithelial mucin (PEM)), MUC1-KLH, MUC16 (CA125), MCP1(monocyte chemotactic protein 1), MelanA/MART1, ML-IAP, MPG, MS4A1 (membrane-spanning 4-domains subfamily A), MYCN, Myelin-associated glycoprotein, Myostatin, NA17, NARP-1, NCA-90 (granulocyte antigen), Nectin-4 (ASG-22ME), NGF, Neural apoptosis-regulated proteinase 1, NOGO-A, Notch receptor, Nucleolin, Neu oncogene product, NY-BR-1, NY-ESO-1, OX-40, OxLDL (Oxidized low-density lipoprotein), OY-TES1, P21, p53 nonmutant, P97, Page4, PAP, Paratope of anti-(N-glycolylneuraminic acid), PAX3, PAX5, PCSK9, PDCD1 (PD-1, Programmed cell death protein 1,CD279), PDGF-Rα (Alpha-type platelet-derived growth factor receptor ), PDGFR-β, PDL-1, PLAC1, PLAP-like testicular alkaline phosphatase, Platelet-derived growth factor receptor beta, Phosphate-sodium co-transporter, PMEL 17, Polysialic acid, Proteinase3 (PR1), Prostatic carcinoma, PS (Phosphatidylserine), Prostatic carcinoma cells, Pseudomonas aeruginosa, PSMA, PSA, PSCA, Rabies virus glycoprotein, RHD (Rh polypeptide 1 (RhPI), CD240), Rhesus factor, RANKL, RhoC, Ras mutant, RGS5, ROBO4, Respiratory syncytial virus, RON, Sarcoma translocation breakpoints, SART3, Sclerostin, SLAMF7 (SLAM family member 7), Selectin P, SDC1 (Syndecan 1), sLe(a), Somatomedin C, SIP (Sphingosine-1-phosphate), Somatostatin, Sperm protein 17, SSX2, STEAP1 (six-transmembrane epithelial antigen of the prostate 1), STEAP2, STn, TAG-72 (tumor associated glycoprotein 72), Survivin, T-cell receptor, T cell transmembrane protein, TEM1 (Tumor endothelial marker 1), TENB2, Tenascin C (TN-C), TGF-α, TGF-β (Transforming growth factor beta), TGF-β1, TGF-β2 (Transforming growth factor-beta 2), Tie (CD202b), Tie2, TIM-1 (CDX-014), Tn, TNF, TNF-α, TNFRSF8, TNFRSF10B (tumor necrosis factor receptor superfamily member 10B), TNFRSF13B (tumor necrosis factor receptor superfamily member 13B), TPBG (trophoblast glycoprotein), TRAIL-R1 (Tumor necrosis apoprosis Inducing ligand Receptor 1), TRAILR2 (Death receptor 5 (DR5)), tumor-associated calcium signal transducer 2, tumor specific glycosylation of MUC1, TWEAK receptor, TYRP1(glycoprotein 75), TRP-2, Tyrosinase, VCAM-1 (CD106), VEGF, VEGF-A, VEGF-2 (CD309), VEGFR-1, VEGFR2, or vimentin, WT1, XAGE 1, or cells expressing any insulin growth factor receptors, or any epidermal growth factor receptors.

In another specific embodiment, the cell-binding ligand-drug conjugates via the bridge linkers of this invention are for use in the targeted treatment of cancers. The targeted cancers include, but are not limited, Adrenocortical Carcinoma, Anal Cancer, Bladder Cancer, Brain Tumor (Adult, Brain Stem Glioma, Childhood, Cerebellar Astrocytoma, Cerebral Astrocytoma, Ependymoma, Medulloblastoma, Supratentorial Primitive Neuro-ectodermal and Pineal Tumors, Visual Pathway and Hypothalamic Glioma), Breast Cancer, Carcinoid Tumor, Gastrointestinal, Carcinoma of Unknown Primary, Cervical Cancer, Colon Cancer, Endometrial Cancer, Esophageal Cancer, Extrahepatic Bile Duct Cancer, Ewings Family of Tumors (PNET), Extracranial Germ Cell Tumor, Eye Cancer, Intraocular Melanoma, Gallbladder Cancer, Gastric Cancer (Stomach), Germ Cell Tumor, Extragonadal, Gestational Trophoblastic Tumor, Head and Neck Cancer, Hypopharyngeal Cancer, Islet Cell Carcinoma, Kidney Cancer (renal cell cancer), Laryngeal Cancer, Leukemia (Acute Lymphoblastic, Acute Myeloid, Chronic Lymphocytic, Chronic Myelogenous, Hairy Cell), Lip and Oral Cavity Cancer, Liver Cancer, Lung Cancer (Non-Small Cell, Small Cell, Lymphoma (AIDS-Related, Central Nervous System, Cutaneous T-Cell, Hodgkin's Disease, Non-Hodgkin's Disease, Malignant Mesothelioma, Melanoma, Merkel Cell Carcinoma, Metasatic Squamous Neck Cancer with Occult Primary, Multiple Myeloma, and Other Plasma Cell Neoplasms, Mycosis Fungoides, Myelodysplastic Syndrome, Myeloproli-ferative Disorders, Nasopharyngeal Cancer, Neuroblastoma, Oral Cancer, Oropharyngeal Cancer, Osteosarcoma, Ovarian Cancer (Epithelial, Germ Cell Tumor, Low Malignant Potential Tumor), Pancreatic Cancer (Exocrine, Islet Cell Carcinoma), Paranasal Sinus and Nasal Cavity Cancer, Parathyroid Cancer, Penile Cancer, Pheochromocytoma Cancer, Pituitary Cancer, Plasma Cell Neoplasm, Prostate Cancer Rhabdomyosarcoma, Rectal Cancer, Renal Cell Cancer (kidney cancer), Renal Pelvis and Ureter (Transitional Cell), Salivary Gland Cancer, Sezary Syndrome, Skin Cancer, Skin Cancer (Cutaneous T-Cell Lymphoma, Kaposi's Sarcoma, Melanoma), Small Intestine Cancer, Soft Tissue Sarcoma, Stomach Cancer, Testicular Cancer, Thymoma (Malignant), Thyroid Cancer, Urethral Cancer, Uterine Cancer (Sarcoma), Unusual Cancer of Childhood, Vaginal Cancer, Vulvar Cancer, Wilms' Tumor.

In another specific embodiment, the cell-binding-drug conjugates via the bridge likers of this invention are used in accordance with the compositions and for use in methods for the treatment or prevention of an autoimmune disease. The autoimmune diseases include, but are not limited, Achlorhydra Autoimmune Active Chronic Hepatitis, Acute Disseminated Encephalomyelitis, Acute hemorrhagic leukoencephalitis, Addison's Disease, Agamma-globulinemia, Alopecia areata, Amyotrophic Lateral Sclerosis, Ankylosing Spondylitis, Anti-GBM/TBM Nephritis, Antiphospholipid syndrome, Antisynthetase syndrome, Arthritis, Atopic allergy, Atopic Dermatitis, Autoimmune Aplastic Anemia, Autoimmune cardiomyopathy, Autoimmune hemolytic anemia, Autoimmune hepatitis, Autoimmune inner ear disease, Autoimmune lymphoproliferative syndrome, Autoimmune peripheral neuropathy, Autoimmune pancreatitis, Autoimmune polyendocrine syndrome Types I, II, & III, Autoimmune progesterone dermatitis, Autoimmune thrombocytopenic purpura, Autoimmune uveitis, Balo disease/Balo concentric sclerosis, Bechets Syndrome, Berger's disease, Bickerstaff s encephalitis, Blau syndrome, Bullous Pemphigoid, Castleman's disease, Chagas disease, Chronic Fatigue Immune Dysfunction Syndrome, Chronic inflammatory demyelinating polyneuropathy, Chronic recurrent multifocal ostomyelitis, Chronic lyme disease, Chronic obstructive pulmonary disease, Churg-Strauss syndrome, Cicatricial Pemphigoid, Coeliac Disease, Cogan syndrome, Cold agglutinin disease, Complement component 2 deficiency, Cranial arteritis, CREST syndrome, Crohns Disease (a type of idiopathic inflammatory bowel diseases), Cushing's Syndrome, Cutaneous leukocytoclastic angiitis, Dego's disease, Dercum's disease, Dermatitis herpetiformis, Dermatomyositis, Diabetes mellitus type 1, Diffuse cutaneous systemic sclerosis, Dressler's syndrome, Discoid lupus erythematosus, Eczema, Endometriosis, Enthesitis-related arthritis, Eosino-philic fasciitis, Epidermolysis bullosa acquisita, Erythema nodosum, Essential mixed cryoglobulinemia, Evan's syndrome, Fibrodysplasia ossificans progressiva, Fibromyalgia, Fibromyositis, Fibrosing aveolitis, Gastritis, Gastrointestinal pemphigoid, Giant cell arteritis, Glomerulonephritis, Goodpasture's syndrome, Graves' disease, Guillain-Barré syndrome, Hashimoto's encephalitis, Hashimoto's thyroiditis, Haemolytic anaemia, Henoch-Schonlein purpura, Herpes gestationis, Hidradenitis suppurativa, Hughes syndrome (See Antiphospholipid syndrome), Hypogamma-globulinemia, Idiopathic Inflammatory Demyelinating Diseases, Idiopathic pulmonary fibrosis, Idiopathic thrombocytopenic purpura (See Autoimmune thrombocytopenic purpura), IgA nephropathy (Also Berger's disease), Inclusion body myositis, Inflammatory demyelinating polyneuopathy, Interstitial cystitis, Irritable Bowel Syndrome , Juvenile idiopathic arthritis, Juvenile rheumatoid arthritis, Kawasaki's Disease, Lambert-Eaton myasthenic syndrome, Leukocyto-clastic vasculitis, Lichen planus, Lichen sclerosus, Linear IgA disease (LAD), Lou Gehrig's Disease (Also Amyotrophic lateral sclerosis), Lupoid hepatitis, Lupus erythematosus, Majeed syndrome, Ménière's disease, Microscopic polyangiitis, Miller-Fisher syndrome, Mixed Connective Tissue Disease, Morphea, Mucha-Habermann disease, Muckle-Wells syndrome, Multiple Myeloma, Multiple Sclerosis, Myasthenia gravis, Myositis, Narcolepsy, Neuromyelitis optica (Devic's Disease), Neuromyotonia, Occular cicatricial pemphi-goid, Opsoclonus myoclonus syndrome, Ord thyroiditis, Palindromic rheumatism, PANDAS (Pediatric Autoimmune Neuropsychiatric Disorders Associated with Streptococcus), Paraneoplastic cerebellar degeneration, Paroxysmal nocturnal hemoglobinuria, Parry Romberg syndrome, Parsonnage-Turner syndrome, Pars planitis, Pemphigus, Pemphigus vulgaris, Pernicious anaemia, Perivenous encephalomyelitis, POEMS syndrome, Polyarteritis nodosa, Polymyalgia rheumatica, Polymyositis, Primary biliary cirrhosis, Primary sclerosing cholangitis, Progressive inflammatory neuropathy, Psoriasis, Psoriatic Arthritis, Pyoderma gangrenosum, Pure red cell aplasia, Rasmussen's encephalitis, Raynaud phenomenon, Relapsing polychondritis, Reiter's syndrome, Restless leg syndrome, Retroperitoneal fibrosis, Rheumatoid arthritis, Rheumatoid fever, Sarcoidosis, Schizophrenia, Schmidt syndrome, Schnitzler syndrome, Scleritis, Scleroderma, Sjögren's syndrome, Spondyloarthropathy, Sticky blood syndrome, Still's Disease, Stiff person syndrome, Subacute bacterial endocarditis, Susac's syndrome, Sweet syndrome, Sydenham Chorea, Sympathetic ophthalmia, Takayasu's arteritis, Temporal arteritis (giant cell arteritis), Tolosa-Hunt syndrome, Transverse Myelitis, Ulcerative Colitis (a type of idiopathic inflammatory bowel diseases), Undifferentiated connective tissue disease, Undifferentiated spondyloarthropathy, Vasculitis, Vitiligo, Wegener's granulomatosis, Wilson's syndrome, Wiskott-Aldrich syndrome

In another specific embodiment, a binding molecule used for the conjugate via the bridge linkers of this invention for use in the treatment or prevention of an autoimmune disease can be, but are not limited to, anti-elastin antibody; Abys against epithelial cells antibody; Anti-Basement Membrane Collagen Type IV Protein antibody; Anti-Nuclear Antibody; Anti ds DNA; Anti ss DNA, Anti Cardiolipin Antibody IgM, IgG; anti-celiac antibody; Anti Phospholipid Antibody IgK, IgG; Anti SM Antibody; Anti Mitochondrial Antibody; Thyroid Antibody; Microsomal Antibody, T-cells antibody; Thyroglobulin Antibody, Anti SCL-70; Anti-Jo; Anti-U.sub.1RNP; Anti-La/SSB; Anti SSA; Anti SSB; Anti Perital Cells Antibody; Anti Histones; Anti RNP; C-ANCA; P-ANCA; Anti centromere; Anti-Fibrillarin, and Anti GBM Antibody, Anti-ganglioside antibody; Anti-Desmogein 3 antibody; Anti-p62 antibody; Anti-sp100 antibody; Anti-Mitochondrial(M2) antibody; Rheumatoid factor antibody; Anti-MCV antibody; Anti-topoisomerase antibody; Anti-neutrophil cytoplasmic(cANCA) antibody.

In certain preferred embodiments, the binding molecule for the conjugate in the present invention, can bind to both a receptor and a receptor complex expressed on an activated lymphocyte which is associated with an autoimmune disease. The receptor or receptor complex can comprise an immunoglobulin gene superfamily member (e.g. CD2, CD3, CD4, CD8, CD19, CD20, CD22, CD28, CD30, CD33, CD37, CD38, CD56, CD70, CD79, CD79b, CD90, CD125, CD147, CD152/CTLA-4, PD-1, or ICOS), a TNF receptor superfamily member (e.g. CD27, CD40, CD95/Fas, CD134/OX40, CD137/4-1BB, INF-R1, TNFR-2, RANK, TACI, BCMA, osteoprotegerin, Apo2/TRAIL-R1, TRAEL-R2, TRAIL-R3, TRAIL-R4, and APO-3), an integrin, a cytokine receptor, a chemokine receptor, a major histocompatibility protein, a lectin (C-type, S-type, or I-type), or a complement control protein.

In another specific embodiment, useful cell binding ligands that are immunospecific for a viral or a microbial antigen are humanized or human monoclonal antibodies. As used herein, the term "viral antigen" includes, but is not limited to, any viral peptide, polypeptide protein (e.g. HIV gp120, HIV nef, RSV F glycoprotein, influenza virus neuramimidase, influenza virus hemagglutinin, HTLV tax, herpes simplex virus glycoprotein (e.g. gB, gC, gD, and gE) and hepatitis B surface antigen) that is capable of eliciting an immune response. As used herein, the term "microbial antigen" includes, but is not limited to, any microbial peptide, polypeptide, protein, saccharide, polysaccharide, or lipid molecule (e.g., a bacteria, fungi, pathogenic protozoa, or yeast polypeptides including, e.g., LPS and capsular polysaccharide 5/8) that is capable of eliciting an immune response. Examples of antibodies available l for the viral or microbial infection include, but are not limited to, Palivizumab which is a humanized anti-respiratory syncytial virus monoclonal antibody for use in the treatment of RSV infection; PRO542 which is a CD4 fusion antibody for use in the treatment of HIV infection; Ostavir which is a human antibody for use in the treatment of hepatitis B virus; PROTVIR which is a humanized IgG.sub.1 antibody for use in the treatment of cytomegalovirus; and anti-LPS antibodies.

The cell binding molecules-drug conjugates via the bridge linkers of this invention can be for use in the treatment of infectious diseases. These infectious diseases include, but are not limited to, Acinetobacter infections, Actinomycosis, African sleeping sickness (African trypanosomiasis), AIDS (Acquired immune deficiency syndrome), Amebiasis, Anaplasmosis, Anthrax, Arcanobacterium haemolyticum infection, Argentine hemorrhagic fever, Ascariasis, Aspergillosis, Astrovirus infection, Babesiosis, Bacillus cereus infection, Bacterial pneumonia, Bacterial vaginosis, Bacteroides infection, Balantidiasis, Baylisascaris infection, BK virus infection, Black piedra, Blastocystis hominis infection, Blastomycosis, Bolivian hemorrhagic fever, Borrelia infection, Botulism (and Infant botulism), Brazilian hemorrhagic fever, Brucellosis, Burkholderia infection, Buruli ulcer, Calicivirus infection (Norovirus and Sapovirus), Campylobacteriosis, Candidiasis (Moniliasis; Thrush), Cat-scratch disease, Cellulitis, Chagas Disease (American trypanosomiasis), Chancroid, Chickenpox, Chlamydia, Chlamydophila pneumoniae infection, Cholera, Chromoblastomycosis, Clonorchiasis, Clostridium difficile infection, Coccidioidomycosis, Colorado tick fever, Common cold (Acute viral rhinopharyngitis; Acute coryza), Creutzfeldt-Jakob disease, Crimean-Congo hemorrhagic fever, Cryptococ-cosis, Cryptosporidiosis, Cutaneous larva migrans, Cyclosporiasis, Cysticercosis, Cytomegalovirus infection, Dengue fever, Dientamoebiasis, Diphtheria, Diphyllobothriasis, Dracunculiasis, Ebola hemorrhagic fever, Echinococcosis, Ehrlichiosis, Enterobiasis (Pinworm infection), Enterococcus infection, Enterovirus infection, Epidemic typhus, Erythema infectiosum (Fifth disease), Exanthem subitum, Fasciolopsiasis, Fasciolosis, Fatal familial insomnia, Filariasis, Food poisoning by Clostridium perfringens, Free-living amebic infection, Fusobacterium infection, Gas gangrene (Clostridial myonecrosis), Geotrichosis, Gerstmann-Sträussler-Scheinker syndrome, Giardiasis, Glanders, Gnathosto-miasis, Gonorrhea, Granuloma inguinale (Donovanosis), Group A streptococcal infection, Group B streptococcal infection, Haemophilus influenzae infection, Hand, foot and mouth disease (HFMD), Hantavirus Pulmonary Syndrome, Helicobacter pylori infection, Hemolyticuremic syndrome, Hemorrhagic fever with renal syndrome, Hepatitis A, Hepatitis B, Hepatitis C, Hepatitis D, Hepatitis E, Herpes simplex, Histoplasmosis, Hookworm infection, Human bocavirus infection, Human ewingii ehrlichiosis, Human granulocytic anaplasmosis, Human metapneumovirus infection, Human monocytic ehrlichiosis, Human papillomavirus infection, Human parainfluenza virus infection, Hymenolepiasis, Epstein-Barr Virus Infectious Mononucleosis (Mono), Influenza, Isosporiasis, Kawasaki disease, Keratitis, Kingella kingae infection, Kuru, Lassa fever, Legionellosis (Legionnaires' disease), Legionellosis (Pontiac fever), Leishmaniasis, Leprosy, Leptospirosis, Listeriosis, Lyme disease (Lyme borreliosis), Lymphatic filariasis (Elephantiasis), Lymphocytic choriomeningitis, Malaria, Marburg hemorrhagic fever, Measles, Melioidosis (Whitmore's disease), Meningitis, Meningococcal disease, Metagonimiasis, Microsporidiosis, Molluscum contagiosum, Mumps, Murine typhus (Endemic typhus), Mycoplasma pneumonia, Mycetoma, Myiasis, Neonatal conjunctivitis (Ophthalmia neonatorum), (New) Variant Creutzfeldt-Jakob disease (vCJD, nvCJD), Nocardiosis, Onchocerciasis (River blindness), Paracoccidioidomycosis (South American blastomycosis), Paragonimiasis, Pasteurellosis, Pediculosis capitis (Head lice), Pediculosis corporis (Body lice), Pediculosis pubis (Pubic lice, Crab lice), Pelvic inflammatory disease, Pertussis (Whooping cough), Plague, Pneumococcal infection, Pneumocystis pneumonia, Pneumonia, Poliomyelitis, Prevotella infection, Primary amoebic meningoencephalitis, Progressive multifocal leukoencephalopathy, Psittacosis, Q fever, Rabies, Rat-bite fever, Respiratory syncytial virus infection, Rhinosporidiosis, Rhinovirus infection, Rickettsial infection, Rickettsial-pox, Rift Valley fever, Rocky mountain spotted fever, Rotavirus infection, Rubella, Salmonellosis, SARS (Severe Acute Respiratory Syndrome), Scabies, Schistosomiasis, Sepsis, Shigellosis (Bacillary dysentery), Shingles (Herpes zoster), Small-pox (Variola), Sporotrichosis, Staphylococcal food poisoning, Staphylococcal infection, Strongyloidiasis, Syphilis, Taeniasis, Tetanus (Lockjaw), Tinea barbae (Barber's itch), Tinea capitis (Ringworm of the Scalp), Tinea corporis (Ringworm of the Body), Tinea cruris (Jock itch), Tinea manuum (Ringworm of the Hand), Tinea nigra, Tinea pedis (Athlete's foot), Tinea unguium (Onychomycosis), Tinea versicolor (Pityriasis versicolor), Toxocariasis (Ocular Larva Migrans), Toxocariasis (Visceral Larva Migrans), Toxoplasmosis, Trichinellosis, Trichomoniasis, Trichuriasis (Whipworm infection), Tuberculosis, Tularemia, Ureaplasma urealyticum infection, Venezuelan equine encephalitis, Venezuelan hemorrhagic fever, Viral pneumonia, West Nile Fever, White piedra (Tinea blanca), Yersinia pseudotuber-culosis infection, Yersiniosis, Yellow fever, Zygomycosis.

The cell binding molecule, which is more preferred to be an antibody described in this patent that are against pathogenic strains include, but are not limit, Acinetobacter baumannii, Actinomyces israelii, Actinomyces gerencseriae and Propionibacterium propionicus, Trypanosoma brucei, HIV (Human immunodeficiency virus), Entamoeba histolytica, Anaplasma genus, Bacillus anthracis, Arcanobacterium haemolyticum, Junin virus, Ascaris lumbricoides, Aspergillus genus, Astroviridae family, Babesia genus, Bacillus cereus, multiple bacteria, Bacteroides genus, Balantidium coli, Baylisascaris genus, BK virus, Piedraia hortae, Blastocystis hominis, Blastomyces dermatitides, Machupo virus, Borrelia genus, Clostridium botulinum, Sabia, Brucella genus, usually Burkholderia cepacia and other Burkholderia species, Mycobacterium ulcerans, Caliciviridae family, Campylobacter genus, usually Candida albicans and other Candida species, Bartonella henselae, Group A Streptococcus and Staphylococcus, Trypanosoma cruzi, Haemophilus ducreyi, Varicella zoster virus (VZV), Chlamydia trachomatis, Chlamydophila pneumoniae, Vibrio cholerae, Fonsecaea pedrosoi, Clonorchis sinensis, Clostridium difficile, Coccidioides immitis and Coccidioides posadasii, Colorado tick fever virus, rhinoviruses, coronaviruses, CJD prion, Crimean-Congo hemorrhagic fever virus, Cryptococcus neoformans, Cryptosporidium genus, Ancylostoma braziliense; multiple parasites, Cyclospora cayetanensis, Taenia solium, Cytomegalovirus, Dengue viruses (DEN-1, DEN-2, DEN-3 and DEN-4) - Flaviviruses, Dientamoeba fragilis, Corynebacterium diphtheriae, Diphyllobothrium, Dracunculus medinensis, Ebolavirus, Echinococcus genus, Ehrlichia genus, Enterobius vermicularis, Enterococcus genus, Enterovirus genus, Rickettsia prowazekii, Parvovirus B19, Human herpesvirus 6 and Human herpesvirus 7, Fasciolopsis buski, Fasciola hepatica and Fasciola gigantica, FFI prion, Filarioidea superfamily, Clostridium perfringens, Fusobacterium genus, Clostridium perfringens; other Clostridium species, Geotrichum candidum, GSS prion, Giardia intestinalis, Burkholderia mallei, Gnathostoma spinigerum and Gnathostoma hispidum, Neisseria gonorrhoeae, Klebsiella granulomatis, Streptococcus pyogenes, Streptococcus agalactiae, Haemophilus influenzae, Enteroviruses, mainly Coxsackie A virus and Enterovirus 71, Sin Nombre virus, Helicobacter pylori, Escherichia coli O157:H7, Bunyaviridae family, Hepatitis A Virus, Hepatitis B Virus, Hepatitis C Virus, Hepatitis D Virus, Hepatitis E Virus, Herpes simplex virus 1, Herpes simplex virus 2, Histoplasma capsulatum, Ancylostoma duodenale and Necator americanus, Hemophilus influenzae, Human bocavirus, Ehrlichia ewingii, Anaplasma phagocytophilum, Human metapneumovirus, Ehrlichia chaffeensis, Human papillomavirus, Human parainfluenza viruses, Hymenolepis nana and Hymenolepis diminuta, Epstein-Barr Virus, Orthomy-xoviridae family, Isospora belli, Kingella kingae, Klebsiella pneumoniae, Klebsiella ozaenas, Klebsiella rhinoscleromotis, Kuru prion, Lassa virus, Legionella pneumophila, Legionella pneumophila, Leishmania genus, Mycobacterium leprae and Mycobacterium lepromatosis, Leptospira genus, Listeria monocytogenes, Borrelia burgdorferi and other Borrelia species, Wuchereria bancrofti and Brugia malayi, Lymphocytic choriomeningitis virus (LCMV), Plasmodium genus, Marburg virus, Measles virus, Burkholderia pseudomallei, Neisseria meningitides, Metagonimus yokagawai, Microsporidia phylum, Molluscum contagiosum virus (MCV), Mumps virus, Rickettsia typhi, Mycoplasma pneumoniae, numerous species of bacteria (Actinomycetoma) and fungi (Eumycetoma), parasitic dipterous fly larvae, Chlamydia trachomatis and Neisseria gonorrhoeae, vCJD prion, Nocardia asteroides and other Nocardia species, Onchocerca volvulus, Paracoccidioides brasiliensis, Paragonimus westermani and other Paragonimus species, Pasteurella genus, Pediculus humanus capitis, Pediculus humanus corporis, Phthirus pubis, Bordetella pertussis, Yersinia pestis, Streptococcus pneumoniae, Pneumocystis jirovecii, Poliovirus, Prevotella genus, Naegleria fowleri, JC virus, Chlamydophila psittaci, Coxiella burnetii, Rabies virus, Streptobacillus moniliformis and Spirillum minus, Respiratory syncytial virus, Rhinosporidium seeberi, Rhinovirus, Rickettsia genus, Rickettsia akari, Rift Valley fever virus, Rickettsia rickettsii, Rotavirus, Rubella virus, Salmonella genus, SARS coronavirus, Sarcoptes scabiei, Schistosoma genus, Shigella genus, Varicella zoster virus, Variola major or Variola minor, Sporothrix schenckii, Staphylococcus genus, Staphylococcus genus, Staphylococcus aureus, Streptococcus pyogenes, Strongyloides stercoralis, Treponema pallidum, Taenia genus, Clostridium tetani, Trichophyton genus, Trichophyton tonsurans, Trichophyton genus, Epidermophyton floccosum, Trichophyton rubrum, and Trichophyton mentagrophytes, Trichophyton rubrum, Hortaea werneckii, Trichophyton genus, Malassezia genus, Toxocara canis or Toxocara cati, Toxoplasma gondii, Trichinella spiralis, Trichomonas vaginalis, Trichuris trichiura, Mycobacterium tuberculosis, Francisella tularensis, Ureaplasma urealyticum, Venezuelan equine encephalitis virus, Vibrio colerae, Guanarito virus, West Nile virus, Trichosporon beigelii, Yersinia pseudotuberculosis, Yersinia enterocolitica, Yellow fever virus, Mucorales order (Mucormycosis) and Entomophthorales order (Entomophthora-mycosis), Pseudomonas aeruginosa, Campylobacter (Vibrio) fetus, Aeromonas hydrophila, Edwardsiella tarda, Yersinia pestis, Shigella dysenteriae, Shigella flexneri, Shigella sonnei, Salmonella typhimurium, Treponema pertenue, Treponema carateneum, Borrelia vincentii, Borrelia burgdorferi, Leptospira icterohemorrhagiae, Pneumocystis carinii, Brucella abortus, Brucella suis, Brucella melitensis, Mycoplasma spp., Rickettsia prowazeki, Rickettsia tsutsugumushi, Clamydia spp.; pathogenic fungi (Aspergillus fumigatus, Candida albicans, Histoplasma capsulatum); protozoa (Entomoeba histolytica, Trichomonas tenas, Trichomonas hominis, Tryoanosoma gambiense, Trypanosoma rhodesiense, Leishmania donovani, Leishmania tropica, Leishmania braziliensis, Pneumocystis pneumonia, Plasmodium vivax, Plasmodium falciparum, Plasmodium malaria); or Helminiths (Schistosoma japonicum, Schistosoma mansoni, Schistosoma haematobium, and hookworms).

Other antibodies as cell binding ligands used in this invention for use in the treatment of viral disease include, but are not limited to, antibodies against antigens of pathogenic viruses, including as examples and not by limitation: Poxyiridae, Herpesviridae, Adenoviridae, Papovaviridae, Enteroviridae, Picornaviridae, Parvoviridae, Reoviridae, Retroviridae, influenza viruses, parainfluenza viruses, mumps, measles, respiratory syncytial virus, rubella, Arboviridae, Rhabdoviridae, Arenaviridae, Non-A/Non-B Hepatitis virus, Rhinoviridae, Coronaviridae, Rotoviridae, Oncovirus [such as, HBV (Hepatocellular carcinoma), HPV (Cervical cancer, Anal cancer), Kaposi's sarcoma-associated herpesvirus (Kaposi's sarcoma), Epstein-Barr virus (Nasopharyngeal carcinoma, Burkitt's lymphoma, Primary central nervous system lymphoma), MCPyV (Merkel cell cancer), SV40 (*Simian virus 40),* HCV (Hepatocellular carcinoma), HTLV-I (Adult T-cell leukemia/lymphoma)], Immune disorders caused virus: [such as Human Immunodeficiency Virus (AIDS)]; Central nervous system virus: [such as, JCV (Progressive multifocal leukoencephalo-pathy), MeV (Subacute sclerosing panencephalitis), LCV (Lymphocytic choriomeningitis), Arbovirus encephalitis, Orthomyxoviridae (probable) (Encephalitis lethargica), RV (Rabies), Chandipura virus, Herpesviral meningitis, Ramsay Hunt syndrome type II; Poliovirus (Poliomyelitis, Post-polio syndrome), HTLV-I (Tropical spastic paraparesis)]; Cytomegalovirus (Cytomegalovirus retinitis, HSV (Herpetic keratitis)); Cardiovascular virus [such as CBV (Pericarditis, Myocarditis)]; Respiratory system/acute viral nasopharyngitis/viral pneumonia: [Epstein-Barr virus (EBV infection/Infectious mononucleosis), Cytomegalovirus; SARS coronavirus (Severe acute respiratory syndrome) Orthomyxoviridae: Influenzavirus A/B/C (Influenza/Avian influenza), Paramyxovirus: Human parainfluenza viruses (Parainfluenza), RSV (Human respiratory syncytial virus), hMPV]; Digestive system virus [MuV (Mumps), Cytomegalovirus (Cytomegalovirus esophagitis); Adenovirus (Adenovirus infection); Rotavirus, Norovirus, Astrovirus, Coronavirus; HBV (Hepatitis B virus), CBV, HAV (Hepatitis A virus), HCV (Hepatitis C virus), HDV (Hepatitis D virus), HEV (Hepatitis E virus), HGV (Hepatitis G virus)]; Urogenital virus [such as, BK virus, MuV (Mumps)].

According to a further object, the present invention also concerns pharmaceutical compositions comprising the conjugate via the bridge linkers of the invention together with a pharmaceutically acceptable carrier, diluent, or excipient for use in the treatment of cancers, infections or autoimmune disorders. The method for treatment of cancers, infections and autoimmune disorders can be practiced *in vitro, in vivo,* or *ex vivo.* Examples of *in vitro* uses include treatments of cell cultures in order to kill all cells except for desired variants that do not express the target antigen; or to kill variants that express undesired antigen. Examples of *ex vivo* uses include pharmaceutical compositions for use in the treatments of hematopoietic stem cells (HSC) prior to the performance of the transplantation (HSCT) into the same patient in order to kill diseased or malignant cells. For instance, clinical *ex vivo* treatment to remove tumour cells or lymphoid cells from bone marrow prior to autologous transplantation in cancer treatment or in treatment of autoimmune disease, or to remove T cells and other lymphoid cells from allogeneic bone marrow or tissue prior to transplant in order to prevent graft-versus-host disease, can be carried out as follows. Bone marrow is harvested from the patient or other individual and then incubated in medium containing serum to which is added the conjugate of the invention, concentrations range from about 1 pM to 0.1 mM, for about 30 minutes to about 48 hours at about 37 °C. The exact conditions of concentration and time of incubation (=dose) are readily determined by the skilled clinicians. After incubation, the bone marrow cells are washed with medium containing serum and returned to the patient by i.v. infusion according to known methods. In circumstances where the patient receives other treatment such as a course of ablative chemotherapy or total-body irradiation between the time of harvest of the marrow and reinfusion of the treated cells, the treated marrow cells are stored frozen in liquid nitrogen using standard medical equipment.

For clinical *in vivo* use, the conjugate via the linkers of the invention will be supplied as solutions or as a lyophilized solid that can be redissolved in sterile water for injection. Examples of suitable protocols of conjugate administration are as follows. Conjugates are given weekly for 8~20 weeks as an i.v. bolus. Bolus doses are given in 50 to 500 ml of normal saline to which human serum albumin (e.g. 0.5 to 5 mL of a concentrated solution of human serum albumin, 100 mg/mL) can be added. Dosages will be about 50 µg to 100 mg/kg of body weight per week, i.v. (range of 10 µg to 50 mg/kg per injection). 4~20 weeks after treatment, the patient may receive a second course of treatment. Specific clinical protocols with regard to route of administration, excipients, diluents, dosages, times, etc., can be determined by the skilled clinicians.

Examples of medical conditions that can be treated according to the *in vivo* or *ex vivo* methods of killing selected cell populations include malignancy of any types of cancer, autoimmune diseases, graft rejections, and infections (viral, bacterial or parasite).

The amount of a conjugate which is required to achieve the desired biological effect, will vary depending upon a number of factors, including the chemical characteristics, the potency, and the bioavailability of the conjugates, the type of disease, the species to which the patient belongs, the diseased state of the patient, the route of administration, all factors which dictate the required dose amounts, delivery and regimen to be administered.

In general terms, the conjugates via the linkers of this invention may be provided in an aqueous physiological buffer solution containing 0.1 to 10% w/v conjugates for parenteral administration. Typical dose ranges are from 1 µg/kg to 0.1 g/kg of body weight per day; a preferred dose range is from 0.01 mg/kg to 20 mg/kg of body weight per day, or per week, or an equivalent dose in a human child. The preferred dosage of drug to be administered is likely to depend on such variables as the type and extent of progression of the disease or disorder, the overall health status of the particular patient, the relative biological efficacy of the compound selected, the formulation of the compound, the route of administration (intravenous, intramuscular, or other), the pharmacokinetic properties of the conjugates by the chosen delivery route, and the speed (bolus or continuous infusion) and schedule of administrations (number of repetitions in a given period of time).

The conjugates via the linkers of the present invention are also capable of being administered in unit dose forms, wherein the term "unit dose" means a single dose which is capable of being administered to a patient, and which can be readily handled and packaged, remaining as a physically and chemically stable unit dose comprising either the active conjugate itself, or as a pharmaceutically acceptable composition, as described hereinafter. As such, typical total daily/weekly/biweekly/monthly dose ranges are from 0.01 to 100 mg/kg of body weight. By way of general guidance, unit doses for humans range from 1 mg to 3000 mg per day, or per week, per two week or per month. Preferably the unit dose range is from 1 to 500 mg administered one to four times a week, and even more preferably from 1 mg to 100 mg, once a week. Conjugates provided herein can be formulated into pharmaceutical compositions by admixture with one or more pharmaceutically acceptable excipients. Such unit dose compositions may be prepared for use by oral administration, particularly in the form of tablets, simple capsules or soft gel capsules; or intranasal, particularly in the form of powders, nasal drops, or aerosols; or dermally, for example, topically in ointments, creams, lotions, gels or sprays, or via trans-dermal patches.

### DRUGS/CYTOTOXIC AGENTS

Drugs that can be conjugated to a cell-binding molecule in the present invention are small molecule drugs including cytotoxic agents, which can be linked to or after they are modified for linkage to the cell-binding agent. A "small molecule drug" is broadly used herein to refer to an organic, inorganic, or organometallic compound that may have a molecular weight of for example 100 to 1800, more suitably from 120 to 1400. Small molecule drugs are well characterized in the art, such as in WO05058367A2, and in U.S. Patent No. 4,956,303, among others. The drugs include known drugs and those that may become known drugs.

Drugs that are known include, but not limited to,
1). Chemotherapeutic agents: a). Alkylating agents: such as Nitrogen mustards: chlorambucil, chlornaphazine, cyclophosphamide, dacarbazine, estramustine, ifosfamide, mechlorethamine, mechlorethamine oxide hydrochloride, mannomustine, mitobronitol, melphalan, mitolactol, pipobroman, novembichin, phenesterine, prednimustine, thiotepa, trofosfamide, uracil mustard; CC-1065 (including its adozelesin, carzelesin and bizelesin synthetic analogues); Duocarmycin (including the synthetic analogues, KW-2189 and CBI-TMI); Benzodiazepine dimers (e.g., dimmers of pyrrolobenzodiazepine (PBD) or tomaymycin, indolinobenzodiazepines, imidazobenzothiadiazepines, or oxazolidino-benzodiazepines); Nitrosoureas: (carmustine, lomustine, chlorozotocin, fotemustine, nimustine, ranimustine); Alkylsulphonates: (busulfan, treosulfan, improsulfan and piposulfan); Triazenes: (dacarbazine); Platinum containing compounds: (carboplatin, cisplatin, oxaliplatin); aziridines, such as benzodopa, carboquone, meturedopa, and uredopa; ethylenimines and methylamelamines including altretamine, triethylenemel-amine, trietylenephosphoramide, triethylenethiophosphaoramide and trimethylolomel-amine]; b). Plant Alkaloids: such as Vinca alkaloids: (vincristine, vinblastine, vindesine, vinorelbine, navelbin); Taxoids: (paclitaxel, docetaxol) and their analogs, Maytansinoids (DM1, DM2, DM3, DM4, maytansine and ansamitocins) and their analogs, cryptophycins (particularly cryptophycin 1 and cryptophycin 8); epothilones, eleutherobin, discodermo-lide, bryostatins, dolostatins, auristatins, tubulysins, cephalostatins; pancratistatin; a sarcodictyin; spongistatin; c). DNA Topoisomerase Inhibitors: such as [Epipodophyllins: (9-aminocamptothecin, camptothecin, crisnatol, daunomycin, etoposide, etoposide phosphate, irinotecan, mitoxantrone, novantrone, retinoic acids (retinols), teniposide, topotecan, 9-nitrocamptothecin (RFS 2000)); mitomycins: (mitomycin C)]; d). Anti-metabolites: such as {[Anti-folate: DHFR inhibitors: (methotrexate, trimetrexate, denopterin, pteropterin, aminopterin (4-aminopteroic acid) or the other folic acid analogues); IMP dehydrogenase Inhibitors: (mycophenolic acid, tiazofurin, ribavirin, EICAR); Ribonucleotide reductase Inhibitors: (hydroxyurea, deferoxamine)]; [Pyrimidine analogs: Uracil analogs: (ancitabine, azacitidine, 6-azauridine, capecitabine (Xeloda), carmofur, cytarabine, dideoxyuridine, doxifluridine, enocitabine, 5-Fluorouracil, floxuridine, ratitrexed (Tomudex)); Cytosine analogs: (cytarabine, cytosine arabinoside, fludarabine); Purine analogs: (azathioprine, fludarabine, mercaptopurine, thiamiprine, thioguanine)]; folic acid replenisher, such as frolinic acid}; e). Hormonal therapies: such as {Receptor antagonists: [Anti-estrogen: (megestrol, raloxifene, tamoxifen); LHRH agonists: (goscrclin, leuprolide acetate); Antiandrogens: (bicalutamide, flutamide, calusterone, dromostanolone propionate, epitiostanol, goserelin, leuprolide, mepitiostane, nilutamide, testolactone, trilostane and other androgens inhibitors)]; Retinoids/Deltoids: [Vitamin D3 analogs: (CB 1093, EB 1089 KH 1060, cholecalciferol, ergocalciferol); Photodynamic therapies: (verteporfin, phthalocyanine, photosensitizer Pc4, demethoxy-hypocrellin A); Cytokines: (Interferon-alpha, Interferon-gamma, tumor necrosis factor (TNFs), human proteins containing a TNF domain)]}; f). Kinase inhibitors, such as BIBW 2992 (anti-EGFR/Erb2), imatinib, gefitinib, pegaptanib, sorafenib, dasatinib, sunitinib, erlotinib, nilotinib, lapatinib, axitinib, pazopanib. vandetanib, E7080 (anti-VEGFR2), mubritinib, ponatinib (AP24534), bafetinib (INNO-406), bosutinib (SKI-606), cabozantinib, vismodegib, iniparib, ruxolitinib, CYT387, axitinib, tivozanib, sorafenib, bevacizumab, cetuximab, Trastuzumab, Ranibizumab, Panitumumab, ispinesib; g). antibiotics, such as the enediyne antibiotics (e.g. calicheamicins, especially calicheamicin γ1, δ1, α1 and β1, see, e.g., J. Med. Chem., 39 (11), 2103-2117 (1996), Angew Chem Intl. Ed. Engl. 33:183-186 (1994); dynemicin, including dynemicin A and deoxydynemicin; esperamicin, kedarcidin, C-1027, maduropeptin, as well as neocarzinostatin chromophore and related chromoprotein enediyne antiobiotic chromomophores), aclacinomysins, actinomycin, authramycin, azaserine, bleomycins, cactinomycin, carabicin, carminomycin, carzinophilin; chromomycins, dactinomycin, daunorubicin, detorubicin, 6-diazo-5-oxo-L-norleucine, doxorubicin, morpholino-doxorubicin, cyanomorpholino-doxorubicin, 2-pyrrolino-doxorubicin and deoxydoxorubicin, epirubicin, esorubicin, idarubicin, marcellomycin, nitomycins, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; f). Others: such as Polyketides (acetogenins), especially bullatacin and bullatacinone; gemcitabine, epoxomicins (e. g. carfilzomib), bortezomib, thalidomide, lenalidomide, pomalidomide, tosedostat, zybrestat, PLX4032, STA-9090, Stimuvax, allovectin-7, Xegeva, Provenge, Yervoy, Isoprenylation inhibitors (such as Lovastatin), Dopaminergic neurotoxins (such as 1-methyl-4-phenylpyridinium ion), Cell cycle inhibitors (such as staurosporine), Actinomycins (such as Actinomycin D, dactinomycin), Bleomycins (such as bleomycin A2, bleomycin B2, peplomycin), Anthracyclines (such as daunorubicin, doxorubicin (adriamycin), idarubicin, epirubicin, pirarubicin, zorubicin, mtoxantrone, MDR inhibitors (such as verapamil), Ca²⁺ ATPase inhibitors (such as thapsigargin), Histone deacetylase inhibitors (Vorinostat, Romidepsin, Panobinostat, Valproic acid, Mocetinostat (MGCD0103), Belinostat, PCI-24781, Entinostat, SB939, Resminostat, Givinostat, AR-42, CUDC-101, sulforaphane, Trichostatin A) ; Thapsigargin, Celecoxib, glitazones, epigallocatechin gallate, Disulfiram, Salinosporamide A.; Anti-adrenals, such as aminoglutethimide, mitotane, trilostane; aceglatone; aldophosphamide glycoside; aminolevulinic acid; amsacrine; arabinoside, bestrabucil; bisantrene; edatraxate; defofamine; demecolcine; diaziquone; eflornithine (DFMO), elfomithine; elliptinium acetate, etoglucid; gallium nitrate; gacytosine, hydroxyurea; ibandronate, lentinan; lonidamine; mitoguazone; mitoxantrone; mopidamol; nitracrine; pentostatin; phenamet; pirarubicin; podophyllinic acid; 2-ethylhydrazide; procarbazine; PSK^{®}; razoxane; rhizoxin; sizofiran; spirogermanium; tenuazonic acid; triaziquone; 2, 2',2"-trichlorotriethylamine; trichothecenes (especially T-2 toxin, verrucarin A, roridin A and anguidine); urethane, siRNA, antisense drugs, and a nucleolytic enzyme.
2). An anti-autoimmune disease agent includes, but is not limited to, cyclosporine, cyclosporine A, aminocaproic acid, azathioprine, bromocriptine, chlorambucil, chloroquine, cyclophosphamide, corticosteroids (e.g. amcinonide, betamethasone, budesonide, hydrocortisone, flunisolide, fluticasone propionate, fluocortolone danazol, dexamethasone, Triamcinolone acetonide, beclometasone dipropionate), DHEA, enanercept, hydroxychloroquine, infliximab, meloxicam, methotrexate, mofetil, mycophenylate, prednisone, sirolimus, tacrolimus.
3). An anti-infectious disease agent includes, but is not limited to, a). Aminoglycosides: amikacin, astromicin, gentamicin (netilmicin, sisomicin, isepamicin), hygromycin B, kanamycin (amikacin, arbekacin, bekanamycin, dibekacin, tobramycin), neomycin (framycetin, paromomycin, ribostamycin), netilmicin, spectinomycin, streptomycin, tobramycin, verdamicin; b). Amphenicols: azidamfenicol, chloramphenicol, florfenicol, thiamphenicol; c). Ansamycins: geldanamycin, herbimycin; d). Carbapenems: biapenem, doripenem, ertapenem, imipenem/cilastatin, meropenem, panipenem; e). Cephems: carbacephem (loracarbef), cefacetrile, cefaclor, cefradine, cefadroxil, cefalonium, cefaloridine, cefalotin or cefalothin, cefalexin, cefaloglycin, cefamandole, cefapirin, cefatrizine, cefazaflur, cefazedone, cefazolin, cefbuperazone, cefcapene, cefdaloxime, cefepime, cefminox, cefoxitin, cefprozil, cefroxadine, ceftezole, cefuroxime, cefixime, cefdinir, cefditoren, cefepime, cefetamet, cefmenoxime, cefodizime, cefonicid, cefoperazone, ceforanide, cefotaxime, cefotiam, cefozopran, cephalexin, cefpimizole, cefpiramide, cefpirome, cefpodoxime, cefprozil, cefquinome, cefsulodin, ceftazidime, cefteram, ceftibuten, ceftiolene, ceftizoxime, ceftobiprole, ceftriaxone, cefuroxime, cefuzonam, *cephamycin* (cefoxitin, cefotetan, cefmetazole), oxacephem (flomoxef, latamoxef); f). Glycopeptides: bleomycin, vancomycin (oritavancin, telavancin), teicoplanin (dalbavancin), ramoplanin; g). Glycylcyclines: e. g. tigecycline; g). β-Lactamase inhibitors: penam (sulbactam, tazobactam), clavam (clavulanic acid); i). Lincosamides: clindamycin, lincomycin; j). Lipopeptides: daptomycin, A54145, calcium-dependent antibiotics (CDA); k). Macrolides: azithromycin, cethromycin, clarithromycin, dirithromycin, erythromycin, flurithromycin, josamycin, ketolide (telithromycin, cethromycin), midecamycin, miocamycin, oleandomycin, rifamycins (rifampicin, rifampin, rifabutin, rifapentine), rokitamycin, roxithromycin, spectinomycin, spiramycin, tacrolimus (FK506), troleandomycin, telithromycin; 1). Monobactams: aztreonam, tigemonam; m). Oxazolidinones: linezolid; n). Penicillins: amoxicillin, ampicillin (pivampicillin, hetacillin, bacampicillin, metampicillin, talampicillin), azidocillin, azlocillin, benzylpenicillin, benzathine benzylpenicillin, benzathine phenoxymethyl-penicillin, clometocillin, procaine benzylpenicillin, carbenicillin (carindacillin), cloxacillin, dicloxacillin, epicillin, flucloxacillin, mecillinam (pivmecillinam), mezlocillin, meticillin, nafcillin, oxacillin, penamecillin, penicillin, pheneticillin, phenoxymethyl-penicillin, piperacillin, propicillin, sulbenicillin, temocillin, ticarcillin; o). Polypeptides: bacitracin, colistin, polymyxin B; p). Quinolones: alatrofloxacin, balofloxacin, ciprofloxacin, clinafloxacin, danofloxacin, difloxacin, enoxacin, enrofloxacin, floxin, garenoxacin, gatifloxacin, gemifloxacin, grepafloxacin, kano trovafloxacin, levofloxacin, lomefloxacin, marbofloxacin, moxifloxacin, nadifloxacin, norfloxacin, orbifloxacin, ofloxacin, pefloxacin, trovafloxacin, grepafloxacin, sitafloxacin, sparfloxacin, temafloxacin, tosufloxacin, trovafloxacin; q). Streptogramins: pristinamycin, quinupristin/dalfopristin); r). Sulfonamides: mafenide, prontosil, sulfacetamide, sulfamethizole, sulfanilimide, sulfasalazine, sulfisoxazole, trimethoprim, trimethoprim-sulfamethoxazole (co-trimoxazole); s). Steroid antibacterials: e.g. fusidic acid; t). Tetracyclines: doxycycline, chlortetracycline, clomocycline, demeclo-cycline, lymecycline, meclocycline, metacycline, minocycline, oxytetracycline, penimepicycline, rolitetracycline, tetracycline, glycylcyclines (e.g. tigecycline); u). Other types of antibiotics: annonacin, arsphenamine, bactoprenol inhibitors (Bacitracin), DADAL/AR inhibitors (cycloserine), dictyostatin, discodermolide, eleutherobin, epothilone, ethambutol, etoposide, faropenem, fusidic acid, furazolidone, isoniazid, laulimalide, metronidazole, mupirocin, mycolactone, NAM synthesis inhibitors (e. g. fosfomycin), nitrofurantoin, paclitaxel, platensimycin, pyrazinamide, quinupristin/dalfopristin, rifampicin (rifampin), tazobactam tinidazole, uvaricin;
4). Anti-viral drugs: a). Entry/fusion inhibitors: aplaviroc, maraviroc, vicriviroc, gp41 (enfuvirtide), PRO 140, CD4 (ibalizumab); b). Integrase inhibitors: raltegravir, elvitegravir, globoidnan A; c). Maturation inhibitors: bevirimat, vivecon; d). Neuraminidase inhibitors: oseltamivir, zanamivir, peramivir; e). Nucleosides & nucleotides: abacavir, aciclovir, adefovir, amdoxovir, apricitabine, brivudine, cidofovir, clevudine, dexelvucitabine, didanosine (ddI), elvucitabine, emtricitabine (FTC), entecavir, famciclovir, fluorouracil (5-FU), 3'-fluoro-substituted 2', 3'-dideoxynucleoside analogues (e.g. 3'-fluoro-2',3'-dideoxythymidine (FLT) and 3'-fluoro-2',3'-dideoxyguanosine (FLG), fomivirsen, ganciclovir, idoxuridine, lamivudine (3TC), l-nucleosides (e.g. *β*-l-thymidine and *β*-l-2'-deoxycytidine), penciclovir, racivir, ribavirin, stampidine, stavudine (d4T), taribavirin (viramidine), telbivudine, tenofovir, trifluridine valaciclovir, valganciclovir, zalcitabine (ddC), zidovudine (AZT); f). Non-nucleosides: amantadine, ateviridine, capravirine, diarylpyrimidines (etravirine, rilpivirine), delavirdine, docosanol, emivirine, efavirenz, foscarnet (phosphonoformic acid), imiquimod, interferon alfa, loviride, lodenosine, methisazone, nevirapine, NOV-205, peginterferon alfa, podophyllotoxin, rifampicin, rimantadine, resiquimod (R-848), tromantadine; g). Protease inhibitors: amprenavir, atazanavir, boceprevir, darunavir, fosamprenavir, indinavir, lopinavir, nelfinavir, pleconaril, ritonavir, saquinavir, telaprevir (VX-950), tipranavir; h). Other types of anti-virus drugs: abzyme, arbidol, calanolide a, ceragenin, cyanovirin-n, diarylpyrimidines, epigallocatechin gallate (EGCG), foscarnet, griffithsin, taribavirin (viramidine), hydroxyurea, KP-1461, miltefosine, pleconaril, portmanteau inhibitors, ribavirin, seliciclib.
5). The drugs used for conjugates via a bridge linker of the present invention also include radioisotopes. Examples of radioisotopes (radionuclides) are ³H, ¹¹C, ¹⁴C, ¹⁸F, ³²P, ³⁵S, ⁶⁴Cu, ⁶⁸Ga, ⁸⁶Y, ⁹⁹Tc, ¹¹¹In, ¹²³I, ¹²⁴I, ¹²⁵I, ¹³¹I, ¹³³Xe, ¹⁷⁷Lu, ²¹¹At, or ²¹³Bi. Radioisotope labeled antibodies are useful in receptor targeted imaging experiments or can be for targeted treatment such as with the antibody-drug conjugates of the invention (Wu et al (2005) Nature Biotechnology 23(9): 1137-1146). The cell binding molecules, e.g. an antibody can be labeled with ligand reagents through the bridge linkers of the present patent that bind, chelate or otherwise complex a radioisotope metal, using the techniques described in Current Protocols in Immunology, Volumes 1 and 2, Coligen et al, Ed. Wiley-Interscience, New York, N.Y., Pubs. (1991). Chelating ligands which may complex a metal ion include DOTA, DOTP, DOTMA, DTPA and TETA (Macrocyclics, Dallas, Tex.).
6). The pharmaceutically acceptable salts, acids or derivatives of any of the above drugs.

In another embodiment, the drug in the Formula (II) and (IV) can a chromophore molecule, for which the conjugate can be used for detection, monitoring, or study the interaction of the cell binding molecule with a target cell. Chromophore molecules are a compound that have the ability to absorb a kind of light, such as UV light, florescent light, IR light, near IR light, visual light; A chromatophore molecule includes a class or subclass of xanthophores, erythrophores, iridophores, leucophores, melanophores, and cyanophores; a class or subclass of fluorophore molecules which are fluorescent chemical compounds re-emitting light upon light; a class or subclass of visual phototransduction molecules; a class or subclass of photophore molecules; a class or subclass of luminescence molecules; and a class or subclass of luciferin compounds.

The chromophore molecule can be selected from, but not limited, Non-protein organic fluorophores, such as: Xanthene derivatives (fluorescein, rhodamine, Oregon green, eosin, and Texas red); Cyanine derivatives: (cyanine, indocarbocyanine, oxacarbocyanine, thiacarbocyanine, and merocyanine); Squaraine derivatives and ring-substituted squaraines, including Seta, SeTau, and Square dyes; Naphthalene derivatives (dansyl and prodan derivatives); Coumarin derivatives; Oxadiazole derivatives (pyridyloxazole, nitro-benzoxadiazole and benzoxadiazole); Anthracene derivatives (anthraquinones, including DRAQ5, DRAQ7 and CyTRAK Orange); Pyrene derivatives (cascade blue, etc); Oxazine derivatives (Nile red, Nile blue, cresyl violet, oxazine 170 etc). Acridine derivatives (proflavin, acridine orange, acridine yellow etc). Arylmethine derivatives (auramine, crystal violet, malachite green). Tetrapyrrole derivatives (porphin, phthalocyanine, bilirubin).

Or a chromophore molecule can be selected from any analogs and derivatives of the following fluorophore compounds: CF dye (Biotium), DRAQ and CyTRAK probes (Bio-Status), BODIPY (Invitrogen), Alexa Fluor (Invitrogen), DyLight Fluor (Thermo Scientific, Pierce), Atto and Tracy (Sigma Aldrich), FluoProbes (Interchim), Abberior Dyes (Abberior), DY and MegaStokes Dyes (Dyomics), Sulfo Cy dyes (Cyandye), HiLyte Fluor (AnaSpec), Seta, SeTau and Square Dyes (SETA BioMedicals), Quasar and Cal Fluor dyes (Biosearch Technologies), SureLight Dyes (APC, RPEPerCP, Phycobili-somes)(Columbia Biosciences), APC, APCXL, RPE, BPE (Phyco-Biotech);

Examples of the widely used fluorophore compounds which are reactive or conjugatable with the linkers of the invention are: Allophycocyanin (APC), Aminocou-marin, APC-Cy7 conjugates, BODIPY-FL, Cascade Blue, Cy2, Cy3, Cy3.5, Cy3B, Cy5, Cy5.5, Cy7, Fluorescein, FluorX, Hydroxycoumarin, Lissamine Rhodamine B, Lucifer yellow, Methoxycoumarin, NBD, Pacific Blue, Pacific Orange, PE-Cy5 conjugates, PE-Cy7 conjugates, PerCP, R-Phycoerythrin(PE), Red 613, Seta-555-Azide, Seta-555-DBCO, Seta-555-NHS, Seta-580-NHS, Seta-680-NHS, Seta-780-NHS, Seta-APC-780, Seta-PerCP-680, Seta-R-PE-670, SeTau-380-NHS, SeTau-405-Maleimide, SeTau-405-NHS, SeTau-425-NHS, SeTau-647-NHS, Texas Red, TRITC, TruRed, X-Rhodamine.

The fluorophore compounds that can be linked to the linkers of the invention for study of nucleic acids or proteins are selected from the following compounds or their derivatives: 7-AAD (7-aminoactinomycin D, CG-selective), Acridine Orange, Chromomycin A3, CyTRAK Orange (Biostatus, red excitation dark), DAPI, DRAQ5, DRAQ7, Ethidium Bromide, Hoechst33258, Hoechst33342, LDS 751, Mithramycin, PropidiumIodide (PI), SYTOX Blue, SYTOX Green, SYTOX Orange, Thiazole Orange, TO-PRO: Cyanine Monomer, TOTO-1, TO-PRO-1, TOTO-3, TO-PRO-3, YOSeta-1, YOYO-1. The fluorophore compounds that can be linked to the linkers of the invention for study cells are selected from the following compounds or their derivatives: DCFH (2'7'Dichorodihydro-fluorescein, oxidized form), DHR (Dihydrorhodamine 123, oxidized form, light catalyzes oxidation), Fluo-3 (AM ester. pH > 6), Fluo-4 (AM ester. pH 7.2), Indo-1 (AM ester, low/high calcium (Ca2+)), SNARF(pH 6/9). The preferred fluorophore compounds that can be linked to the linkers of the invention for study proteins/antibodies are selected from the following compounds or their derivatives: Allophycocyanin(APC), AmCyan1 (tetramer, Clontech), AsRed2 (tetramer, Clontech), Azami Green (monomer, MBL), Azurite, B-phycoerythrin(BPE), Cerulean, CyPet, DsRed monomer (Clontech), DsRed2 ("RFP", Clontech), EBFP, EBFP2, ECFP, EGFP (weak dimer, Clontech), Emerald (weak dimer, Invitrogen), EYFP (weak dimer, Clontech), GFP (S65A mutation), GFP (S65C mutation), GFP (S65L mutation), GFP (S65T mutation), GFP (Y66F mutation), GFP (Y66H mutation), GFP (Y66W mutation), GFPuv, HcRed1, J-Red, Katusha, Kusabira Orange (monomer, MBL), mCFP, mCherry, mCitrine, Midoriishi Cyan (dimer, MBL), mKate (TagFP635, monomer, Evrogen), mKeima-Red (monomer, MBL), mKO, mOrange, mPlum, mRaspberry, mRFP1 (monomer, Tsien lab), mStrawberry, mTFP1, mTurquoise2, P3 (phycobilisome complex), Peridinin Chlorophyll (PerCP), R-phycoerythrin(RPE), T-Sapphire, TagCFP (dimer, Evrogen), TagGFP (dimer, Evrogen), TagRFP (dimer, Evrogen), TagYFP (dimer, Evrogen), tdTomato (tandem dimer), Topaz, TurboFP602 (dimer, Evrogen), TurboFP635 (dimer, Evrogen), TurboGFP (dimer, Evrogen), TurboRFP (dimer, Evrogen), TurboYFP (dimer, Evrogen), Venus, Wild Type GFP, YPet, ZsGreen1 (tetramer, Clontech), ZsYellow1 (tetramer, Clontech).

In yet another embodiment, the preferred cytotoxic agents that conjugated to a cell-binding molecule via a bridge linker of this patent are tubulysins, maytansinoids, taxanoids (taxanes), CC-1065 analogs, daunorubicin and doxorubicin compounds, benzodiazepine dimers (e.g., dimers of pyrrolobenzodiazepine (PBD), tomaymycin, anthramycin, indolinobenzodiazepines, imidazobenzothiadiazepines, or oxazolidino-benzodiazepines), calicheamicins and the enediyne antibiotics, actinomycin, azaserines, bleomycins, epirubicin, tamoxifen, idarubicin, dolastatins, auristatins (e.g. monomethyl auristatin E, MMAE , MMAF, auristatin PYE, auristatin TP, Auristatins 2-AQ, 6-AQ, EB (AEB), and EFP (AEFP)), duocarmycins, thiotepa, vincristines, hemiasterlins, nazumamides, microginins, radiosumins, alterobactins, microsclerodermins, theonellamides, esperamicins, PNU-159682, and their analogues and derivatives above thereof.

Tubulysins that are preferred for conjugation in the present invention are well known in the art and can be isolated from natural sources according to known methods or prepared synthetically according to known methods (e. g. Balasubramanian, R.; et al. J. Med. Chem., 2009, 52, 238-240. Wipf, P.; et al. Org. Lett., 2004, 6, 4057-4060. Pando, O.; et al. J. Am. Chem. Soc., 2011, 133, 7692-7695. Reddy, J. A.; et al. Mol. Pharmaceutics, 2009, 6, 1518-1525. Raghavan, B.; et al. J. Med. Chem., 2008, 51, 1530-1533. Patterson, A. W.; et al. J. Org. Chem., 2008, 73, 4362-4369. Pando, O.; et al. Org. Lett., 2009, 11 (24), pp 5567-5569. Wipf, P.; et al. Org. Lett., 2007, 9 (8), 1605-1607. Friestad, G. K.; Org. Lett., 2004, 6, pp 3249-3252. Hillary M. Peltier, H. M.; et al. J. Am. Chem. Soc., 2006, 128, 16018-16019. Chandrasekhar, S.; et al. J. Org. Chem., 2009, 74, 9531-9534. Liu, Y.; et al. Mol. Pharmaceutics, 2012, 9, 168-175. Friestad, G. K.; et al. Org. Lett., 2009, 11, 1095-1098. Kubicek, K.; et al., Angew Chem Int Ed Engl, 2010. 49: p. 4809-12. Chai, Y.; et al., Chem Biol, 2010, 17: 296-309. Ullrich, A.; et al., Angew Chem Int Ed Engl, 2009, 48, 4422-5. Sani, M.; et al. Angew Chem Int Ed Engl, 2007, 46, 3526-9. Domling, A.; et al., Angew Chem Int Ed Engl, 2006. 45, 7235-9. Patent applications: Zanda, M. ; et al, Can. Pat. Appl. CA 2710693 (2011). Chai, Y.; et al. Eur. Pat. Appl. 2174947 (2010), PCT WO 2010034724. Leamon, C.; et al, PCT WO 2010033733, WO 2009002993. Ellman, J.; et al, PCT WO 2009134279; PCT WO 2009012958, US appl. 20110263650, 20110021568, Matschiner, G.; et al, PCT WO 2009095447.Vlahov, I.; et al, PCT WO 2009055562, WO 2008112873. Low, P.; et al, PCT WO 2009026177. Richter, W., PCT WO 2008138561. Kjems, J.; et al, PCT WO 2008125116. Davis, M.; et al, PCT WO 2008076333. Diener, J.; et al, U.S. Pat. Appl. 20070041901, WO 2006096754. Matschiner, G.; et al, PCT WO 2006056464. Vaghefi, F.; et al, 5 PCT WO 2006033913. Doemling, A., Ger. Offen. DE 102004030227; PCT WO 2004005327; WO 2004005326; WO2004005269. Stanton, M.; et al, U.S. Pat. Appl. Publ. 20040249130. Hoefle, G.; et al, Ger. Offen. DE 10254439 ; DE 10241152; DE 10008089. Leung, D.; et al, WO 2002077036. Reichenbach, H.; et al, Ger. Offen. DE 19638870; Wolfgang, R.; US 20120129779, Chen, H.,US appl. 20110027274. The preferred structure of tubulysins for conjugation of cell binding molecules are described in the patent application of PCT/IB2012/053554.

Examples of the structures of the conjugates of the antibody-tubulysin analogs via the bridge linker are T01, T02, T03, T04, T05, T06 and T07 as following:

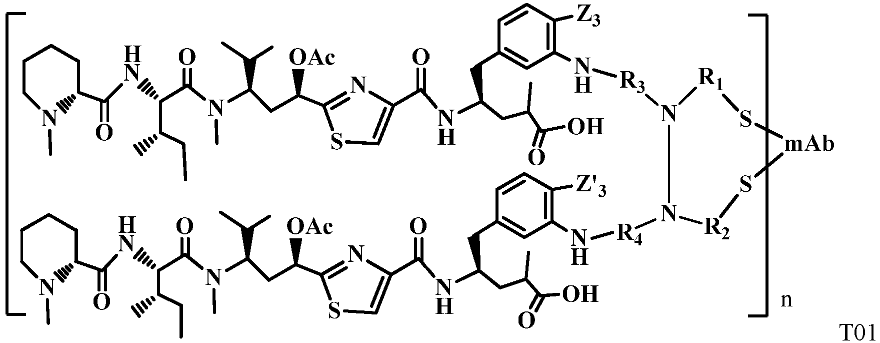

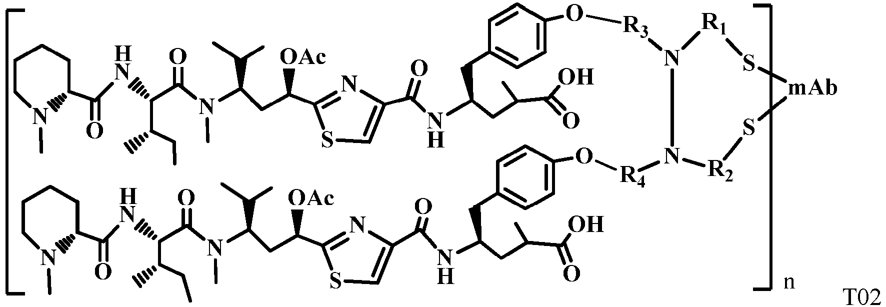

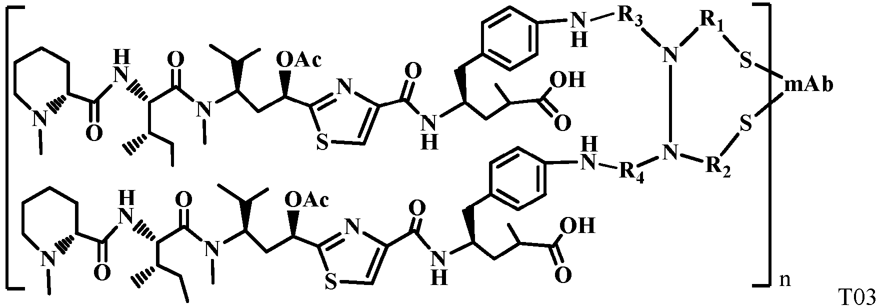

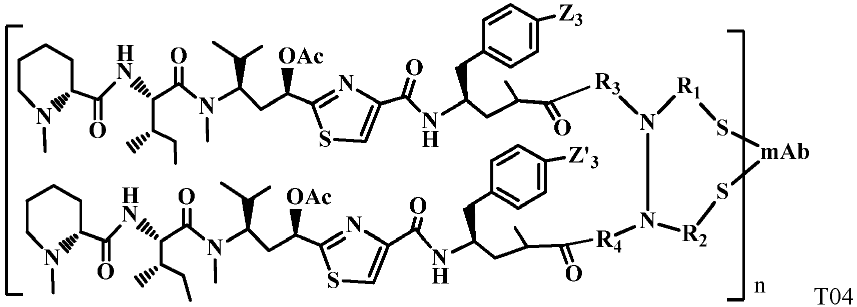

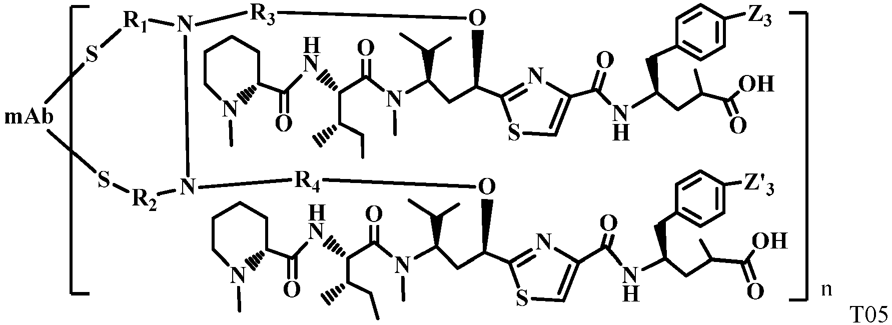

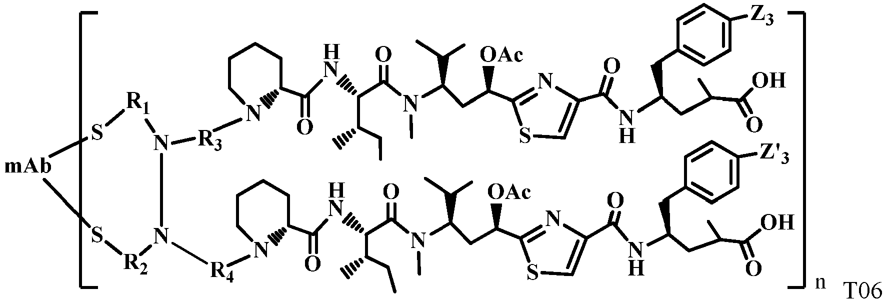

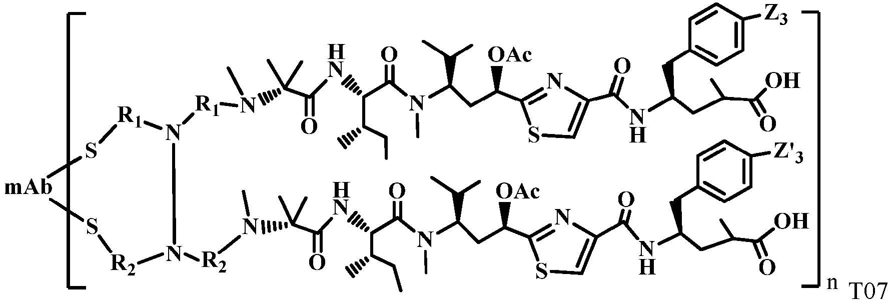

Wherein mAb is an antibody; Z₃ and Z'₃ are independently H, R₁, OP(O)(OM₁)(OM₂), OCH₂OP(O)(OM₁)(OM₂), OSO₃M₁, or O-glycoside (glucoside, galactoside, mannoside, glucuronoside, alloside, fructoside, etc), NH-glycoside, S-glycoside, or CH₂-glycoside; M₁ and M₂ are independently H, Na, K, Ca, Mg, NH₄, or NR₁R₂R₃R₄; n is 1~20; R₁, R₂, R₃ and R₄ are the same defined in Formula (I).

Calicheamicins and their related enediyne antibiotics that are preferred for cell-binding molecule-drug conjugates of this patent are described in: Nicolaou, K. C. et al, Science 1992, 256, 1172-1178; Proc. Natl. Acad. Sci USA. 1993, 90, 5881-5888), U.S. Patent Nos. 4,970,198; 5,053,394; 5,108,912; 5,264,586; 5,384,412; 5,606,040; 5,712,374; 5,714,586; 5,739,116; 5,770,701; 5,770,710; 5,773,001; 5,877,296; 6,015,562; 6,124,310; 8,153,768. An Example of the structure of the conjugate of the antibody-Calicheamicin analog via the bridge linker is C01 as the following: 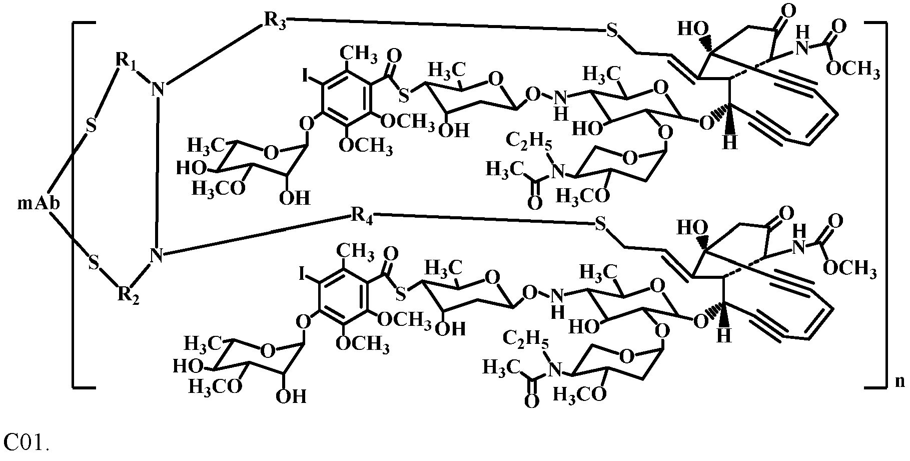 Wherein mAb is an antibody; n is 1~20; R₁, R₂, R₃ and R₄ are the same defined in Formula (I).

Maytansinoids that are preferred to be used in the present invention including maytansinol and its analogues are described in U.S. Patent Nos. 4,256,746, 4,361,650, 4,307,016, 4,294,757, 4,294,757, 4,371,533, 4,424,219, 4,331,598, 4,450,254, 4,364,866, 4,313,946, 4,315,929 4,362,663, 4,322,348, 4,371,533, 4,424,219, 5,208,020, 5,416,064, 5,208,020; 5,416,064; 6,333.410; 6,441,163; 6,716,821, 7,276,497, 7,301,019, 7,303,749, 7,368,565, 7,411,063, 7,851,432, and 8,163,888 . An example of the structure of the conjugate of the antibody- Maytansinoids via the bridge linker is as the following M01: 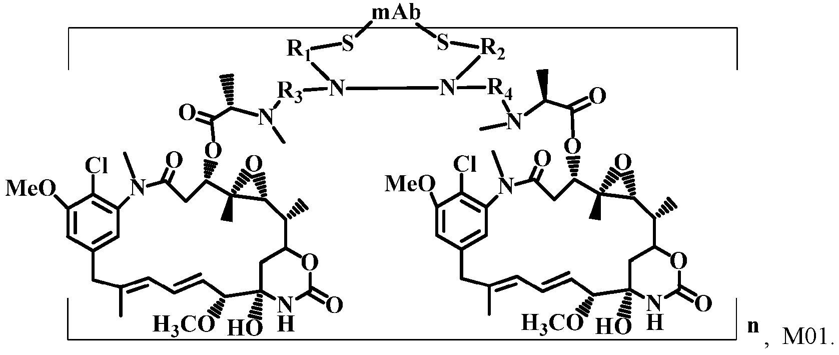 Wherein mAb is an antibody; n is 1~20; R₁, R₂, R₃ and R₄ are the same defined in Formula (I).

Taxanes, which includes Paclitaxel (Taxol), a cytotoxic natural product, and docetaxel (Taxotere), a semi-synthetic derivative, and their analogs which are preferred for conjugation via the bridge linkers of the present patent are exampled in:. K C. Nicolaou et al., J. Am. Chem. Soc. 117, 2409-2420, (1995); Ojima et al, J. Med. Chem. 39:3889-3896 (1996); 40:267-278 (1997); 45, 5620-5623 (2002); Ojima et al., Proc. Natl. Acad. Sci., 96:4256-4261 (1999; Kim et al., Bull. Korean Chem. Soc., 20, 1389-1390 (1999); Miller, et al. J. Med. Chem., 47, 4802-4805(2004); U.S. Patent No. 5,475,011 5,728,849, 5,811,452; 6,340,701; 6,372,738; 6,391,913, 6.436,931; 6,589,979; 6,596,757; 6,706,708; 7,008,942; 7,186,851; 7,217,819; 7,276,499; 7,598,290; and 7,667,054.

Examples of the structures of the conjugate of the antibody- taxanes via the bridge linker are as the following Tx01, Tx02 and Tx03. 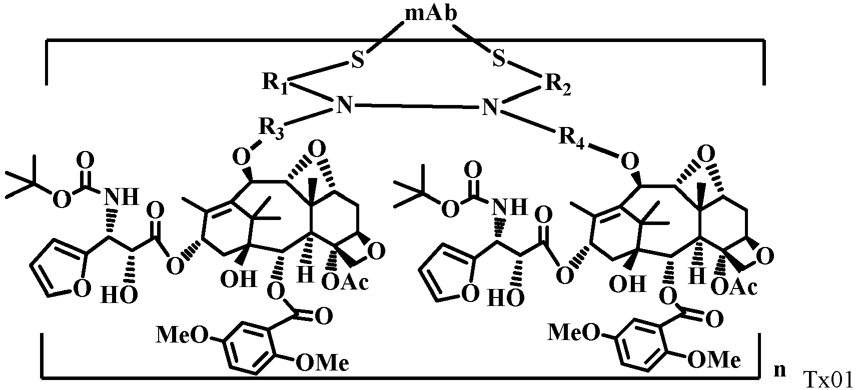 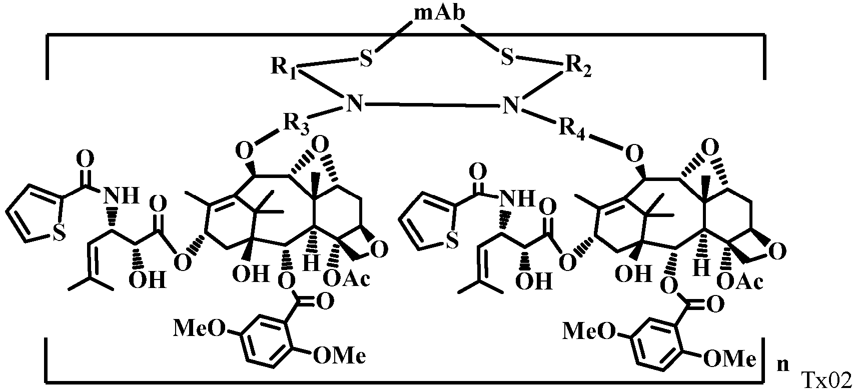 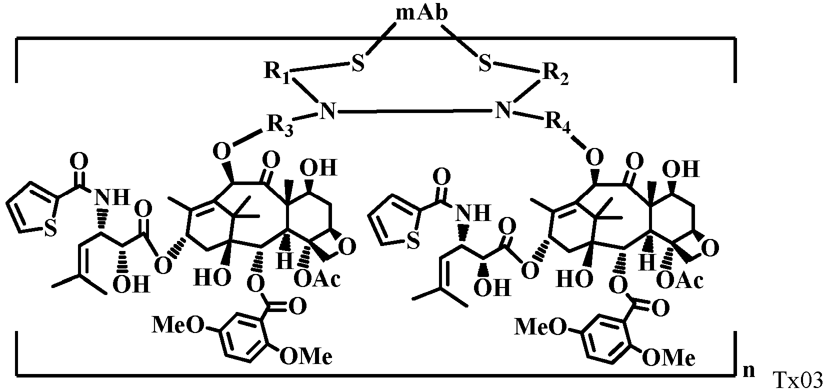 Wherein mAb is an antibody; n is 1~20; R₁, R₂, R₃ and R₄ are the same defined in Formula (I).

CC-1065 analogues and doucarmycin analogs are also preferred to be used for a conjugate with the bridge linkers of the present patent. The examples of the CC-1065 analogues and doucarmycin analogs as well as their synthesis are described in: e.g.Warpehoski, et al, J. Med. Chem. 31:590-603 (1988), D. Boger et al., J. Org. Chem; 66; 6654-6661, 2001; U. S. Patent Nos: 4169888, 4391904, 4671958, 4816567, 4912227, 4923990, 4952394, 4975278, 4978757, 4994578, 5037993, 5070092, 5084468, 5101038, 5117006, 5137877, 5138059, 5147786, 5187186, 5223409, 5225539, 5288514, 5324483, 5332740, 5332837, 5334528, 5403484, 5427908, 5475092, 5495009, 5530101, 5545806, 5547667, 5569825, 5571698, 5573922, 5580717, 5585089, 5585499, 5587161, 5595499, 5606017, 5622929, 5625126, 5629430, 5633425, 5641780, 5660829, 5661016, 5686237, 5693762, 5703080, 5712374, 5714586, 5739116, 5739350, 5770429, 5773001, 5773435, 5786377 5786486, 5789650, 5814318, 5846545, 5874299, 5877296, 5877397, 5885793, 5939598, 5962216, 5969108, 5985908, 6060608, 6066742, 6075181, 6103236, 6114598, 6130237, 6132722, 6143901, 6150584, 6162963, 6172197, 6180370, 6194612, 6214345, 6262271, 6281354, 6310209, 6329497, 6342480, 6486326, 6512101, 6521404, 6534660, 6544731, 6548530, 6555313, 6555693, 6566336, 6,586,618, 6593081, 6630579, 6756397, 6759509, 6762179, 6884869, 6897034, 6946455, 7,049,316, 7087600, 7091186, 7115573, 7129261, 7214663, 7223837, 7304032, 7329507, 7,329,760, 7,388,026, 7,655,660, 7,655,661, 7,906,545, and 8,012,978. Examples of the structures of the conjugate of the antibody- CC-1065 analogs via the bridge linker are as the following CC01, CC02, and CC03. 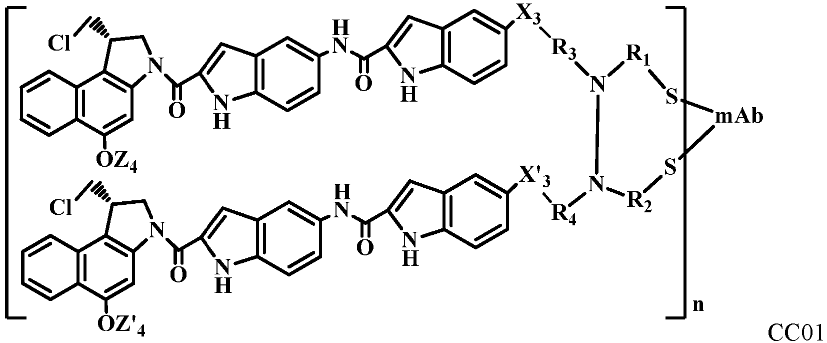 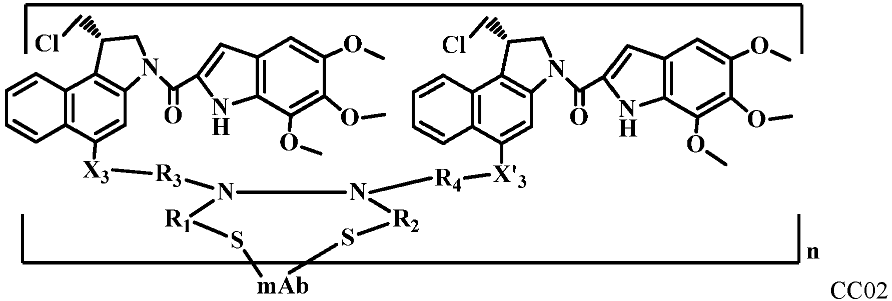 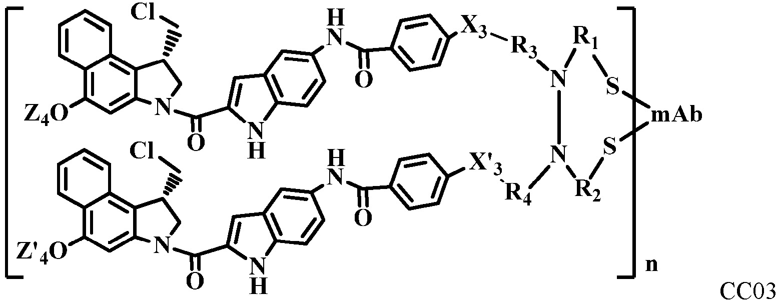 Wherein mAb is an antibody; n is 1~20; Z₄ and Z'₄ are independently H, PO(OM₁)(OM₂), SO₃M₁, CH₂PO(OM₁)(OM₂), CH₃N(CH₂CH₂)₂NC(O)-, O(CH₂CH₂)₂NC(O)-, or glycoside; X₃ and X'₃ are independently O, NH, NR₁, NHC(O), OC(O), CO, R₁, or absent; M₁ and M₂ is independently Na, K, H, NH₄, NR₁R₂R₃R₄; R₁, R₂, R₃, and R₄ are the same defined in Formula (I).

Daunorubicin/Doxorubicin Analogues are also preferred for conjugation via the bridge linkers of the present patent. The preferred structures and their synthesis are exampled in: Hurwitz, E., et al., Cancer Res. 35, 1175-1181 (1975). Yang, H. M., and Reisfeld, R. A., Proc. Natl. Acad. Sci. 85, 1189-1193 (1988); Pietersz, C. A., E., et al., E., et al.," Cancer Res. 48, 926-9311 (1988); Trouet, et al., 79, 626-629 (1982); Z. Brich et al., J. Controlled Release, 19, 245-258 (1992); Chen et al., Syn. Comm., 33, 2377-2390, 2003; King et al., Bioconj. Chem., 10, 279-288, 1999; King et al., J. Med. Chem., 45, 4336-4343, 2002; Kratz et al., J Med Chem. 45, 5523-33. 2002; Kratz et al., Biol Pharm Bull. Jan. 21, 56-61 , 1998; Lau et al., Bioorg. Med. Chem. 3, 1305-1312, 1995; Scott et al., Bioorg. Med.1 Chem. Lett. 6, 1491-1496; 1996; Watanabe et al., Tokai J. Experimental Clin. Med. 15, 327-334, 1990; Zhou et al., J. Am. Chem. Soc. 126, 15656-7, 2004; WO 01/38318; U.S. Patent No.). 5,106,951; 5,122,368; 5,146,064; 5,177,016; 5,208,323; 5,824,805; 6,146,658; 6,214,345; 7569358; 7,803,903; 8,084,586; 8,053,205. Examples of the structures of the conjugate of the antibody- CC-1065 analogs via the bridge linker are as the following Da01, Da02, Da03 and Da04.

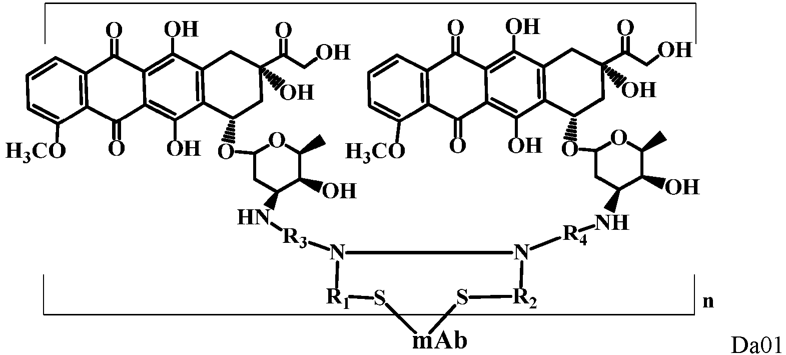

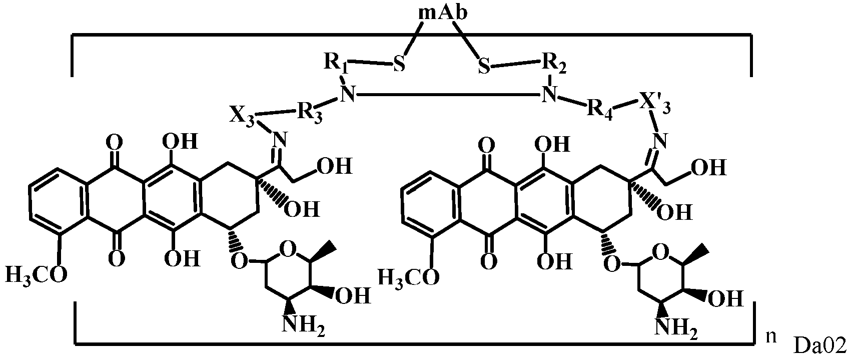

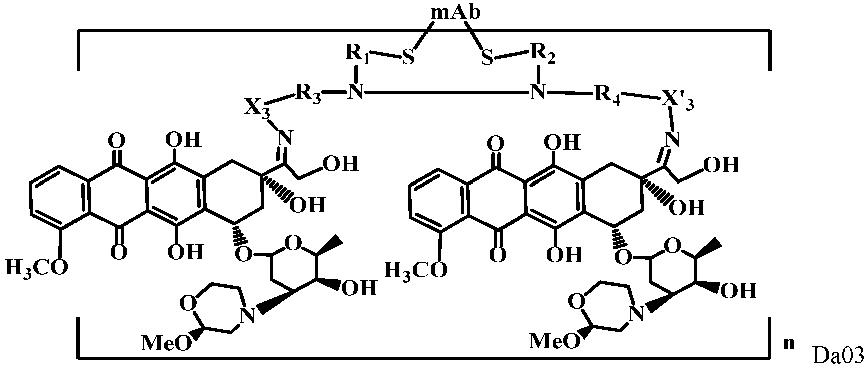

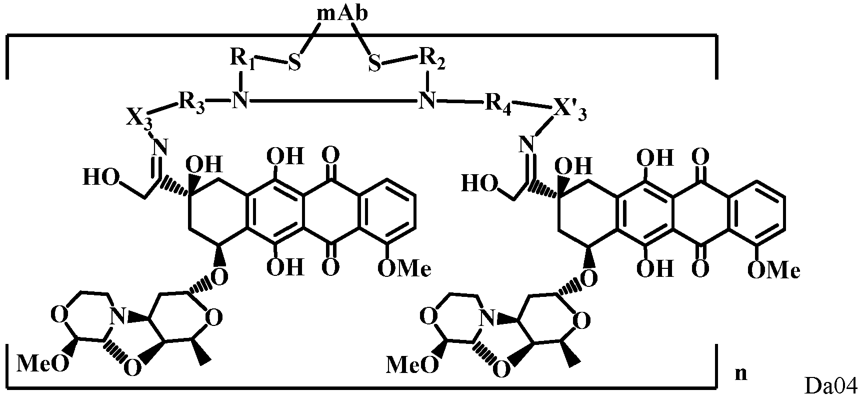

Wherein mAb is an antibody; n is 1~20; X₃ and X₃' are independently O, NH, NR₁, NHC(O), OC(O), CO, C(O)NR₁, C(O)NH, R₁, or absent; R₁, R₂, R₃, and R₄ are the same defined in Formula (I).

Auristatins and dolastatins are preferred in conjugation via the bridge linkers of this patent. The auristatins (e. g. auristain E (AE) auristatin EB (AEB), auristatin EFP (AEFP), monomethyl auristatin E (MMAE), Monomethylauristatin (MMAF), Auristatin F phenylene diamine (AFP) and a phenylalanine variant of MMAE) which are synthetic analogs of dolastatins, are described in Int. J. Oncol. 15:367-72 (1999); Molecular Cancer Therapeutics, vol. 3, No. 8, pp. 921-932 (2004); U.S. Application Nos. 11134826, 20060074008, 2006022925. U.S. Patent Nos. 4414205, 4753894, 4764368, 4816444, 4879278, 4943628, 4978744, 5122368, 5165923, 5169774,5286637, 5410024, 5521284, 5530097, 5554725, 5585089, 5599902, 5629197, 5635483, 5654399, 5663149, 5665860, 5708146, 5714586, 5741892, 5767236, 5767237, 5780588, 5821337, 5840699, 5965537, 6004934, 6033876, 6034065, 6048720, 6054297, 6054561, 6124431, 6143721, 6162930, 6214345, 6239104, 6323315, 6342219, 6342221, 6407213, 6569834, 6620911, 6639055, 6884869, 6913748, 7090843, 7091186, 7097840, 7098305, 7098308, 7498298, 7375078, 7462352, 7553816, 7659241, 7662387, 7745394, 7754681, 7829531, 7837980, 7837995, 7902338, 7964566, 7964567, 7851437, 7994135. Examples of the structures of the conjugate of the antibody-auristatins via the bridge linker are as the following Au01, Au02, Au03, Au04, and Au05.

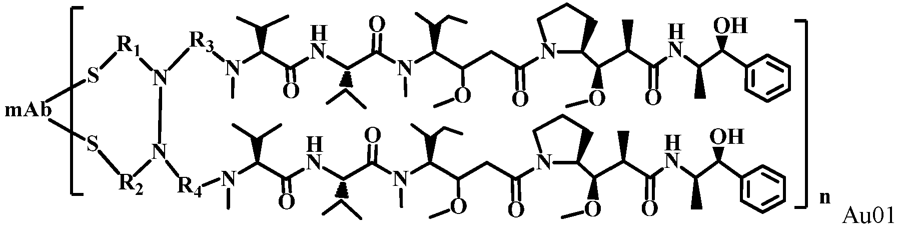

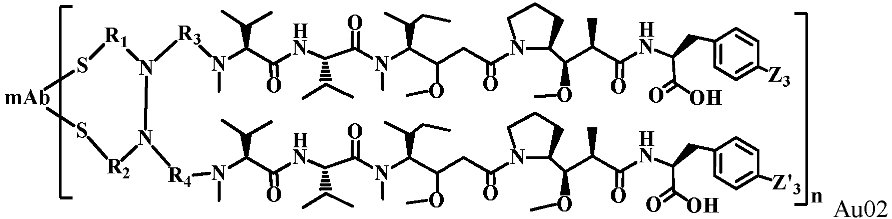

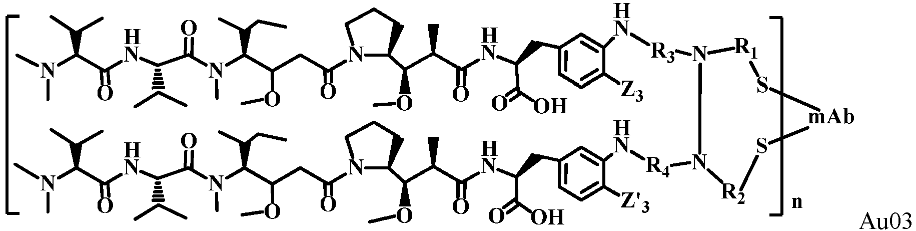

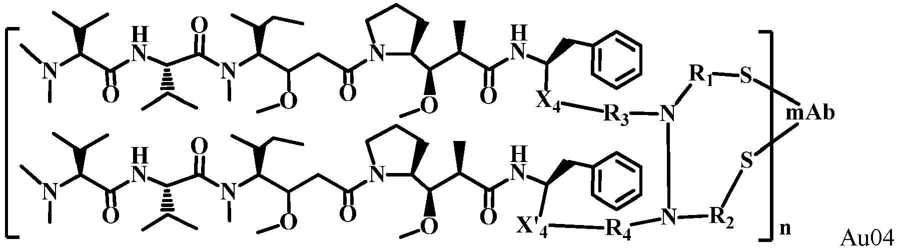

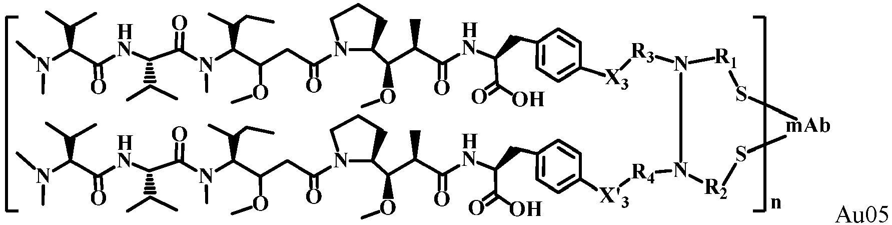

Wherein mAb is an antibody; n is 1~20; X₃ and X'₃ are independently CH₂, O, NH, NR₁, NHC(O), NHC(O)NH, C(O), OC(O), R₁, or absent; X₄ and X'₄ are independently CH₂, C(O), C(O)NH, C(O)N(R₁), R₁, or C(O)O; Z₃ and Z'₃ are independently H, R₁, OP(O)(OM₁)(OM₂), OCH₂OP(O)(OM₁)(OM₂), OSO₃M₁, or O-glycoside (glucoside, galactoside, mannoside, glucuronoside, alloside, fructoside, etc), NH-glycoside, S-glycoside, or CH₂-glycoside; M₁ and M₂ are independently H, Na, K, Ca, Mg, NH₄, or NR₁R₂R₃R₄; R₁, R₂, R₃, and R₄ are the same defined in in Formula (I).

The benzodiazepine dimers (e. g. dimmers of pyrrolobenzodiazepine (PBD) or (tomaymycin), indolinobenzodiazepines, imidazobenzothiadiazepines, or oxazolidinobenzodiazepines) which are preferred cytotoxic agents according to the present invention are exampled in the art: US Patent Nos. 8,163,736; 8,153,627; 8,034,808; 7,834,005; 7,741,319; 7,704,924; 7,691,848; 7,678,787; 7,612,062; 7,608,615; 7,557,099; 7,528,128; 7,528,126; 7,511,032; 7,429,658; 7,407,951; 7,326,700; 7,312,210; 7,265,105; 7,202,239; 7,189,710; 7,173,026; 7,109,193; 7,067,511; 7,064,120; 7,056,913; 7,049,311; 7,022,699; 7,015,215; 6,979,684; 6,951,853; 6,884,799; 6,800,622; 6,747,144; 6,660,856; 6,608,192; 6,562,806; 6,977,254; 6,951,853; 6,909,006; 6,344,451; 5,880,122; 4,935,362; 4,764,616; 4,761,412; 4,723,007; 4,723,003; 4,683,230; 4,663,453; 4,508,647; 4,464,467; 4,427,587; 4,000,304; US patent appl. 20100203007, 20100316656, 20030195196. Examples of the structures of the conjugate of the antibody- benzodiazepine dimers via the bridge linker are as the following PB01, PB02, PB03, PB04, PB05, PB06, PB07, PB08, PB09, PB10 and PB11 as following: 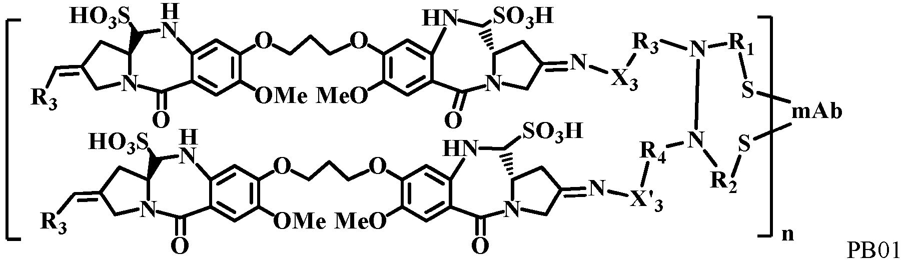 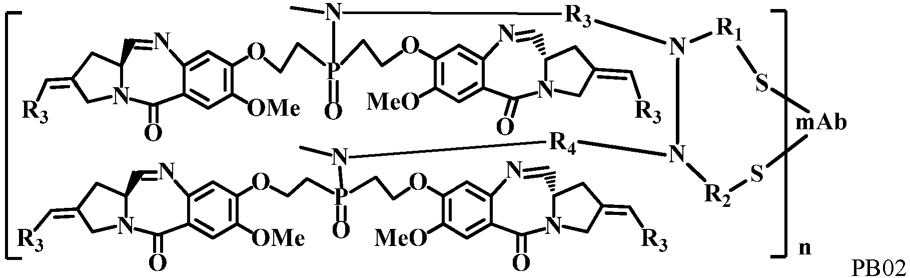 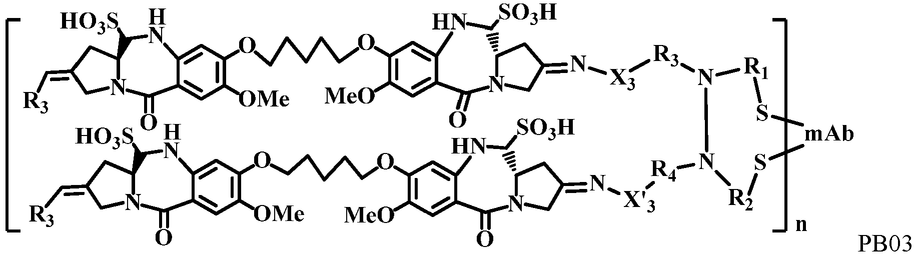 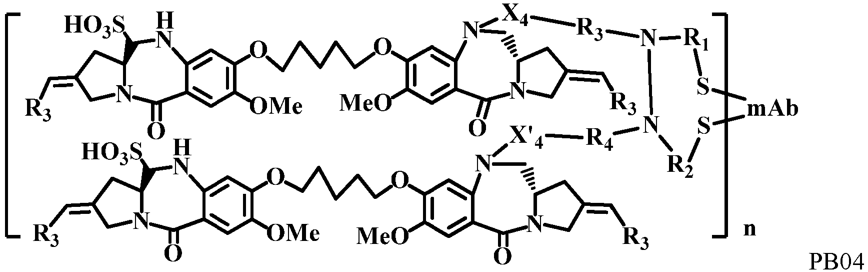 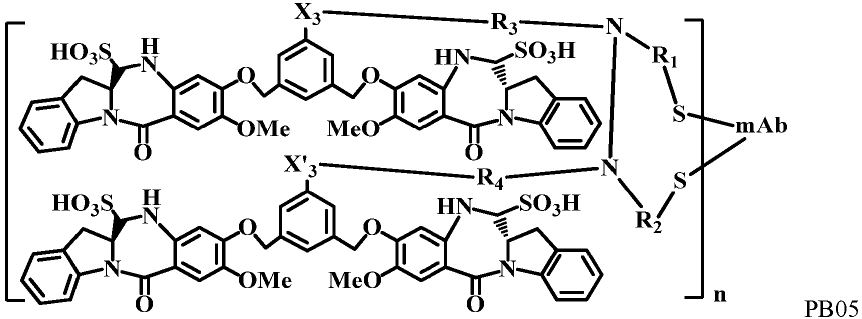 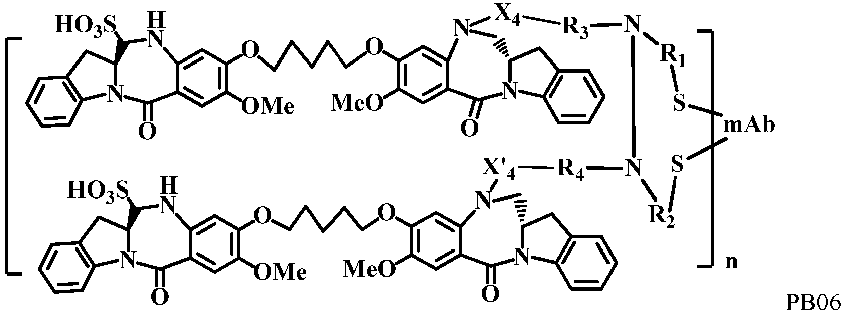 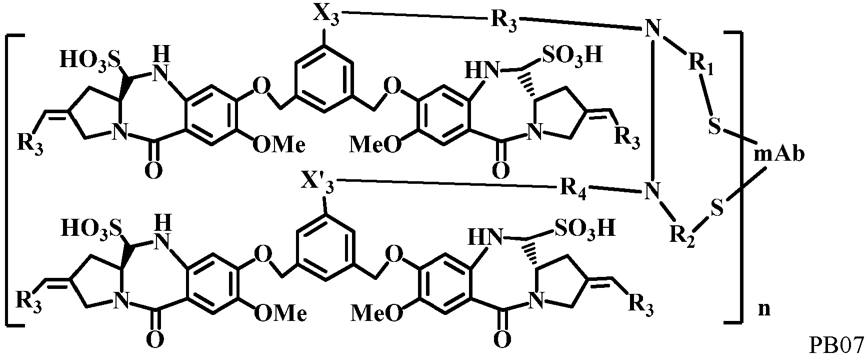 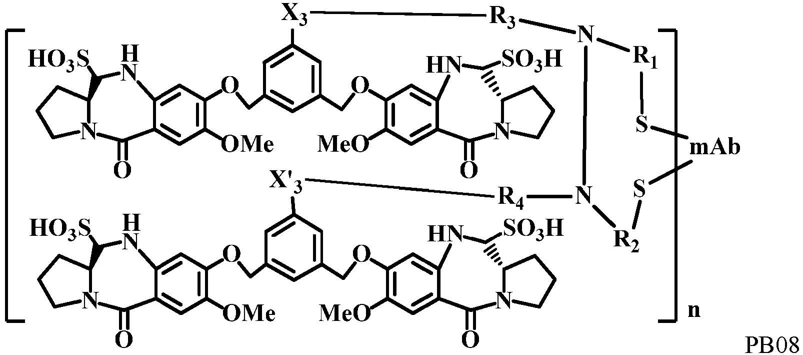 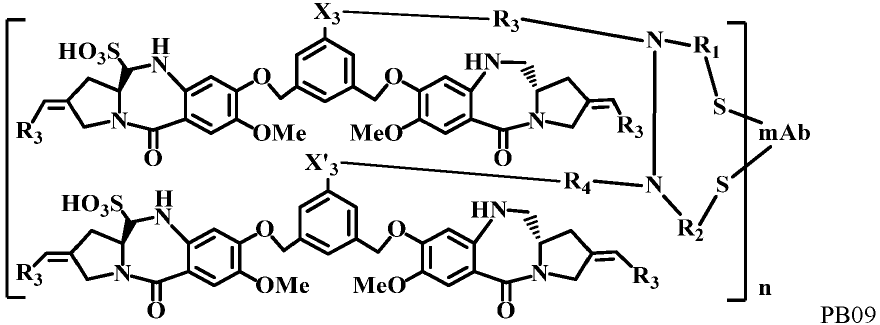 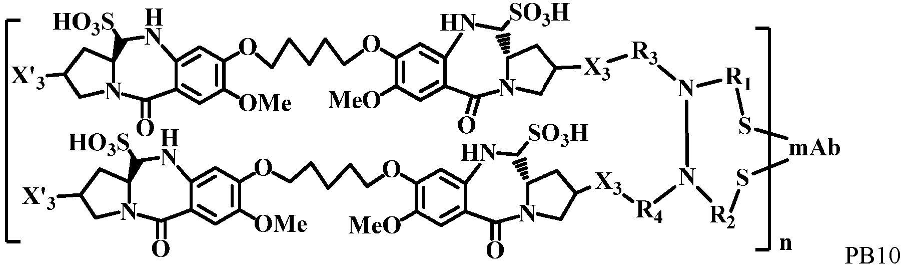 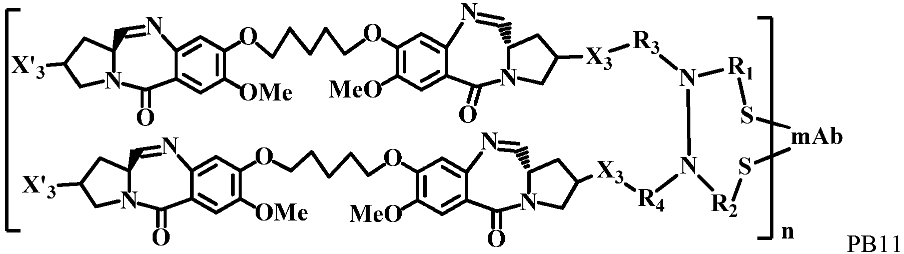 Wherein mAb is an antibody; n is 1~20; X₃ and X'₃ are independently CH₂, O, NH, NR₁, NHC(O), NHC(O)NH, C(O), C(O)R₁, OC(O), C(O)N(R₁), R₁, or absent; X₄ and X'₄ are independently CH₂, C(O), C(O)NH, C(O)N(R₁), C(O)O, R₁, or absent; R₁, R₂, R₃, and R₄ are the same defined in Formula (I). In addition, R₃ and/or R₄ can be absent.

The drugs/ cytotoxic agents used for conjugation via a bridge linker of the present patent can be any analogues and/or derivatives of drugs/molecules described in the present patent. One skilled in the art of drugs/cytotoxic agents will readily understand that each of the drugs/cytotoxic agents described herein can be modified in such a manner that the resulting compound still retains the specificity and/or activity of the starting compound. The skilled artisan will also understand that many of these compounds can be used in place of the drugs/cytotoxic agents described herein. Thus, the drugs/cytotoxic agents of the present invention include analogues and derivatives of the compounds described herein.

### EXAMPLES

The invention is further described in the following examples, which are not intended to limit the scope of the invention. Cell lines described in the following examples were maintained in culture according to the conditions specified by the American Type Culture Collection (ATCC) or Deutsche Sammlung von Mikroorganismen und Zellkulturen GmbH, Braunschweig, Germany (DMSZ), or The Shanghai Cell Culture Institute of Chinese Acadmy of Science, unless otherwise specified. Cell culture reagents were obtained from Invitrogen Corp., unless otherwise specified. All anhydrous solvents were commercially obtained and stored in Sure-seal bottles under nitrogen. All other reagents and solvents were purchased as the highest grade available and used without further purification. The preparative HPLC separations were performed with Varain PreStar HPLC. NMR spectra were recorded on Varian Mercury 400 MHz Instrument. Chemical shifts (delta) are reported in parts per million (ppm) referenced to tetramethylsilane at 0.00 and coupling constants (J) are reported in Hz. The mass spectral data were acquired on a Waters Xevo Qtof mass spect equipped with Waters Acquity UPLC separations module and Acquity TUV detector.

### Example 1. tert-Butyl 3-((benzyloxy)amino)propanoate (3).

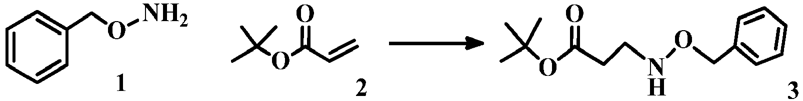

O-benzylhydroxylamine hydrochloride salt (10.0 g, 62.7 mmol) in THF (100 ml) was added Et₃N (15 ml) and tert-butyl acrylate (12.1 g, 94.5 mmol). The mixture was refluxed for overnight, concentrated and purified on SiO₂ column eluted with EtOAc/Hexane (1:4) to afford the title compound 3 (13.08 g, 83% yield). 1H NMR (CDCl₃) 7.49~7.25 (m, 5H), 4.75 (s, 2H), 3.20 (t, J=6.4Hz, 2H), 2.54 (t, J=6.4Hz, 2H), 1.49 (s, 9H); ESI MS m/z+ C₁₄H₂₁NNaO₃ (M+Na), cacld. 274.15, found 274.20.

### Example 2. tert-Butyl 3-(hydroxyamino)propanoate (4).

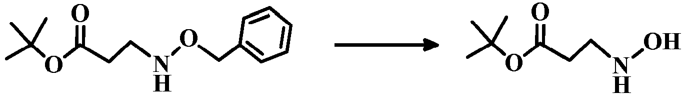

Compound **3** (13.0 g, 51.76 mmol) in methanol (100 ml) was added Pd/C (0.85 g, 10%Pd, 50% wet) in a hydrogenation vessel. After the system was evacuated under vacuum and placed under 2 atm of hydrogen gas, the reaction mixture was stirred overnight at room temperature. The crude reaction was passed through a short pad of celite rinsing with ethanol, concentrated and purified on SiO₂ column eluted with MeOH/DCM (1:10~1:5) to afford the title compound (7.25 g, 87% yield). 1H NMR (CDCl₃) 3.22 (t, J=6.4Hz, 2H), 2.55 (t, J=6.4Hz, 2H), 1.49 (s, 9H); ESI MS m/z+ C₇H₁₅NNaO₃ (M+Na), cacld. 184.10, found 184.30.

### Example 3. tert-Butyl 3-((tosyloxy)amino)propanoate (5).

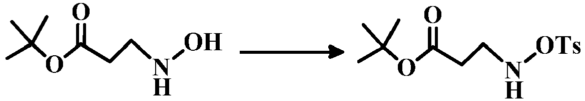

Compound 4 (5.10 g, 31.65 mmol) in the mixture of DCM (50 ml) and pyridine (20 ml) was added tosylate chloride (12.05 g, 63.42) at 4°C. After addition, the mixture was stirred at room temperature overnight, concentrated and purified on SiO₂ column eluted with EtOAc/DCM (1:10~1:6) to afford the title compound (8.58 g, 86% yield). 1H NMR (CDCl₃) 7.81 (s, 2H), 7.46 (s, 2H), 3.22 (t, J=6.4Hz, 2H), 2.55 (t, J=6.4Hz, 2H), 2.41 (s, 3H), 1.49 (s, 9H); ESI MS m/z+ C₁₄H₂₁NNaO₅S (M+Na), cacld. 338.11, found 338.30.

### Example 4. di-tert-Butyl 3,3'-(hydrazine-1,2-diyl)dipropanoate (7).

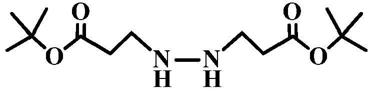

Tert-butyl 3-aminopropanoate **6** (3.05 g, 21.01 mmol) in THF (80 ml) was added tert-Butyl 3-((tosyloxy)amino)propanoate **5** (5.10 g, 16.18 mmol). The mixture was stirred at room temperature for 1 h and then 45 °C for 6 h. The mixture was concentrated and purified on SiO₂ column eluted with CH₃OH/DCM/Et₃N (1:12:0.01~1:8:0.01) to afford the title compound (2.89 g, 62% yield). ESI MS m/z+ C₁₄H₂₈N₂NaO₄ (M+Na), cacld. 311.20, found 311.40.

### Example 5. di-tert-Butyl 3,3'-(1,2-bis(3-(2,5-dioxo-2,5-dihydro-1H-pyrrol-1-yl)propanoyl)hydrazine-1,2-diyl)dipropanoate (13).

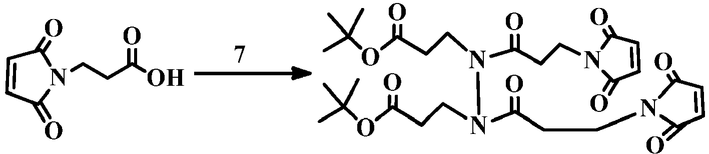

3-Maleido-propanoic acid (1.00 g, 5.91 mmol) in DCM (50 ml) was added oxalyl dichloride (2.70 g, 21.25 mmol) and DMF (50 µL). The mixture was stirred at room temperature for 2 h, evaporated, and co-evaporated with DCM/toluene to obtain crude 3-maleido-propanoic acid chloride. To the compound 7 (0.51 g, 1.76 mmol) in the mixture of DCM (35 ml) was added the crude 3-maleido-propanoic acid chloride. The mixture was stirred for overnight, evaporated, concentrated and purified on SiO₂ column eluted with EtOAc/DCM (1:15~1:8) to afford the title compound **13** (738 mg, 71% yield). ESI MS m/z+ C₂₃H₃₈N₄NaO₁₀ (M+Na), cacld. 613.26, found 613.40.

### Example 6. 3,3'-(1,2-bis(3-(2,5-dioxo-2,5-dihydro-1H-pyrrol-1-yl)propanoyl)-hydrazine-1,2-diyl)dipropanoic acid (14)

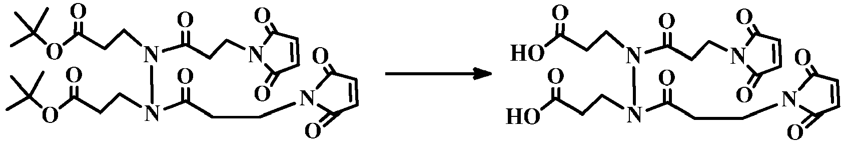

Compound 14 (700 mg, 1.18 mmol) in dioxane (4 ml) was added HCl (conc. 1 ml). The mixture was stirred for 30 min, diluted with EtOH (10 mL) and toluene (10 ml), evaporated and coevaporated with EtOH (10 ml) and toluene (10 ml) to afford the crude title product (560 mg) for next step without further purification. ESI MS m/z-C₂₀H₂₁N₄O₁₀ (M-H), cacld. 477.13, found 477.20.

### Example 7. Bis(2,5-dioxopyrrolidin-1-yl)-3,3'-(1,2-bis(3-(2,5-dioxo-2,5-dihydro-1H-pyrrol-1-yl)propanoyl)hydrazine-1,2-diyl)dipropanoate (79).

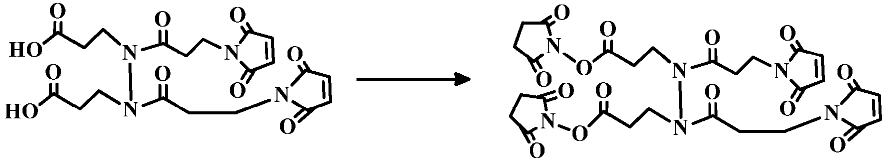

To the crude product **14** (~560 mg, ~1.17 mmol) in DMA (8 ml) was added NHS (400 mg, 3.47 mmol) and EDC (1.01 g, 5.26 mmol). The mixture was stirred for overnight, evaporated, concentrated and purified on SiO₂ column eluted with EtOAc/DCM (1:12~1:7) to afford the title compound **79** (520 mg, 65% yield in 2 steps). ESI MS m/z+ C₂₈H₂₈N₆NaO₁₄ (M+Na), cacld. 695.17, found 695.40.

### Example 8. tert-Butyl 3-(2-(2-(2-hydroxyethoxy)ethoxy)ethoxy)propanoate (84)

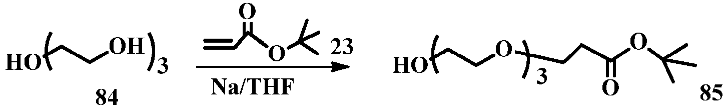

To 350 mL of anhydrous THF was added 80 mg (0.0025 mol) of sodium metal and triethylene glycol **84** (150.1 g, 1.00 mol) with stirring. After the sodium had completely dissolved, tert-butyl acrylate (24 mL, 0.33 mol) was added. The solution was stirred for 20 h at room temperature and neutralized with 8 mL of 1.0 M HCl. The solvent was removed in vacuo and the residue was suspended in brine (250 mL) and extracted with ethyl acetate (3 x 125 mL). The combined organic layers were washed with brine (100 mL) then water (100 mL), dried over sodium sulfate, and the solvent was removed. The resulting colorless oil was dried under vacuum to give 69.78 g (76% yields) of product **85.** ¹H NMR: 1.41 (s, 9H), 2.49 (t, 2H, J=6.4 Hz), 3.59-3.72 (m, 14H). ESI MS m/z- C₁₃H₂₅O₆ (M-H), cacld. 277.17, found 277.20.

### Example 9. tert-Butyl 3-(2-(2-(2-(tosyloxy)ethoxy)ethoxy)ethoxy)propanoate (35)

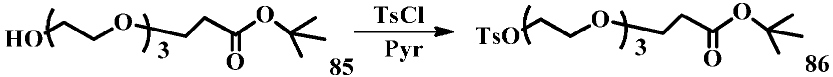

A solution of 85 (10.0 g, 35.95 mmol) in acetonitrile (50.0 mL) was treated with pyridine (20.0 mL). A solution of tosyl chloride (7.12 g, 37.3 mmol) in 50 mL acetonitrile was added dropwise via an addition funnel over 30 minutes. After 5 h TLC analysis revealed that the reaction was complete. The pyridine hydrochloride that had formed was filtered off and the solvent was removed. The residue was purified on silica gel by eluting from with 20% ethyl acetate in hexane to with neat ethyl acetate to give 11.2 g (76% yield) of compound **86.** ¹H NMR: 1.40 (s, 9H), 2.40 (s, 3H), 2.45 (t, 2H, J=6.4 Hz), 3.52-3.68 (m, 14H), 4.11 (t, 2H, J=4.8 Hz), 7.30 (d, 2H, J=8.0 Hz), 7.75 (d, 2H, J=8.0 Hz); ESI MS m/z+ C₂₀H₃₃O₈S (M+H), cacld. 433.18, found 433.30.

### Example 10. tert-Butyl 3-(2-(2-(2-azidoethoxy)ethoxy)ethoxy)propanoate (87)

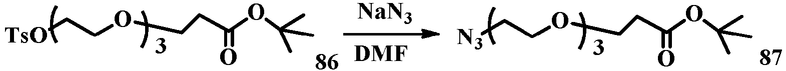

To 50 mL of DMF was added tert-butyl 3-(2-(2-(2-(tosyloxy)ethoxy)ethoxy)ethoxy)-propanoate **86** (4.0 g, 9.25 mmol) and sodium azide (0.737 g, 11.3 mmol) with stirring. The reaction was heated to 80 °C. After 4 h TLC analysis revealed that the reaction was complete. The reaction was cooled to room temperature and quenched with water (25 mL). The aqueous layer was separated and extracted into ethyl acetate (3 x 35 mL). The combined organic layers were dried over anhydrous magnesium sulfate, filtered, and the solvent removed in vacuo. The crude azide **87** (about 90% pure by TLC) was used for next step without further purification. ¹H NMR (CDCl₃): 1.40 (s, 9H), 2.45 (t, 2H, J=6.4 Hz), 3.33 (t, 2H, J=5.2 Hz), 3.53-3.66 (m, 12H). ESI MS m/z+ C₁₃H₂₆N₃O₈ (M+H), cacld. 304.18, found 304.20.

### Example 11. 13-Amino-4,7,10-trioxadodecanoic acid tert-butyl ester, 88; 13-Amino-bis(4,7,10-trioxadodecanoic acid tert-Butyl Ester), 89.

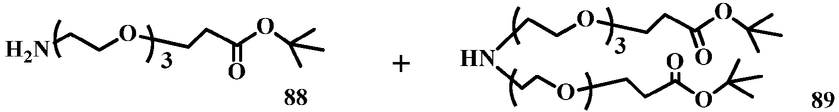

The crude azide material **87** (5.0 g, ~14.84 mmol) was dissolved in ethanol (80 mL) and 300 mg of 10% Pd/C was added. The system was evacuated under vacuum and placed under 2 atm of hydrogen gas via hydrogenation reactor with vigorous stirring. The reaction was then stirred overnight at room temperature and TLC showed that the starting materials disappeared. The crude reaction was passed through a short pad of celite rinsing with ethanol. The solvent was removed and the amine purified on silica gel using a mixture of methanol (from 5% to 15%) and 1% triethylamine in methylene chloride as the eluant to give 13-amino-4,7,10-trioxadodecanoic acid tert-butyl ester **88** (1.83 g, 44% yield, ESI MS m/z+ C₁₃H₂₇NO₅ (M+H), cacld. 278.19, found 278.30) and 13-amino-bis(4,7,10-trioxadodecanoic acid tert-butyl ester), **89** (2.58 g, 32% yield, ESI MS m/z+ C₂₆H₅₂NO₁₀ (M+H), cacld. 538.35, found 538.40).

### Example 12. 3-(2-(2-(2-Aminoethoxy)ethoxy)ethoxy)propanoic acid, HCl salt, 90.

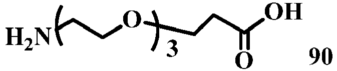

To 13-amino-4,7,10-trioxadodecanoic acid tert-butyl ester, **88** (0.80 g, 2.89 mmol) in 30 mL of dioxane was 10 ml of HCl (36%) with stirring. After 0.5 h TLC analysis revealed that the reaction was complete, the reaction mixture was evaporated, and co-evaporated with EtOH and EtOH/Toluene to form the title product in HCl salt (>90% pure, 0.640 g, 86% yield) without further purification. ESI MS m/z+ C₉H₂₀NO₅ (M+H), cacld. 222.12, found 222.20.

### Example 13. 13-Amino-bis(4,7,10-trioxadodecanoic acid, HCl salt, 91.

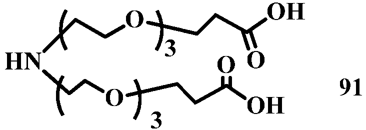

To 13-amino-bis(4,7,10-trioxadodecanoic acid tert-butyl ester), **89** (1.00 g, 1.85 mmol) in 30 mL of dioxane was 10 ml of HCl (36%) with stirring. After 0.5 h TLC analysis revealed that the reaction was complete, the reaction mixture was evaporated, and co-evaporated with EtOH and EtOH/Toluene to form the title product in HCl salt (>90% pure, 0.71 g, 91% yield) without further purification. ESI MS m/z+ C₁₈H₃₆NO₁₀ (M+H), cacld. 426.22, found 426.20.

### Example 14. (S)-2-(Methylamino)propanoic acid, maytansinol ester.

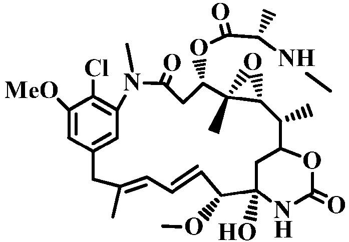

Maytansinol (200 mg, 0.354 mmol) was dissolved in DMF (5 ml) and THF (3 ml) on an ice/water bath was added DIPEA (0.3 ml), zinc triflate (380 mg, 1.05 mmol) and (S)-3,4-dimethyloxazolidine-2,5-dione (92 mg, 0.71 mmol). The reaction mixture was stirred under argon at room temperature for overnight, diluted with EtOAc (20mL), washed with a solution of 1:1 brine/saturated sodium bicarbonate (8 mL). The white precipitate was filtered off, and the resulting aqueous solution was extracted with EtOAc (2 x10mL) and organic layer was washed with brine. The resulting organic layers were reduced under pressure and purified on C-18 preparative HPLC eluted with MeOH/water (10% to 65% of MeOH in 45 min, Φ50 mm x 250 mm, v = 80 ml/min) to obtain the title compound (156 mg, 68% yield). ESI MS m/z+ C₃₂H₄₅ClN₃O₉ (M+H), cacld. 650.28, found 650.20.

### Example 15. (2S,2'S)-2,2'-((3,3'-(1,2-bis(3-(maleimido)propanoyl)hydrazine-1,2-diyl)bis(propanoyl))bis(methylazanediyl))dipropanoic acid, di-maytansinol esters, 108.

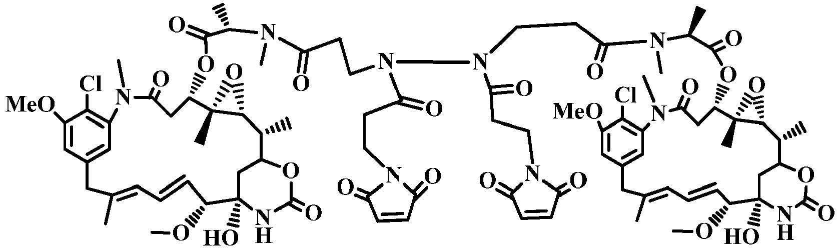

To a stirred solution of (S)-2-(Methylamino)propanoic acid, maytansinol ester (150 mg, 0.231 mmol) and 3,3'-(1,2-bis(maleido)propanoyl)-hydrazine-1,2-diyl)dipropanoic acid **14** (50 mg, 0.104 mmol) in DMA (10 ml) was added EDC (300 mg, 1.56 mmol). The mixture was stirred overnight, evaporated and purified on C-18 preparative HPLC eluted with MeOH/water (10% to 85% of MeOH in 45 min, Φ20 mm x 250 mm, v = 20 ml/min) to afford the title compound **108** (115 mg, 63% yield). ESI MS m/z+ C₈₄H₁₀₇Cl₂N₁₀O₂₆ (M+H), cacld. 1741.67, found 1741.90.

### Example 16. 3,3'-(1,2-bis(3-(maleimido)propanoyl)hydrazine-1,2-diyl)bis(N-MMAF propanamide)

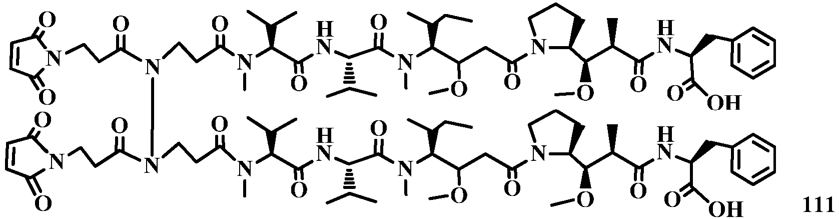

To a stirred solution of 3,3'-(1,2-bis(maleido)propanoyl)-hydrazine-1,2-diyl) dipropanoic acid **14** (50 mg, 0.104 mmol) in DCM (4 ml) was added oxalyl dichloride (0.4 ml, 2 M in DCM, 0.8 mmol) and DMF (10 µl). The mixture was stirred at room temperature for 2 h, evaporated, and co-evaporated with DCM/toluene to obtain crude 3,3'-(1,2-bis(maleido)propanoyl)-hydrazine-1,2-diyl)dipropanoic acid chloride **107.** To a solution of MMAF **110** (160 mg, 0.219 mmol) in DCM (5 ml) was added the crude acid chloride **107.** The mixture was stirred for overnight, evaporated, concentrated and purified on C-18 preparative HPLC eluted with MeOH/water (10% to 85% of MeOH in 45 min, Φ20 mm x 250 mm, v = 20 ml/min) to afford the title compound **111** (98 mg, 49% yield). ESI MS m/z+ C₉₈H₁₄₉Cl₂N₁₄O₂₄ (M+H), cacld. 1906.08, found 1906.50.

### Example 17. 3,3'-(1,2-bis(3-(maleimido)propanoyl)hydrazine-1,2-diyl)bis(propanamide of N-tubulysin analog), 119.

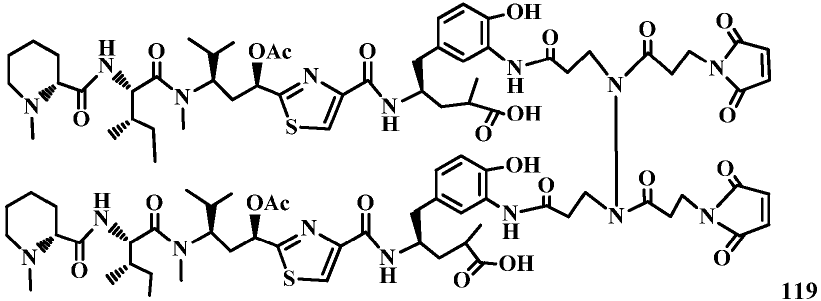

To a stirred solution of 3,3'-(1,2-bis(maleido)propanoyl)-hydrazine-1,2-diyl) dipropanoic acid **14** (50 mg, 0.104 mmol) in DCM (4 ml) was added oxalyl dichloride (0.4 ml, 2 M in DCM, 0.8 mmol) and DMF (10 µl). The mixture was stirred at room temperature for 2 h, evaporated, and co-evaporated with DCM/toluene to obtain crude 3,3'-(1,2-bis(maleido)propanoyl)-hydrazine-1,2-diyl)dipropanoic acid chloride **107.** To a solution of added (4R)-4-(2-((1R,3R)-1-acetoxy-3-((2S,3S)-N,3-dimethyl-2-((R)-1-methylpiperidine-2-carboxamido)pentanamido)-4-methylpentyl)thiazole-4-carboxamido)-5-(3-amino-4-hydroxyphenyl)-2-methylpentanoic acid, **118** (Huang Y. et al, Med Chem. #44, 249th ACS National Meeting, Denver, CO, Mar. 22~26, 2015; WO2014009774) (172 mg, 0.226 mmol) in DCM (5 ml) was added the crude acid chloride **107.** The mixture was stirred for overnight, evaporated, concentrated and purified on C-18 preparative HPLC eluted with MeOH/water (10% to 80% of MeOH in 45 min, Φ20 mm x 250 mm, v = 20 ml/min) to afford the title compound **119** (106 mg, 52% yield). ESI MS m/z+ C₉₇H₁₃₉Cl₂N₁₆O₂₄S₂ (M+H), cacld. 1975.95, found 1976.50.

### Example 15. Conjugated compound 108, 111 or 119 to an antibody for 109, 112 or 120 independently.

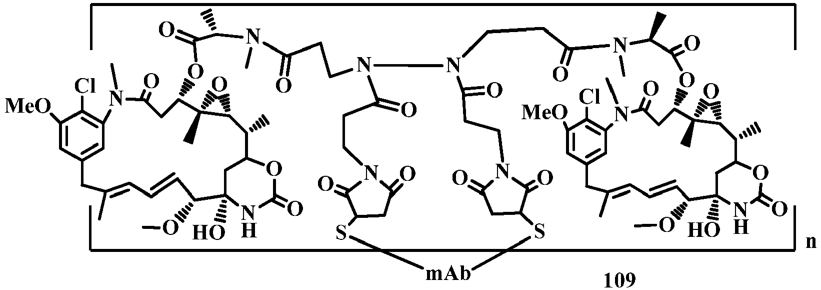

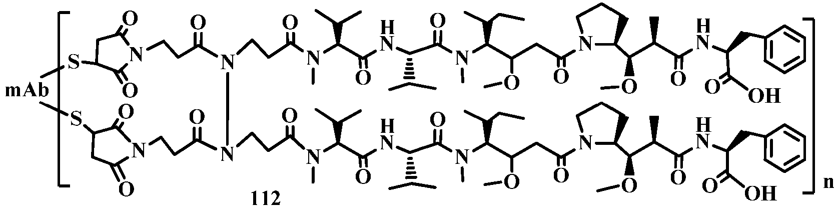

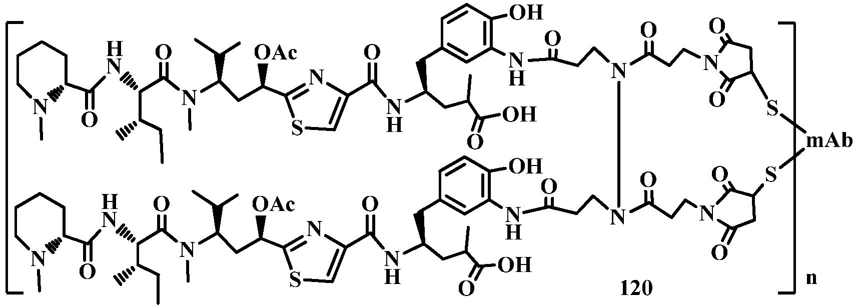

To a mixture of 2.0 mL of 10 mg/ml Herceptin in pH 6.0~8.0, were added of 0.70 ~ 2.0 mL PBS buffer of 100 mM NaH₂PO₄, pH 6.5~7.5 buffers, TCEP (28 µL, 20 mM in water) and the compound **108, 111 or 119** (14 µL, 20 mM in DMA) independently. The mixture was incubated at RT for 2~16 h, then DHAA (135 µL, 50 mM) was added in. After continuous incubation at RT overnight, the mixture was purified on G-25 column eluted with 100 mM NaH₂PO₄, 50 mM NaCl pH 6.0~7.5 buffer to afford the conjugate compound **109, 112 or 120** independently in 13.0~15.6 ml buffer. The drug/antibody ratio (DAR) was determined via UPLC-Qtof mass spectrum. They were 95~99% monomer analyzed by SEC HPLC (Tosoh Bioscience, Tskgel G3000SW, 7.8 mm ID x 30 cm, 0.5 ml/min, 100 min) and a single band measured by SDS-PAGE gel.

Conjugate **109,** 15.8 mg (79% yield), DAR = 3.90, 95% monomer; Conjugate **112,** 16.7 mg (83% yield), DAR = 3.95, 96% monomer; Conjugate **120,** 16.3 mg (81% yield), DAR = 3.96, 97% monomer.

### Example 18. In vitro cytotoxicity evaluation of conjugates 109, 112 and 120 in comparison withT-DM1:

The cell lines used in the cytotoxicity assays were HL-60, a human promyelocytic leukemia cell line; NCI-N87, a human gastric carcinoma cell line; and SKOV3, a human ovarian carcinoma cell line. For HL-60, and NCI-N87, the cells were grown in RPMI-1640 with 10% FBS. For SKOV3 cells, the cells were grown in McCoy's 5A Medium with 10% FBS. To run the assay, the cells (180 µl, 6000 cells) were added to each well in a 96-well plate and incubated for 24 hours at 37°C with 5% CO₂. Next, the cells were treated with test compounds (20 µl) at various concentrations in appropriate cell culture medium (total volume, 0.2 mL). The control wells contain cells and the medium but lack the test compounds. The plates were incubated for 120 hours at 37°C with 5% CO₂. MTT (5mg/ml) was then added to the wells (20 µl) and the plates were incubated for 1.5hr at 37°C. The medium was carefully removed and DMSO (180 µl) was added afterward. After it was shaken for 15min, the absorbance was measured at 490nm and 570nm with a reference filter of 620nm. The inhibition% was calculated according to the following equation: inhibition% = [1-(assay-blank)/(control-blank)] × 100.

### The cytotoxicity results:

| IC₅₀ (nM) | N87 cell (Ag+) | SK-OV-3 cell (Ag+) | HL60 cell (Ag-) |
|---|---|---|---|
| Conjugate **109** | 0.161 nM | 0.118 nM | >15 nM |
| Conjugate **112** | 0.101 nM | 0.088 nM | >10 nM |
| Conjugate **120** | 0.095 nM | 0.073 nM | >10 nM |
| T-DM1 | 0.266 nM | 0.177 nM | >15 nM |

All three conjugates were more potent than T-DM1. Specificity of conjugate **109** for N87 cell was over 93 and for SK-OV-3 cell was over 127; Specificity of conjugate **112** for N87 cell was over 99 and for SK-OV-3 cell was over 113; Specificity of conjugate **120** for N87 cell was over 105 and for SK-OV-3 cell was over 136; Specificity of conjugate **T-DM1** for N87 cell was over 56 and for SK-OV-3 cell was over 84.

## Claims

1. A product that is:
(A) a cell-binding molecule-linker-drug conjugate represented as 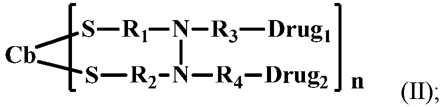 or
(B) a compound of formula (IV) 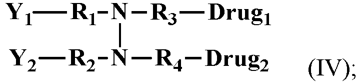 wherein:
Y₁ and Y₂ are
are selected from the structures displayed below: 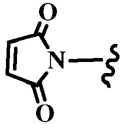 maleimide; 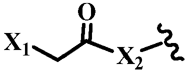 haloacetyl; and 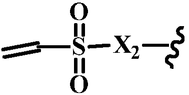 ethenesulfonyl; wherein X₁ is F, Cl, Br, I or Lv; X₂ is O, NH, N(R₁), or CH₂; Lv is selected from nitrophenol; N-hydroxysuccinimide (NHS); phenol; dinitrophenol; pentafluorophenol; tetrafluorophenol; difluorophenol; monofluorophenol; pentachlorophenol; triflate; imidazole; dichlorophenol; tetrachlorophenol; 1-hydroxybenzo-triazole; tosylate; mesylate; 2-ethyl-5-phenylisoxazolium-3'-sulfonate, or anhydrides; or an intermediate molecule generated with a condensation reagent for peptide coupling reactions or for Mitsobonu reactions,
R₁, R₂, R₃ and R₄ are the same or different, and are absent, linear alkyl having from 1-8 carbon atoms, branched or cyclic alkyl having from 3 to 8 carbon atoms, linear, branched or cyclic alkenyl or alkynyl, or 1~8 carbon atoms of esters, ether, amide, or polyethyleneoxy unit of formula (OCH₂CH₂)ₚ, wherein p is an integer from 0 to 1000, or combination thereof; and
additionally R₁, R₂, R₃ and R₄ are respectively a chain of atoms selected from C, N, O, S, Si, and P, which covalently connects to Y₁ or Y₂ and Z₁ or Z₂, wherein the atoms used in forming the R₁, R₂, R₃ and R₄ may be combined in all chemically relevant ways;
Cb represents a cell-binding agent;
Drug₁ and Drug₂ represent the same or different drugs/cytotoxic agents, linked to the cell-binding agent via the bridge linker through an alkylene, alkenylene, alkynylene, ether, polyoxyalkylene, ester, amine, imine, polyamine, hydrazine, hydrazone, amide, urea, semicarbazide, carbazide, alkoxyamine, urethanes, amino acid, peptide, acyloxylamine, hydroxamic acid, disulfide, thioether, thioester, carbamate, carbonate, heterocyclic ring, heteroaromatic, or alkoxime bond; and
n is an integer from 1 to 20.

2. The product of claim 1, wherein "Drug₁" and "Drug₂" are selected from tubulysins, calicheamicins, auristatins, maytansinoids, CC-1065 analogs, daunorubicin and doxorubicin compounds, taxanoids (taxanes), cryptophycins, epothilones, benzodiazepine dimers (e.g., dimers of pyrrolobenzodiazepine (PBD), tomaymycin, anthramycin, indolinobenzodiazepines, imidazobenzothiadiazepines, or oxazolidinobenzodiazepines), calicheamicins and the enediyne antibiotics, actinomycin, azaserines, bleomycins, epirubicin, tamoxifen, idarubicin, dolastatins/auristatins (e.g. monomethyl auristatin E, MMAE, MMAF, auristatin PYE, auristatin TP, Auristatins 2-AQ, 6-AQ, EB (AEB), and EFP (AEFP)), duocarmycins, thiotepa, vincristine, hemiasterlins, nazumamides, microginins, radiosumins, alterobactins, microsclerodermins, theonellamides, esperamicins, siRNA, nucleolytic enzymes, and/or pharmaceutically acceptable salts, and/or acids of any of the above molecules.

3. The product of claim 1 wherein the cell binding agent/molecule is selected from an antibody, a protein, a vitamin (e.g. folate), peptides, a polymeric micelle, a liposome, a lipoprotein-based drug carrier, a nano-particle drug carrier, a dendrimer, and a molecule above coated with cell-binding ligands, or a combination thereof.

4. The product according to claim 1, wherein the cell-binding agent or compound is able to target against a tumor cell, a virus infected cell, a microorganism infected cell, a parasite infected cell, an autoimmune disease cell, an activated tumor cell, a myeloid cell, an activated T-cell, an affecting B cell, or a melanocyte.

5. The product according to claim 4, wherein the tumor cell is selected from lymphoma cells, myeloma cells, renal cells, breast cancer cells, prostate cancer cells, ovarian cancer cells, colorectal cancer cells, gastric cancer cells, squamous cancer cells, small-cell lung cancer cells, non small-cell lung cancer cells, testicular cancer cells, or any cells that grow and divide at an unregulated, quickened pace to cause cancers.

6. The product according to claim 1, wherein the linkage components R₁ R₂, R₃ and/or R₄ are composed of one or more linker components of: 6-maleimidocaproyl (MC), maleimido propanoyl (MP), valine-citrulline (val-cit), alanine-phenylalanine (ala-phe), lysine-phenylalanine (lys-phe), p-aminobenzyloxycarbonyl (PAB), 4-thio-pentanoate (SPP), 4-(N-maleimidomethyl)-cyclohexane-1-carboxylate (MCC), 4-thio-butyrate (SPDB), maleimidoethyl (ME), 4-thio-2-hydroxysulfonyl-butyrate (2-Sulfo-SPDB), pyridinyldithiol (PySS), alkoxy amino (AOA), ethyleneoxy (EO), 4-methyl-4-dithio-pentanoic (MPDP), azido (N₃), alkynyl, dithio, peptide, and/or (4-acetyl)aminobenzoate (SIAB).

7. The product of claim 1, wherein "Drug₁" and "Drug₂" are:
(A) a Tubulysin analog;
(B) a Calicheamicin analog;
(C) a Maytansinoid analog;
(D) a Taxane analog;
(E) a CC-1065 analog and/or doucarmycin analog;
(F) a Daunorubicin or Doxorubicin analog;
(G) an Auristatin and dolastatin analog; or
(H) a benzodiazepine dimer analog.

8. The product of claim 1, wherein the conjugate compound of Formula (II) is:
(A) the structure of T01, T02, T03, T04, T05, T06 or T07 as follows: 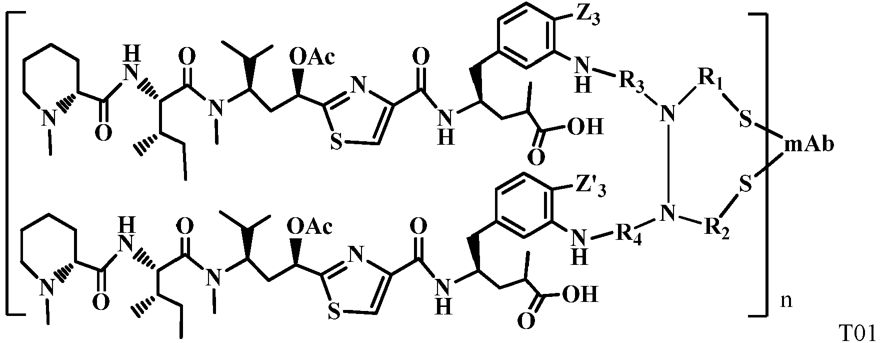 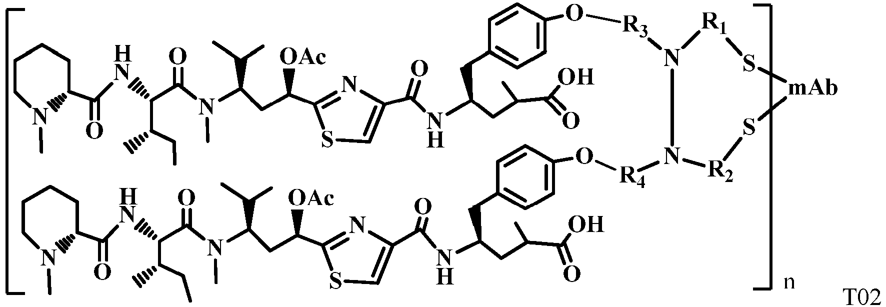 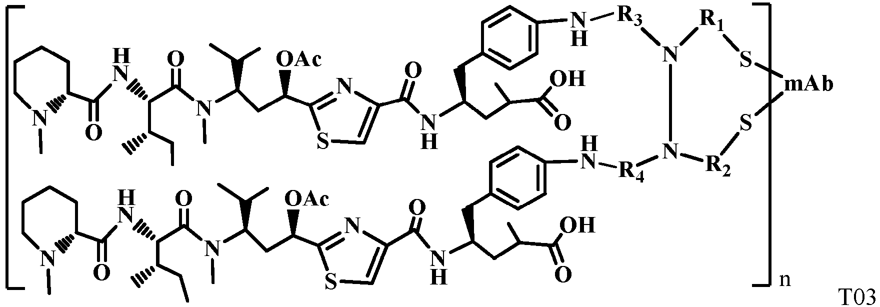 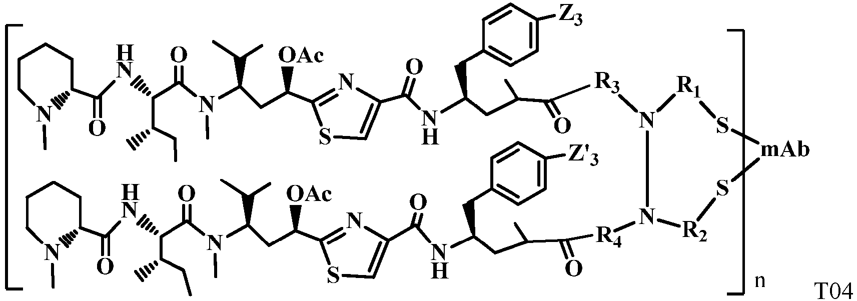 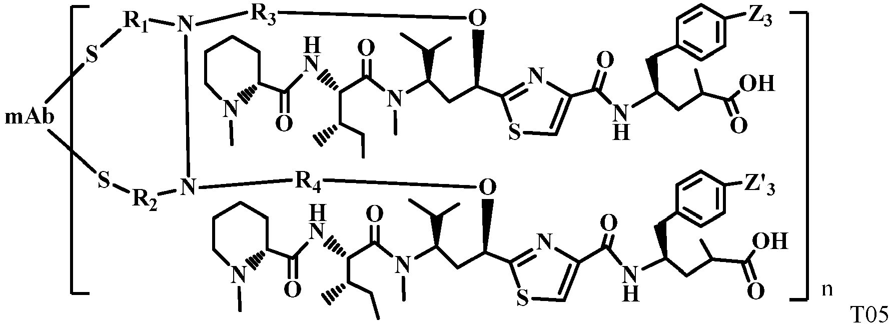 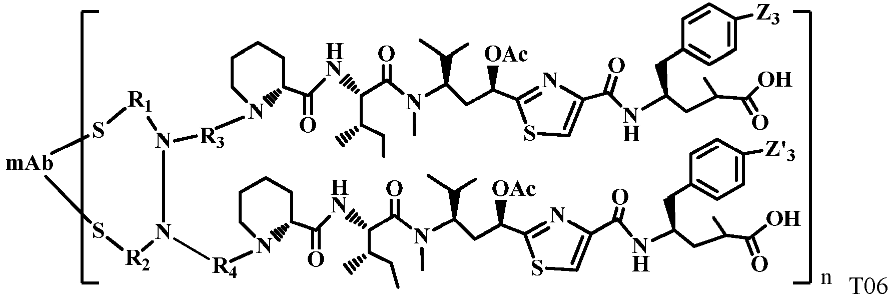 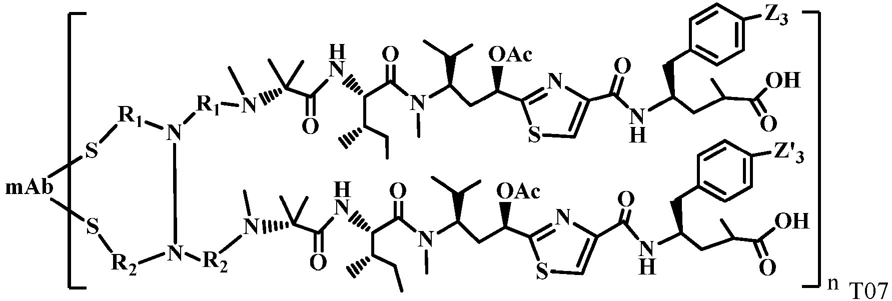 wherein mAb is an antibody; Z₃ is H, R₁, OP(O)(OM₁)(OM₂), OCH₂OP(O)(OM₁)(OM₂), OSO₃M₁, or O-glycoside (glucoside, galactoside, mannoside, glucuronoside, alloside, fructoside, etc), NH-glycoside, S-glycoside, or CH₂-glycoside; M₁ and M₂ are independently H, Na, K, Ca, Mg, NH₄, or NR₁R₂R₃R₄; n is 1~20; R₁, R₂, R₃ and R₄ are the same defined in Claim **1;** or
(B) the structure of C01 as follows: 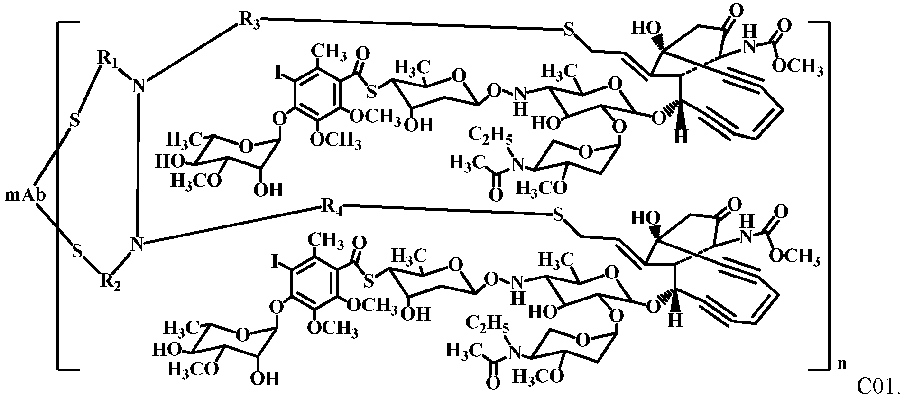 wherein mAb is an antibody; n is 1~20; R₁, R₂, R₃ and R₄ are the same defined in Claim 1; or
(C) the structure of M01 as follows: 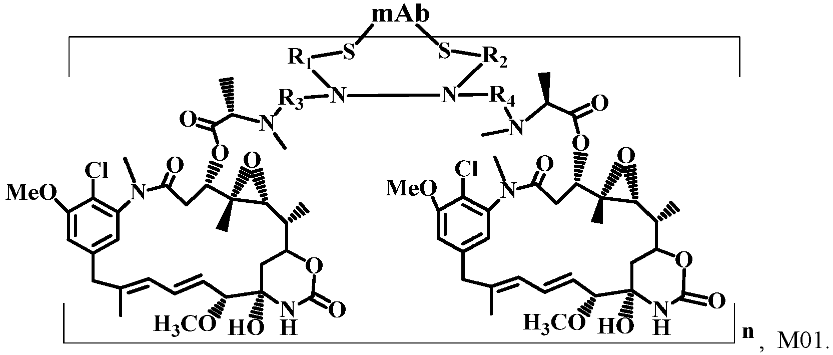 wherein mAb is an antibody; n is 1~20; R₁, R₂, R₃ and R₄ are the same defined in Claim 1; or
(D) the structure of Tx01, Tx02 or Tx03: 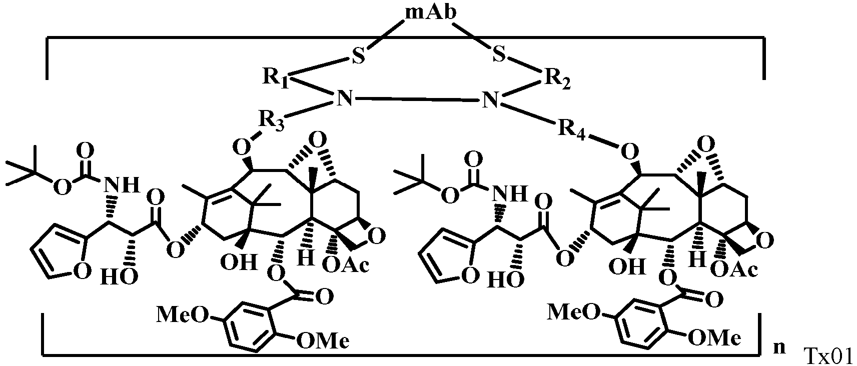 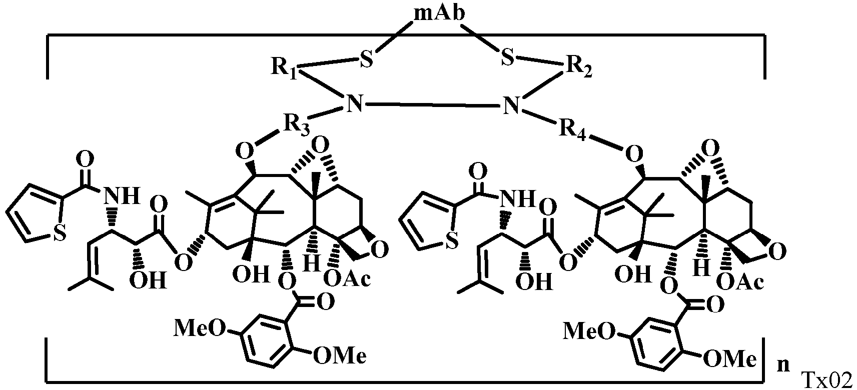 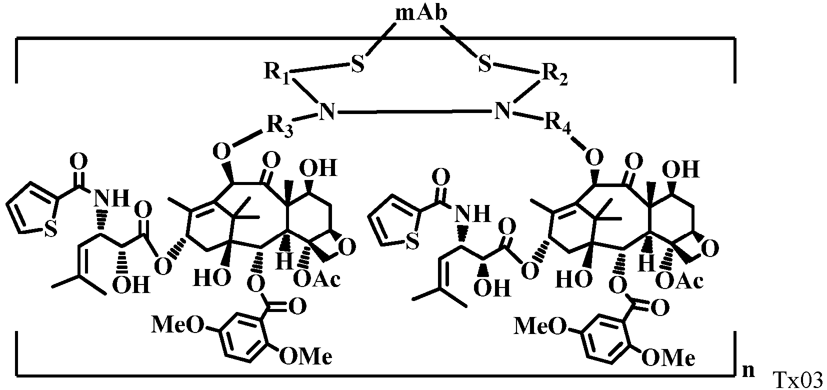 wherein mAb is an antibody; n is 1~20; R₁, R₂, R₃ and R₄ are the same defined in Claim 1; or
(E) the structure of CC01, CC02, and CC03 as follows: 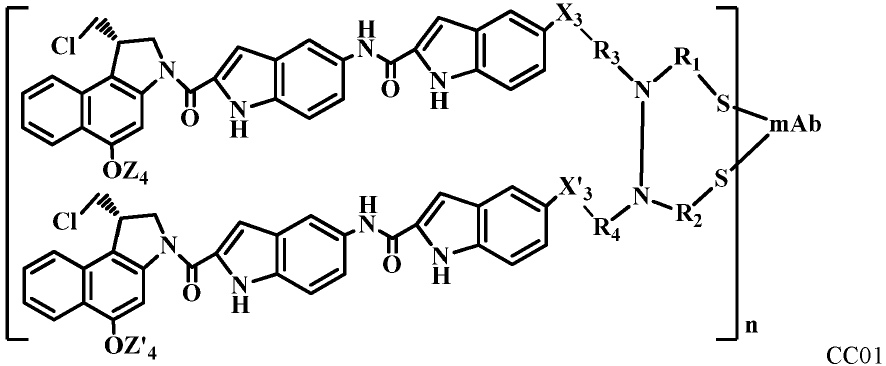 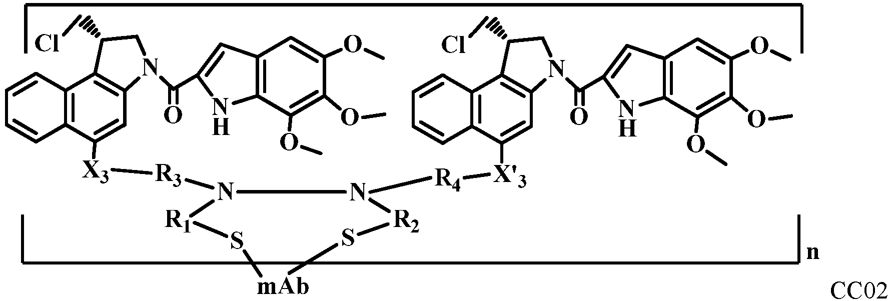 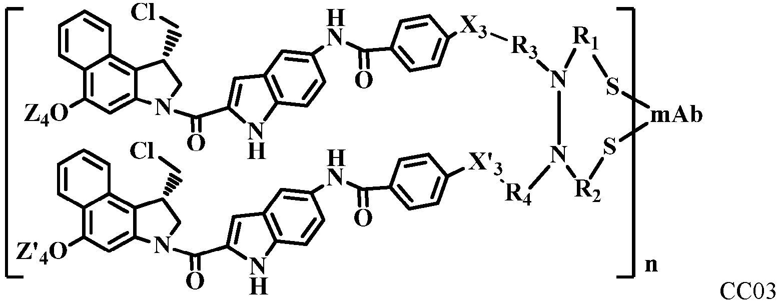 wherein mAb is an antibody; n is 1~20; Z₄ is H, PO(OM₁)(OM₂), SO₃M₁, CH₂PO(OM₁)(OM₂), CH₃N(CH₂CH₂)₂NC(O)-, O(CH₂CH₂)₂NC(O)-, or glycoside; X₃ is O, NH, NR₁, NHC(O), OC(O), CO, R₁, or absent; M₁ and M₂ is independently Na, K, H, NH₄, NR₁R₂R₃R₄; R₁, R₂, R₃, and R₄ are the same defined in Claim 1; or
(F) the structure of Da01, Da02, Da03 or Da04 as follows: 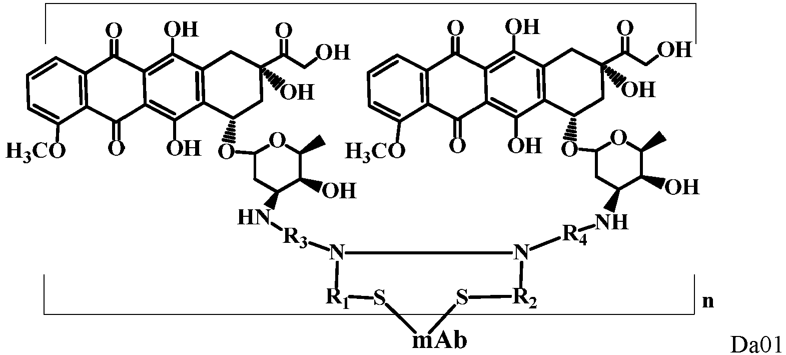 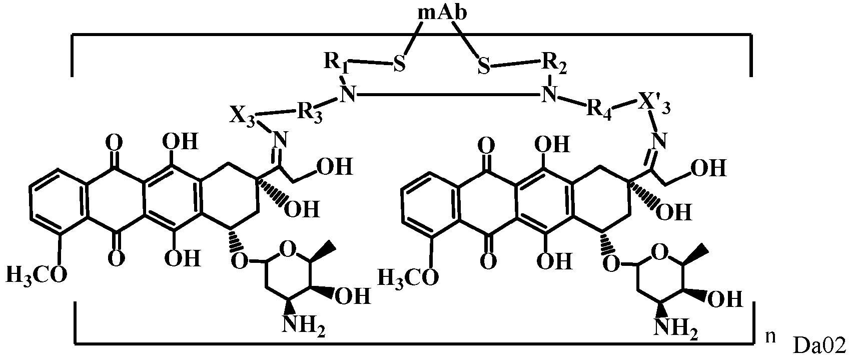 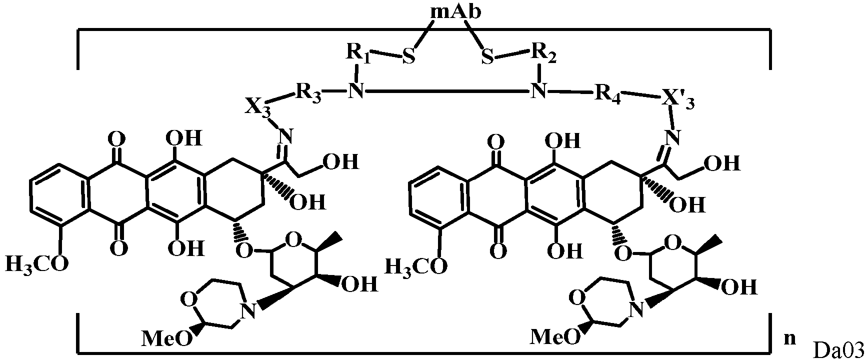 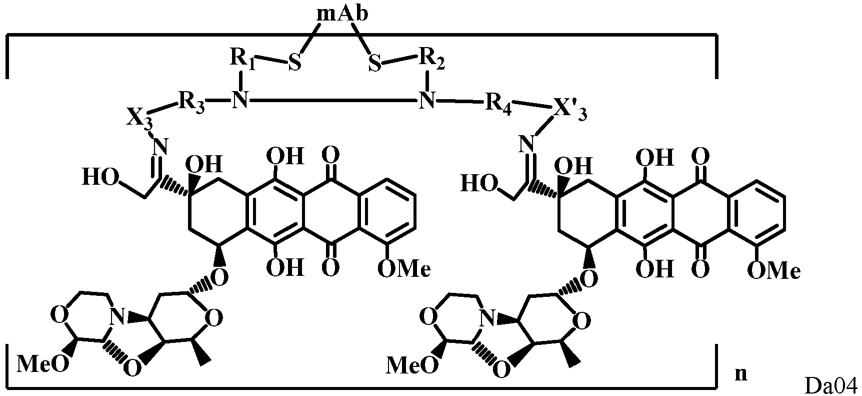 wherein mAb is an antibody; n is 1~20; X₃ is O, NH, NR₁, NHC(O), OC(O), CO, R₁, or absent; R₁, R₂, R₃, and R₄ are the same defined in Claim 1; or
(G) the structure of Au01, Au02, Au03, Au04, or Au05 as follows: 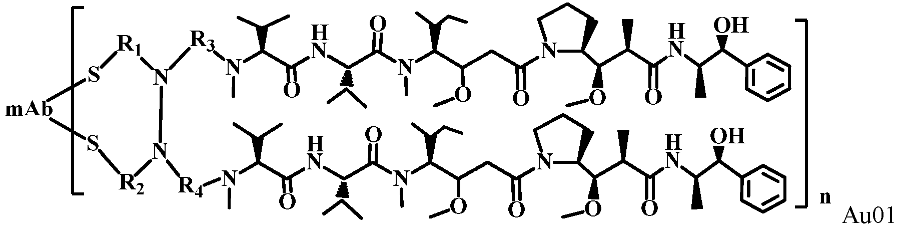 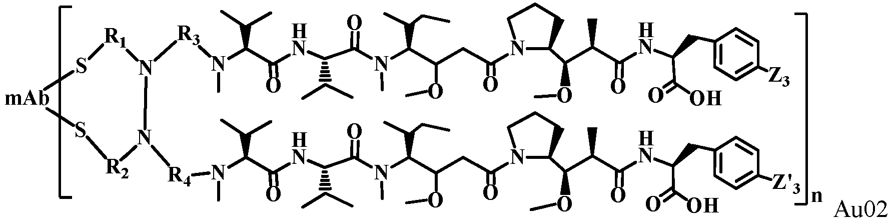 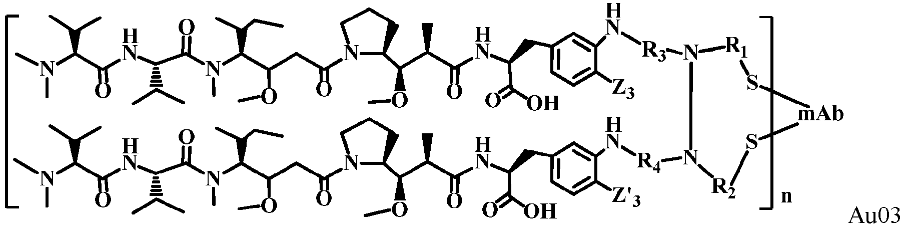 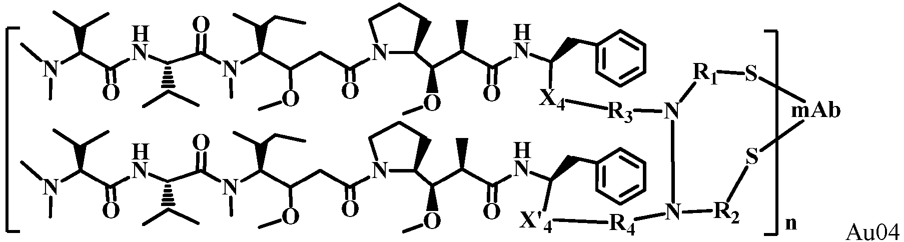 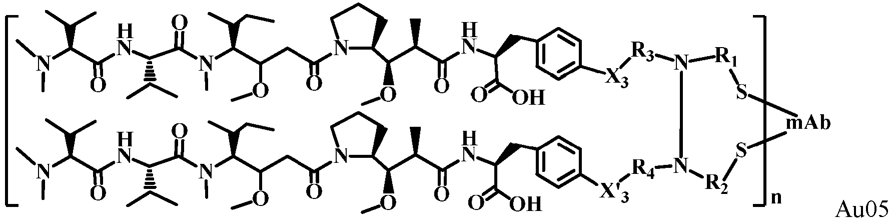 wherein mAb is an antibody; n is 1~20; X₃ is CH₂, O, NH, NR₁, NHC(O), NHC(O)NH, C(O), OC(O), R₁, or absent; X₄ is CH₂, C(O), C(O)NH, C(O)N(R₁), R₁, or C(O)O; R₁, R₂, R₃, and R₄ are the same defined in in Claim **1;** or
(H) the structure of PB01, PB02, PB03, PB04, PB05, PB06, PB07, PB08 and PB09 as follows: 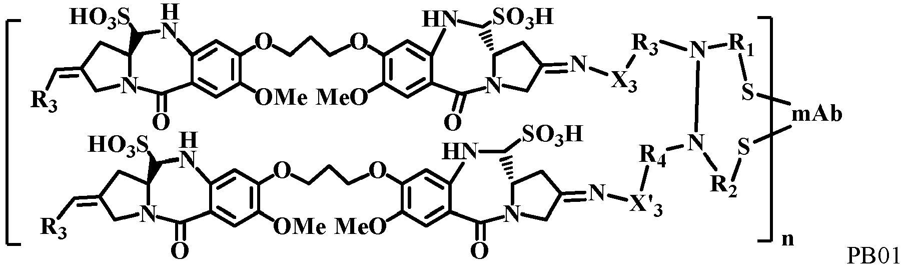 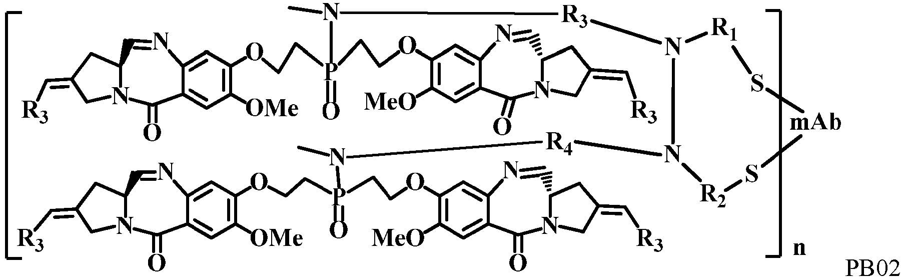 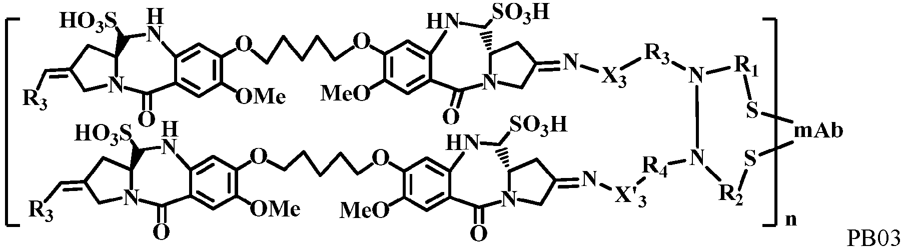 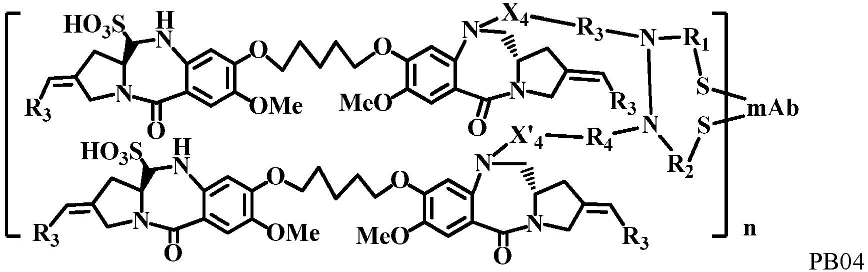 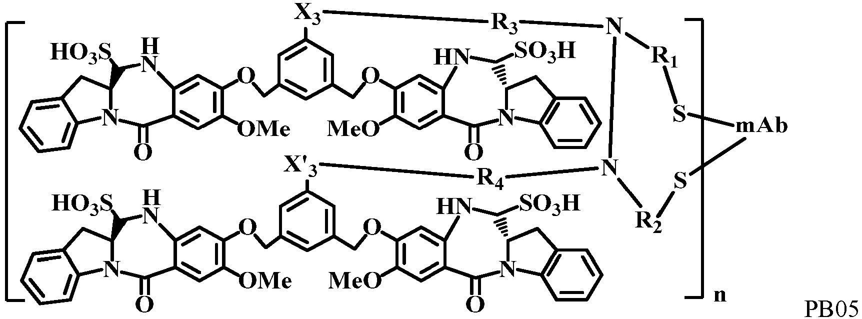 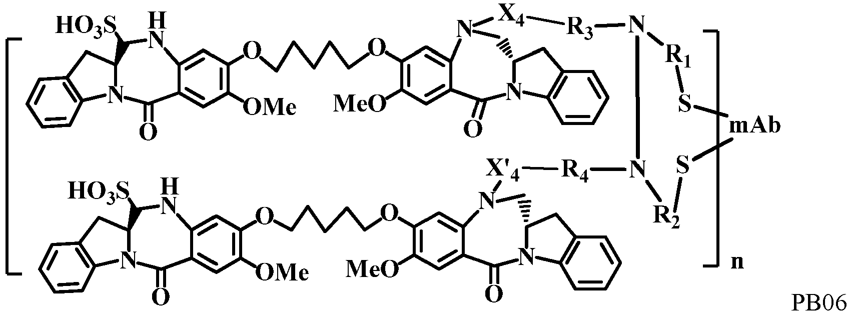 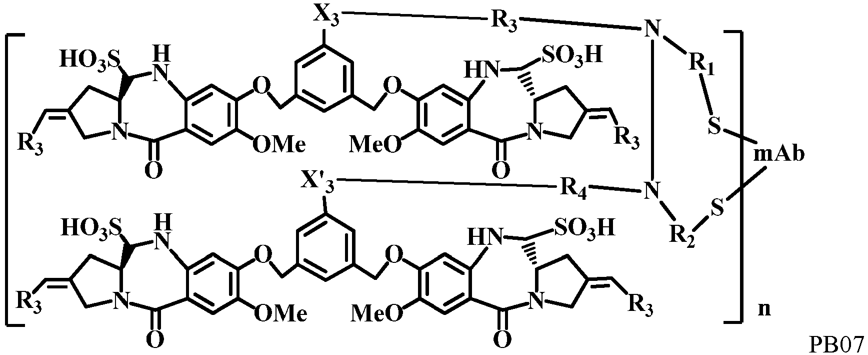 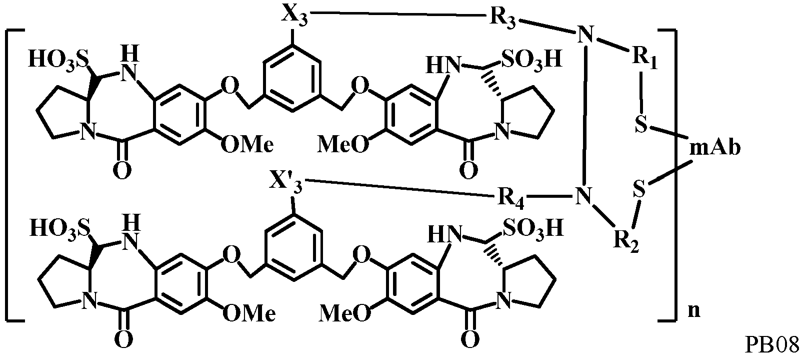 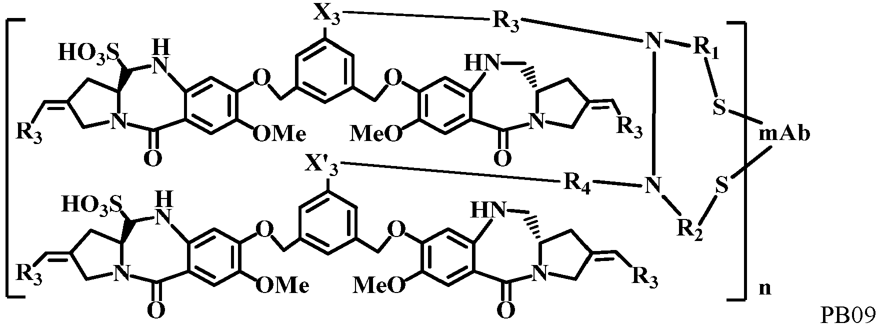 wherein mAb is an antibody; n is 1~20; X₃ is CH₂, O, NH, NR₁, NHC(O), NHC(O)NH, C(O), OC(O), R₁, or absent; X₄ is CH₂, C(O), C(O)NH, C(O)N(R₁), C(O)O, R₁, or absent; R₁, R₂, R₃, and R₄ are the same defined in Claim 1; and R₃ and R₄ may be absent.

9. The product of claim 1 or 7 or 8, wherein the conjugate compound comprises either a peptide of 1~20 units of natural or unnatural amino acids, or a p-aminobenzyl unit, or a 6-maleimidocaproyl unit, or a disulfide unit, or a thioether unit, or a hydrozone unit, a triazole unit, or an alkoxime unit, among the linkage components R₁, R₂, R₃ and/or R₄.

10. The product of claim 1 or 7 or 8, wherein the linkage components R₁, R₂, R₃ and/or R₄ is/are cleavable by a protease.

11. The product of claim 1 or 7 or 8, wherein the linked pairs of sulfur atoms are from the interchain disulfide bonds of an antibody, which are reduced by TCEP and/or DTT molecules to generate the pairs of thiols as the conjugation sites.

12. The product of any of claims 1 to 11, wherein R₁, R₂, R₃ and R₄ are respectively a chain of atoms selected from C, N, O, S, Si, and P, which covalently connects to Y₁ or Y₂ and Z₁ or Z₂, wherein the atoms used in forming the R₁, R₂, R₃ and R₄ may be combined to form alkylate, alkylene, alkenylene, and alkynylene, ethers, polyoxyalkylene, esters, amines, imines, polyamines, hydrazines, hydrazones, amides, ureas, semicarbazides, carbazides, alkoxyamines, alkoxylamines, urethanes, amino acids, peptides, acyloxylamines, hydroxamic acids, or a combination thereof.

13. A pharmaceutical composition comprising a therapeutically effective amount of the conjugate compounds of claim 1 or 7 or 8, and a pharmaceutically acceptable salt, carrier, diluent, or excipient therefore, or a combination thereof, for the treatment or prevention of a cancer, or an autoimmune disease, or an infectious disease.

14. The pharmaceutical composition for use of claim 13, wherein the pharmaceutical composition is administered concurrently with another therapeutic agent such as a chemotherapeutic agent, radiation therapy, an immunotherapy agent, an autoimmune disorder agent, an anti-infectious agent or the other conjugates for synergistically effective treatment or prevention of a cancer, or an autoimmune disease, or an infectious disease.

15. The pharmaceutical composition for use according to claim 14, wherein the therapeutic agents are selected from one or several of the following drugs: Abatacept, Abiraterone acetate, Acetaminophen /hydrocodone, Adalimumab, afatinib dimaleate, alemtuzumab, Alitretinoin, ado-trastuzumab emtansine, Amphetamine mixed salts (Amphetamine/dextroamphetamine,), anastrozole, Aripiprazole, Atazanavir, Atezolizumab, Atorvastatin, axitinib, belinostat, Bevacizumab, Cabazitaxel, Cabozantinib, bexarotene, blinatumomab, Bortezomib, bosutinib, brentuximab vedotin, Budesonide, Budesonide/ formoterol, Buprenorphine, Capecitabine, carfilzomib, Celecoxib, ceritinib, Cetuximab, Ciclosporin, Cinacalcet, crizotinib, Dabigatran, dabrafenib, Darbepoetin alfa, Darunavir, imatinib mesylate, dasatinib, denileukin diftitox, Denosumab, Depakote, Dexlansoprazole, Dexmethylphenidate, Dinutuximab, Doxycycline, Duloxetine, Emtricitabine/Rilpivirine/ Tenofovir disoproxil fumarate, Emtricitabine /tenofovir/efavirenz, Enoxaparin, Enzalutamide, Epoetin alfa, erlotinib, Esomeprazole, Eszopiclone, Etanercept, Everolimus, exemestane, everolimus, Ezetimibe, Ezetimibe/simvastatin, Fenofibrate, Filgrastim, fingolimod, Fluticasone propionate, Fluticasone/salmeterol, fulvestrant, gefitinib, Glatiramer, Goserelin acetate, Imatinib, Ibritumomab tiuxetan, ibrutinib, idelalisib, Infliximab, Insulin aspart, Insulin detemir, Insulin glargine, Insulin lispro, Interferon beta 1a, Interferon beta 1b, lapatinib, Ipilimumab, Ipratropium bromide/salbutamol, Lanreotide acetate, lenaliomide, lenvatinib mesylate, letrozole, Levothyroxine, Levothyroxine, Lidocaine, Linezolid, Liraglutide, Lisdexamfetamine, MEDI4736, Memantine, Methylphenidate, Metoprolol, Modafinil, Mometasone, Nilotinib, Nivolumab, ofatumumab, obinutuzumab, olaparib, Olmesartan, Olmesartan/hydrochlorothiazide, Omalizumab, Omega-3 fatty acid ethyl esters, Oseltamivir, Oxycodone, palbociclib, Palivizumab, panitumumab, panobinostat, pazopanib, pembrolizumab, Pemetrexed, pertuzumab, Pneumococcal conjugate vaccine, pomalidomide, Pregabalin, Quetiapine, Rabeprazole, radium 223 chloride, Raloxifene, Raltegravir, ramucirumab, Ranibizumab, regorafenib, Rituximab, Rivaroxaban, romidepsin, Rosuvastatin, ruxolitinib phosphate, Salbutamol, Sevelamer, Sildenafil, siltuximab, Sitagliptin, Sitagliptin/metformin, Solifenacin, Sorafenib, Sunitinib, Tadalafil, tamoxifen, Telaprevir, temsirolimus, Tenofovir/emtricitabine, Testosterone gel, Thalidomide (Immunoprin, Talidex), Tiotropium bromide, toremifene, trametinib, Trastuzumab, Tretinoin, Ustekinumab, Valsartan, vandetanib, vemurafenib, vorinostat, ziv-aflibercept, Zostavax., and their pharmaceutically acceptable salts, carriers, diluents, or excipients therefore, or a combination above thereof.

## Patentansprüche

1. Produkt, das:
(A) ein Zellbindungsmoleküllinker-Wirkstoffkonjugat ist, dargestellt als 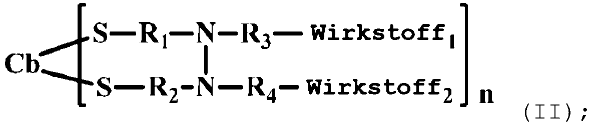 oder
(B) eine Verbindung der Formel (IV) ist 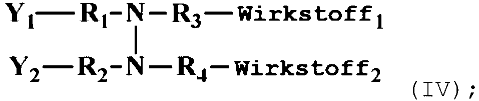 wobei:
Y₁ und Y₂
aus den unten dargestellten Strukturen ausgewählt sind: 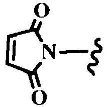 Maleimid; 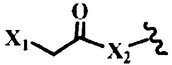 Haloacetyl; und 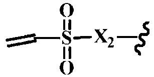 Ethensulfonyl; wobei X₁ F, Cl, Br, I oder Lv ist; X₂ O, NH, N(R₁) oder CH₂ ist; Lv ausgewählt ist aus Nitrophenol; N-Hydroxysuccinimid (NHS); Phenol; Dinitrophenol; Pentafluorphenol; Tetrafluorphenol; Difluorphenol; Monofluorphenol; Pentachlorphenol; Triflat; Imidazol; Dichlorphenol; Tetrachlorphenol; 1-Hydroxybenzo-triazol; Tosylat; Mesylat; 2-Ethyl-5-phenylisoxazolium-3'-sulfonat oder Anhydriden; oder einem Zwischenmolekül, das mit einem Kondensationsreagenz für Peptidkopplungsreaktionen oder für Mitsobonu-Reaktionen erzeugt wird,
R₁, R₂, R₃ und R₄ gleich oder unterschiedlich sind und fehlen, lineares Alkyl mit 1 bis 8 Kohlenstoffatomen, verzweigtes oder zyklisches Alkyl mit 3 bis 8 Kohlenstoffatomen, lineares, verzweigtes oder zyklisches Alkenyl oder Alkinyl oder 1~8 Kohlenstoffatome von Estern, Ether-, Amid- oder Polyethylenoxy-Einheiten der Formel (OCH₂CH₂)ₚ, wobei p eine ganze Zahl von 0 bis 1000 ist, oder eine Kombination davon; und
zusätzlich R₁, R₂, R₃ und R₄ jeweils eine Kette von Atomen, ausgewählt aus C, N, O, S, Si und P, sind, die kovalent mit Y₁ oder Y₂ und Z₁ oder Z₂ verbunden ist, wobei die Atome, die zum Bilden von R₁, R₂, R₃ und R₄ verwendet werden, auf alle chemisch relevanten Arten kombiniert werden können;
Cb für ein zellbindendes Mittel steht;
Wirkstoff₁ und Wirkstoff₂ für gleiche oder unterschiedliche Wirkstoffe/zytotoxische Wirkstoffe stehen, die über den Brückenlinker durch eine Alkylen-, Alkenylen-, Alkinylen-, Ether-, Polyoxyalkylen-, Ester-, Amin-, Imin-, Polyamin-, Hydrazin-, Hydrazon-, Amid-, Harnstoff-, Semicarbazid-, Carbazid-, Alkoxyamin-, Urethan-, Aminosäure-, Peptid-, Acyloxylamin-, Hydroxamsäure-, Disulfid-, Thioether-, Thioester-, Carbamat-, Carbonat-, heterozyklische Ring-, heteroaromatische oder Alkoxim-Bindung an das zellbindende Mittel gebunden sind; und
n eine ganze Zahl von 1 bis 20 ist.

2. Produkt nach Anspruch 1, wobei "Wirkstoff₁" und "Wirkstoff₂" ausgewählt sind aus Tubulysinen, Calicheamicinen, Auristatinen, Maytansinoiden, CC-1065-Analoga, Daunorubicin- und Doxorubicin-Verbindungen, Taxanoiden (Taxanen), Cryptophycinen, Epothilonen, Benzodiazepin-Dimeren (z. B. Dimere von Pyrrolobenzodiazepin (PBD), Tomaymycin, Anthramycin, Indolinobenzodiazepinen, Imidazobenzothiadiazepinen oder Oxazolidinobenzodiazepinen), Calicheamicine und die Enediyne-Antibiotika, Actinomycin, Azaserine, Bleomycine, Epirubicin, Tamoxifen, Idarubicin, Dolastatine/Auristatine (z. B. Monomethyl-Auristatin E, MMAE, MMAF, Auristatin PYE, Auristatin TP, Auristatine 2-AQ, 6-AQ, EB (AEB) und EFP (AEFP)), Duocarmycine, Thiotepa, Vincristin, Hemiasterine, Nazumamide, Mikroginine, Radiosumine, Alterobactine, Mikrosklerodermine, Theonellamide, Esperamicine, siRNA, nukleolytischen Enzymen und/oder pharmazeutisch annehmbaren Salzen und/oder Säuren eines der oben genannten Moleküle.

3. Produkt nach Anspruch 1, wobei das zellbindende Mittel/Molekül ausgewählt ist aus einem Antikörper, einem Protein, einem Vitamin (z. B. Folat), Peptiden, einer polymeren Mizelle, einem Liposom, einem Arzneimittelträger auf Lipoproteinbasis, einem Arzneimittelträger auf Nanopartikelbasis, einem Dendrimer und einem Molekül, das oben mit zellbindenden Liganden beschichtet ist, oder einer Kombination davon.

4. Produkt nach Anspruch 1, wobei das zellbindende Mittel oder die zellbindende Verbindung gegen eine Tumorzelle, eine mit einem Virus infizierte Zelle, eine mit einem Mikroorganismus infizierte Zelle, eine mit einem Parasiten infizierte Zelle, eine Zelle mit einer Autoimmunerkrankung, eine aktivierte Tumorzelle, eine myeloische Zelle, eine aktivierte T-Zelle, eine schädigende B-Zelle oder einen Melanozyten gerichtet werden kann.

5. Produkt nach Anspruch 4, wobei die Tumorzelle ausgewählt ist aus Lymphomzellen, Myelomzellen, Nierenzellen, Brustkrebszellen, Prostatakrebszellen, Eierstockkrebszellen, kolorektalen Krebszellen, Magenkrebszellen, Plattenepithelkarzinomzellen, kleinzelligen Lungenkrebszellen, nicht kleinzelligen Lungenkrebszellen, Hodenkrebszellen oder beliebigen Zellen, die in einem unregulierten, beschleunigten Tempo wachsen und sich teilen, um Krebs zu verursachen.

6. Produkt nach Anspruch 1, wobei die Linkerkomponenten R₁, R₂, R₃ und/oder R₄ aus einer oder mehreren Linkerkomponenten bestehen aus: 6-Maleimidocaproyl (MC), Maleimido-Propanoyl (MP), Valin-Citrullin (Val-Cit), Alanin-Phenylalanin (Ala-Phe), Lysin-Phenylalanin (Lys-Phe), p-Aminobenzyloxycarbonyl (PAB), 4-Thio-Pentanoat (SPP), 4-(N-Maleimidomethyl)-Cyclohexan-1-carboxylat (MCC), 4-Thio-butyrat (SPDB), Maleimidoethyl (ME), 4-Thio-2-hydroxysulfonyl-butyrat (2-Sulfo-SPDB), Pyridinyldithiol (PySS), Alkoxyamino (AOA), Ethylenoxy (EO), 4-Methyl-4-dithio-pentanoic (MPDP), Azido (N₃), Alkynyl, Dithio, Peptid und/oder (4-Acetyl)aminobenzoat (SIAB).

7. Produkt nach Anspruch 1, wobei "Wirkstoff," und "Wirkstoff₂" Folgendes sind:
(A) ein Tubulysin-Analogon;
(B) ein Calicheamicin-Analogon;
(C) ein Maytansinoid-Analogon;
(D) ein Taxan-Analogon;
(E) ein CC-1065-Analogon und/oder ein Doucarmycin-Analogon;
(F) ein Daunorubicin- oder Doxorubicin-Analogon;
(G) ein Auristatin- und Dolastatin-Analogon; oder
(H) ein Benzodiazepin-Dimer-Analogon.

8. Produkt nach Anspruch 1, wobei die konjugierte Verbindung der Formel (II) wie folgt ist:
(A) die Struktur von T01, T02, T03, T04, T05, T06 oder T07 wie folgt: 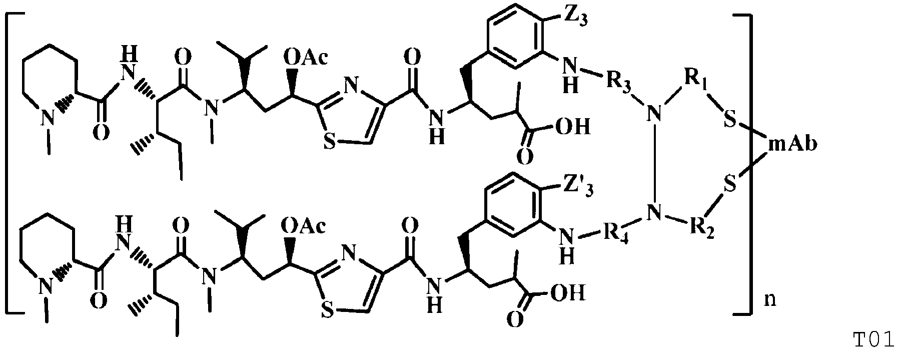 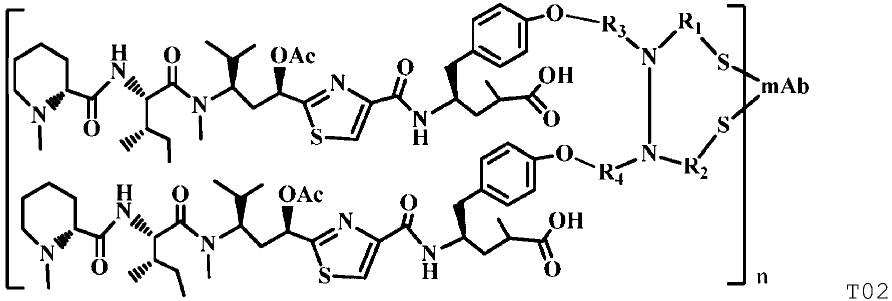 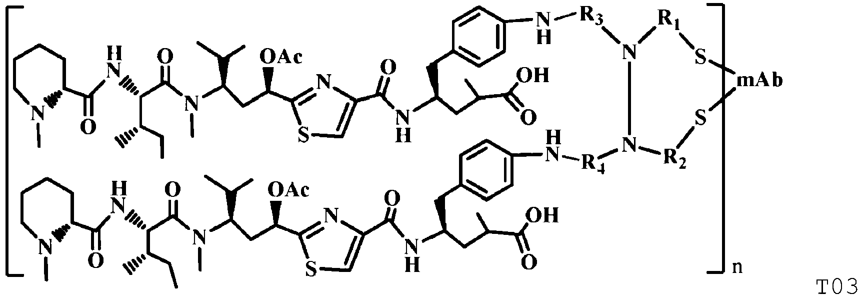 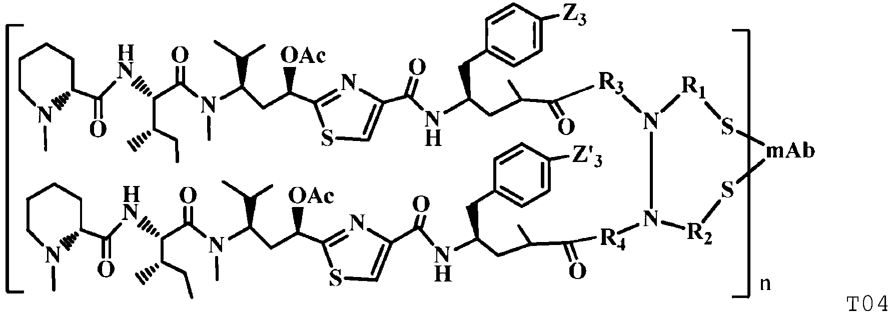 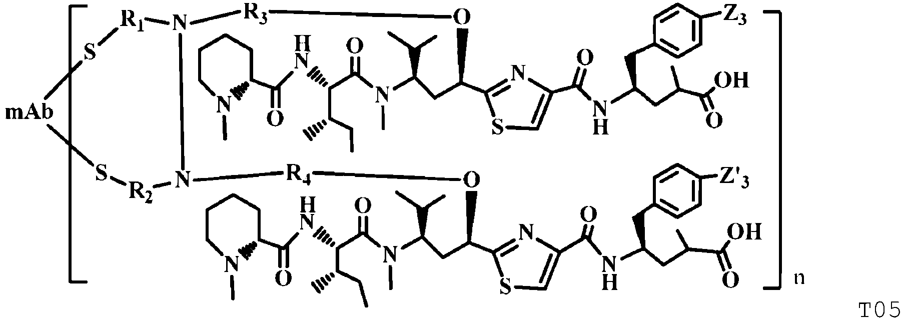 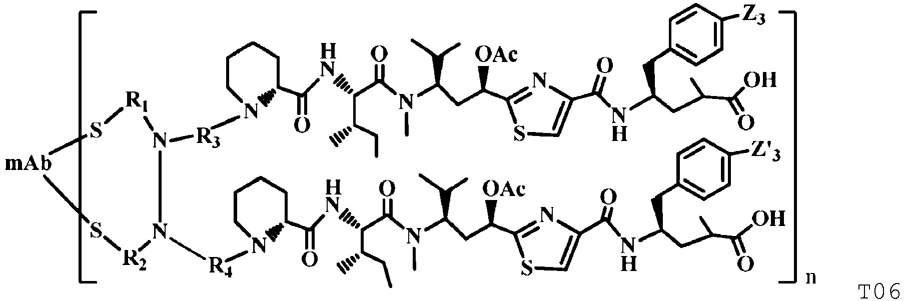 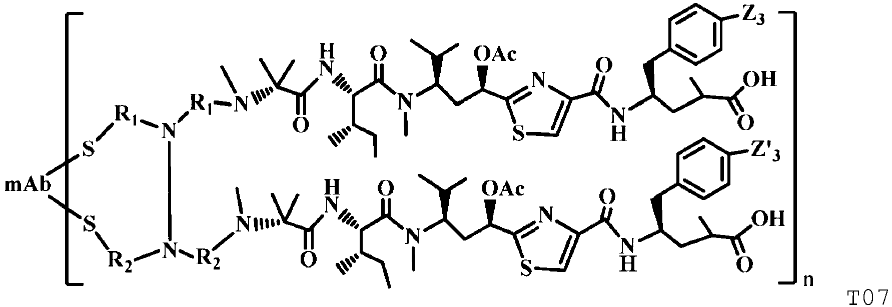 wobei mAb ein Antikörper ist; Z₃ H, R₁, OP(O)(OM₁)(OM₂), OCH₂OP(O)(OM₁)(OM₂), OSO₃M₁ oder O-Glycosid (Glucosid, Galactosid, Mannosid, Glucuronosid, Allosid, Fructosid usw.), NH-Glycosid, S-Glycosid oder CH₂-Glycosid ist; M₁ und M₂ unabhängig voneinander H, Na, K, Ca, Mg, NH₄ oder NR₁R₂R₃R₄ sind; n 1~20 ist; R₁, R₂, R₃ und R₄ die gleichen sind wie in Anspruch **1** definiert ist; oder
(B) die Struktur von C01 wie folgt: 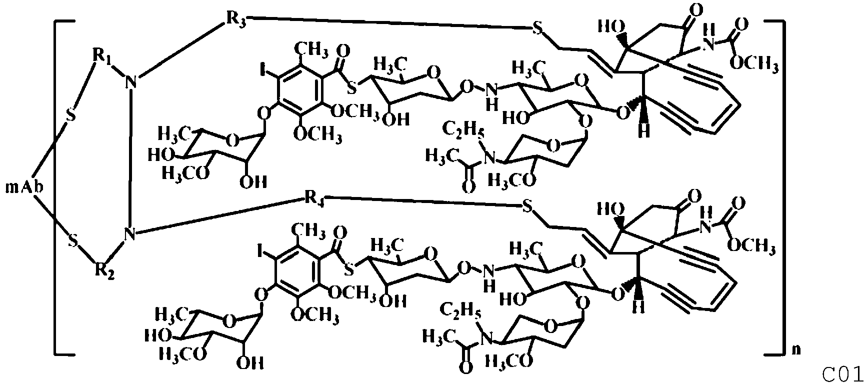 wobei mAb ein Antikörper ist; n 1~20 ist; R₁, R₂, R₃ und R₄ die gleichen sind wie in Anspruch 1 definiert ist; oder
(C) die Struktur von M01 wie folgt: 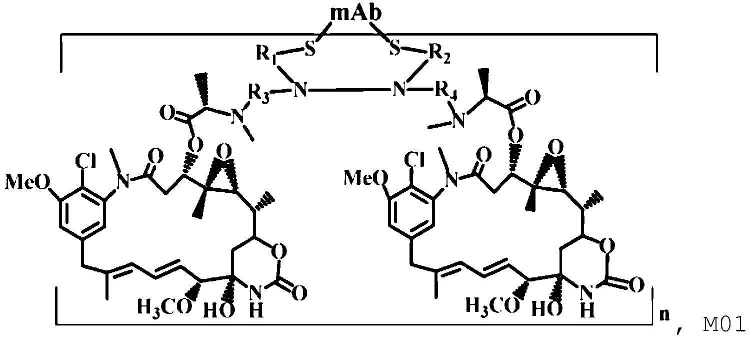 wobei mAb ein Antikörper ist; n 1~20 ist; R₁, R₂, R₃ und R₄ die gleichen sind wie in Anspruch 1 definiert ist; oder
(D) die Struktur von Tx01, Tx02 oder Tx03: 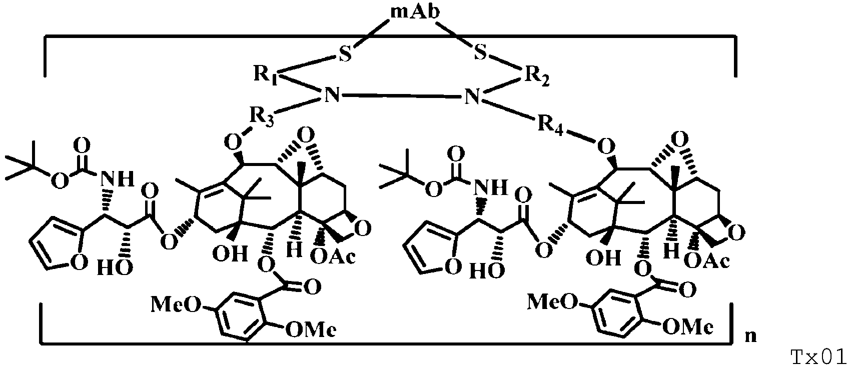 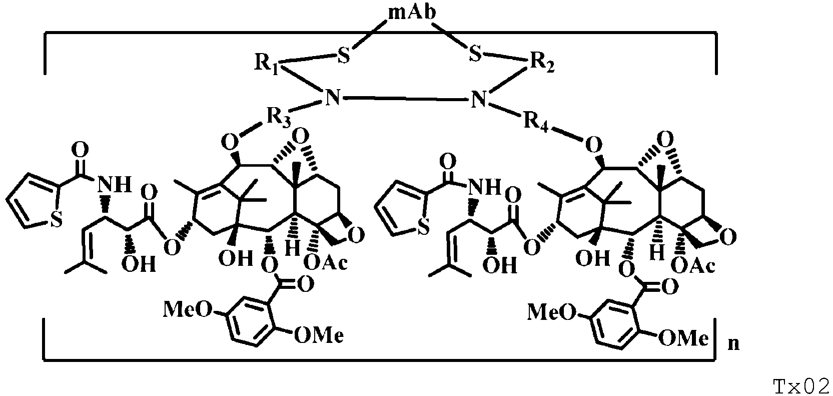 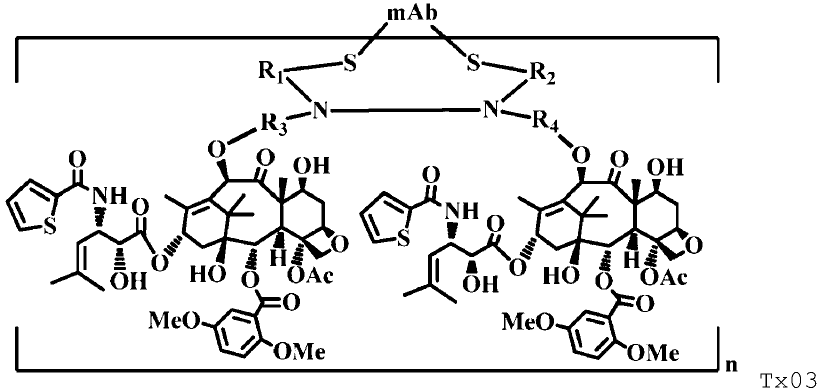 wobei mAb ein Antikörper ist; n 1~20 ist; R₁, R₂, R₃ und R₄ die gleichen sind wie in Anspruch **1** definiert ist; oder
(E) die Struktur von CC01, CC02 und CC03 wie folgt: 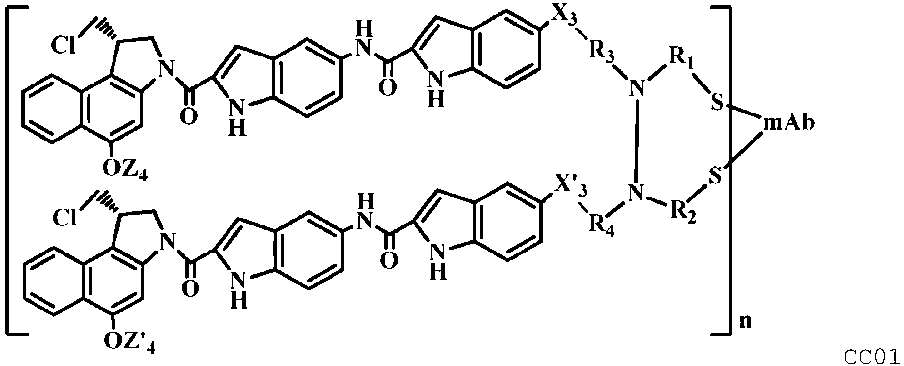 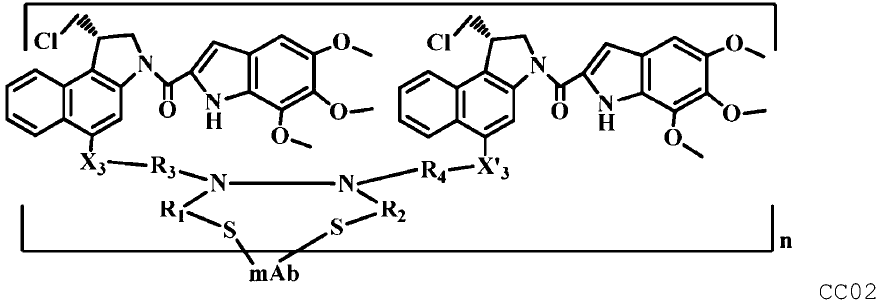 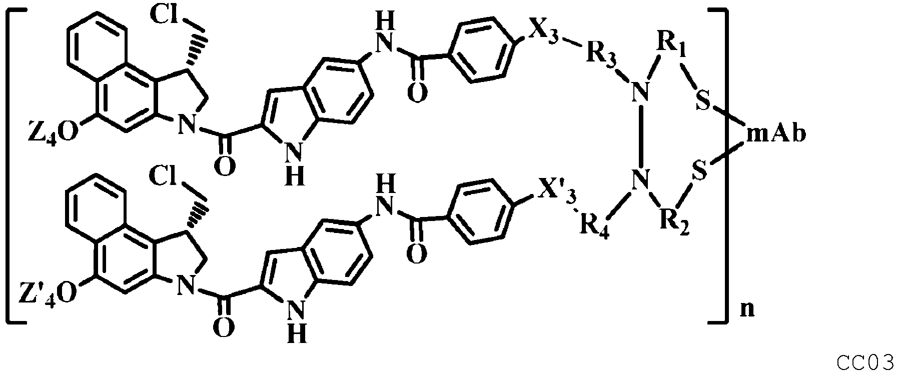 wobei mAb ein Antikörper ist; n 1~20 ist; Z₄ H, PO(OM₁) (OM₂), SO₃M₁, CH₂PO(OM₁)(OM₂), CH₃N(CH₂CH₂)₂NC(O)-, O(CH₂CH₂)₂NC(O)- oder Glycosid ist; X₃ O, NH,NR₁, NHC(O), OC(O), CO, R₁ oder nicht vorhanden ist; M₁ und M₂ unabhängig Na, K, H, NH₄, NR₁R₂R₃R₄ sind; R₁, R₂, R₃ und R₄ die gleichen sind wie in Anspruch **1** definiert ist; oder
(F) die Struktur von Da01, Da02, Da03 oder Da04 wie folgt: 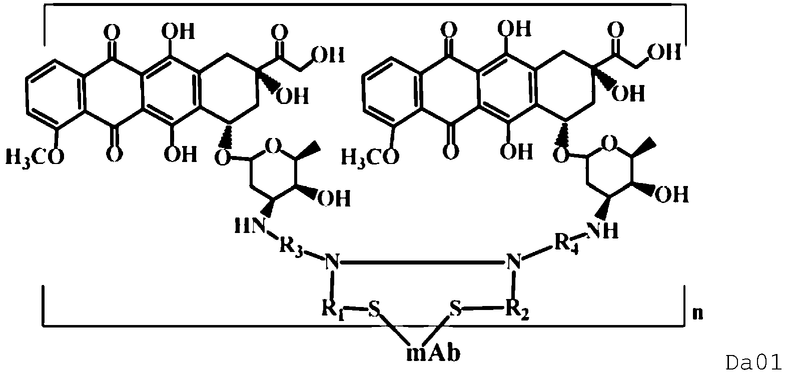 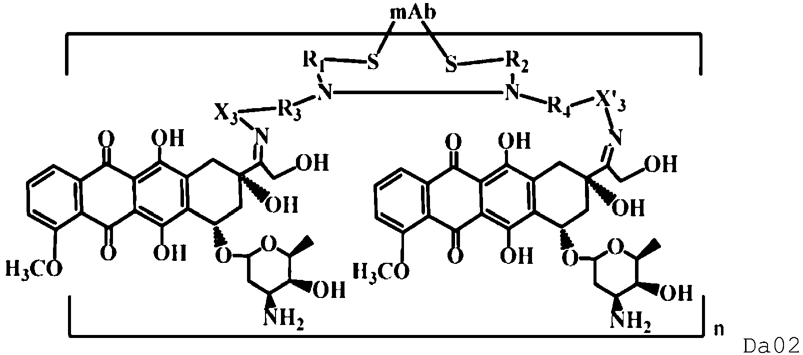 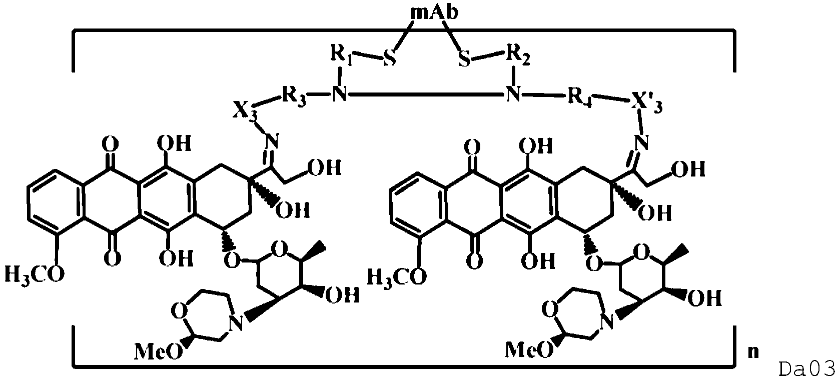 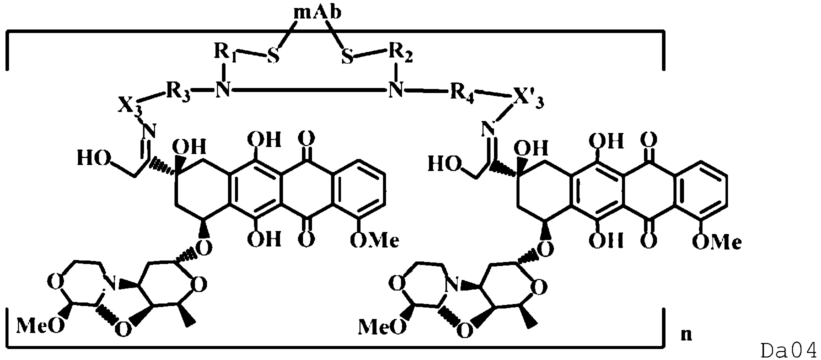 wobei mAb ein Antikörper ist; n 1~20 ist; X₃ O, NH, NR₁, NHC(O), OC(O), CO, R₁ ist oder fehlt; R₁, R₂, R₃ und R₄ die gleichen sind wie in Anspruch **1** definiert ist; oder
(G) die Struktur von Au01, Au02, Au03, Au04 oder Au05 wie folgt: 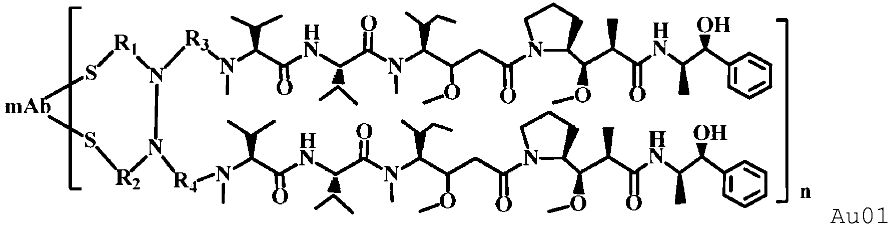 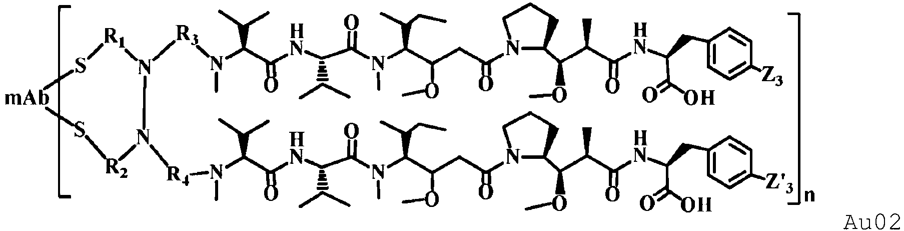 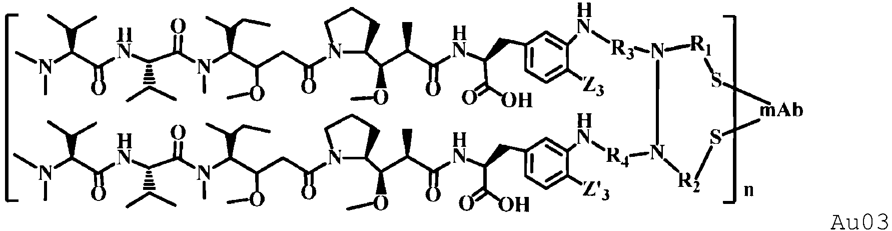 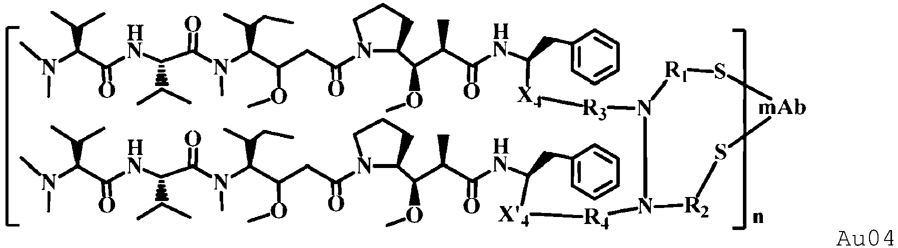 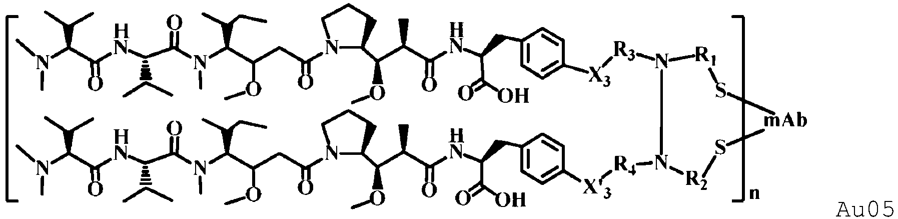 wobei mAb ein Antikörper ist; n 1~20 ist; X₃ CH₂, O, NH, NR₁, NHC(O), NHC(O)NH, C(O), OC(O), R₁ ist oder fehlt; X₄ CH₂, C(O), C(O)NH, C(O)N(R₁), R₁ oder C(O)O ist; R₁, R₂, R₃, und R₄ die gleichen sind wie in Anspruch **1** definiert ist; oder
(H) die Struktur von PB01, PB02, PB03, PB04, PB05, PB06, PB07, PB08 und PB09 wie folgt: 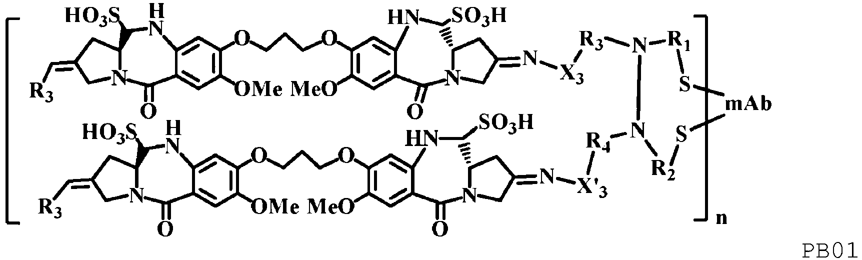 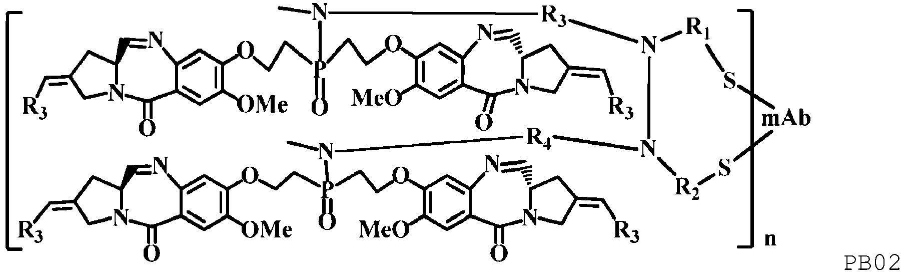 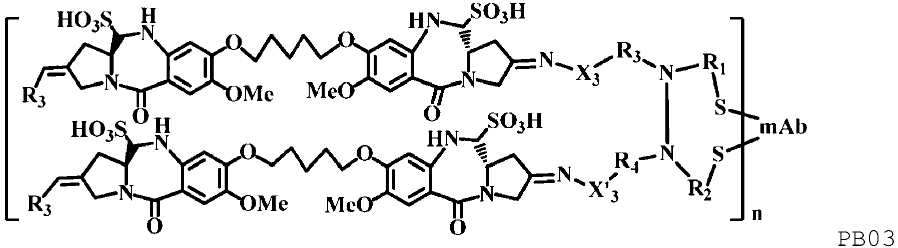 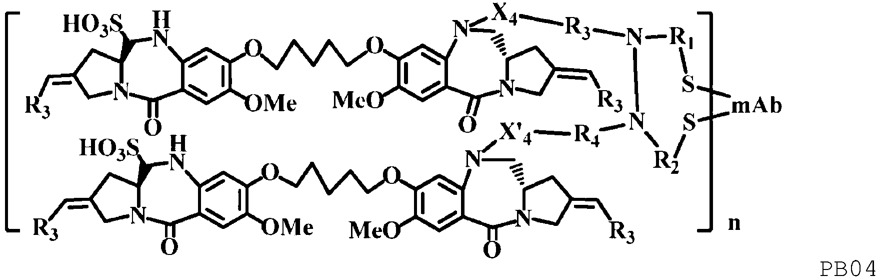 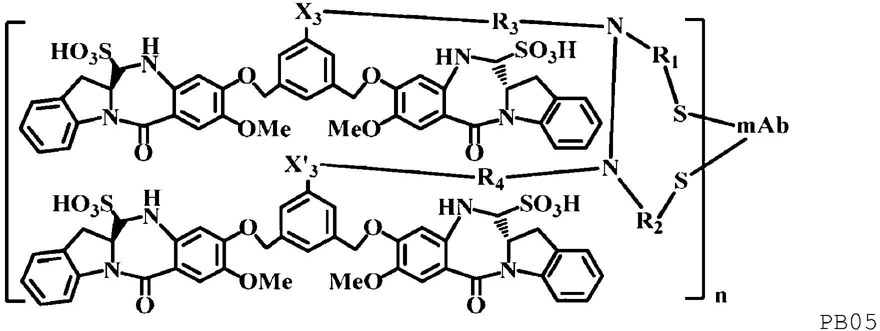 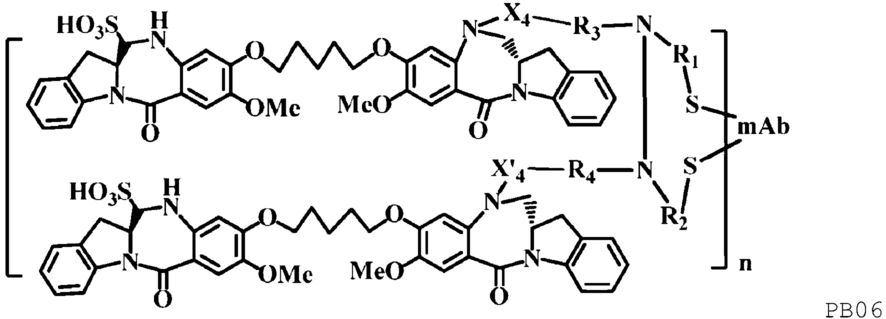 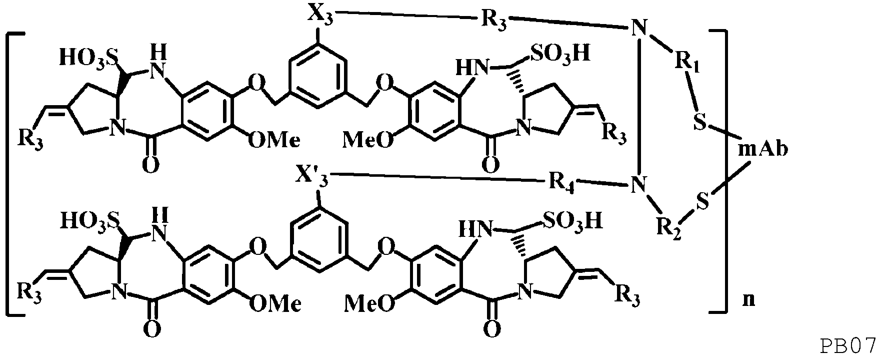 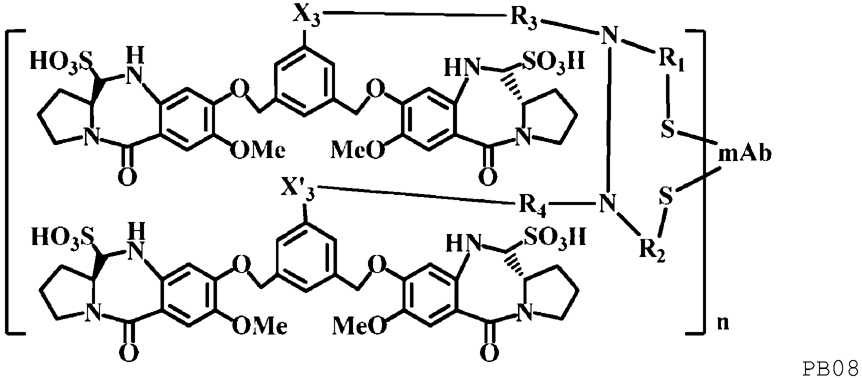 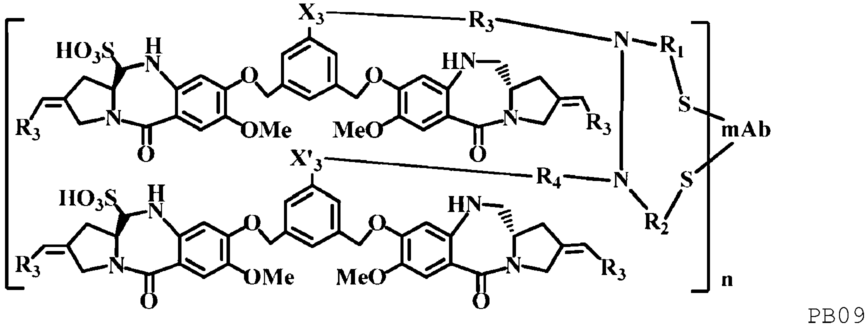 wobei mAb ein Antikörper ist; n 1-20 ist; X₃ CH₂, O, NH, NR₁, NHC(O), NHC(O)NH, C(O), OC(O), R₁ ist oder fehlt; X₄ CH₂, C(O), C(O)NH, C(O)N(R₁), C(O)O, R₁ ist oder fehlt; R₁, R₂, R₃, und R₄ die gleichen sind wie in Anspruch 1 definiert ist; und R₃ und R₄ fehlen können.

9. Produkt nach Anspruch 1 oder 7 oder 8, wobei die Konjugatverbindung entweder ein Peptid aus 1-20 Einheiten natürlicher oder unnatürlicher Aminosäuren oder eine p-Aminobenzyl-Einheit oder eine 6-Maleimidocaproyl-Einheit oder eine Disulfid-Einheit oder eine Thioether-Einheit oder eine Hydrozon-Einheit, eine Triazol-Einheit oder eine Alkoxim-Einheit unter den Linkerkomponenten R₁, R₂, R₃ und/oder R₄ umfasst.

10. Produkt nach Anspruch 1 oder 7 oder 8, wobei die Linkerkomponenten R₁, R₂, R₃ und/oder R₄ durch eine Protease spaltbar ist/sind.

11. Produkt nach Anspruch 1 oder 7 oder 8, wobei die verknüpften Paare von Schwefelatomen aus den Disulfidbindungen zwischen den Ketten eines Antikörpers stammen, die durch TCEP- und/oder DTT-Moleküle reduziert werden, um die Thiolpaare als Konjugationsstellen zu erzeugen.

12. Produkt nach einem der Ansprüche 1 bis 11, wobei R₁, R₂, R₃ und R₄ jeweils eine Kette von Atomen, ausgewählt aus C, N, O, S, Si und P, sind, die kovalent an Y₁ oder Y₂ und Z₁ oder Z₂ gebunden ist, wobei die Atome, die zur Bildung von R₁, R₂, R₃ und R₄ verwendet werden, kombiniert werden können, um Alkylat, Alkylen, Alkenylen und Alkinylen, Ethern, Polyoxyalkylen, Estern, Aminen, Iminen, Polyaminen, Hydrazinen, Hydrazonen, Amiden, Harnstoffen, Semicarbaziden, Carbaziden, Alkoxyaminen, Alkoxylaminen, Urethanen, Aminosäuren, Peptiden, Acyloxylaminen, Hydroxamsäuren oder eine Kombination davon zu bilden.

13. Pharmazeutische Zusammensetzung, umfassend eine therapeutisch wirksame Menge der konjugierten Verbindungen nach Anspruch 1 oder 7 oder 8 und ein pharmazeutisch annehmbares Salz, einen Träger, ein Verdünnungsmittel oder einen Hilfsstoff dafür oder eine Kombination davon, zur Behandlung oder Vorbeugung eines Krebses oder einer Autoimmunerkrankung oder einer Infektionskrankheit.

14. Pharmazeutische Zusammensetzung zur Verwendung nach Anspruch 13, wobei die pharmazeutische Zusammensetzung gleichzeitig mit einem anderen therapeutischen Mittel, wie einem chemotherapeutischen Mittel, einer Strahlentherapie, einem immuntherapeutischen Mittel, einem Mittel gegen Autoimmunerkrankungen, einem antiinfektiösen Mittel oder den anderen Konjugaten zur synergistisch wirksamen Behandlung oder Vorbeugung eines Krebses oder einer Autoimmunerkrankung oder einer Infektionskrankheit verabreicht wird.

15. Pharmazeutische Zusammensetzung zur Verwendung nach Anspruch 14, wobei die therapeutischen Wirkstoffe aus einem oder mehreren der folgenden Arzneimittel ausgewählt sind: Abatacept, Abirateronacetat, Acetaminophen/Hydrocodon, Adalimumab, Afatinibdimaleat, Alemtuzumab, Alitretinoin, Ado-Trastuzumab Emtansin, Amphetamin-Mischsalze (Amphetamin/Dextroamphetamin), Anastrozol, Aripiprazol, Atazanavir, Atezolizumab, Atorvastatin, Axitinib, Belinostat, Bevacizumab, Cabazitaxel, Cabozantinib, Bexaroten, Blinatumomab, Bortezomib, Bosutinib, Brentuximab vedotin, Budesonid, Budesonid/Formoterol, Buprenorphin, Capecitabin, Carfilzomib, Celecoxib, Ceritinib, Cetuximab, Ciclosporin, Cinacalcet, Crizotinib, Dabigatran, Dabrafenib, Darbepoetin alfa, Darunavir, Imatinib-Mesylat, Dasatinib, Denileukin diftitox, Denosumab, Depakote, Dexlansoprazol, Dexmethylphenidat, Dinutuximab, Doxycyclin, Duloxetin, Emtricitabin/Rilpivirin/Tenofovir Disoproxil Fumarat, Emtricitabin/Tenofovir/Efavirenz, Enoxaparin, Enzalutamid, Epoetin alfa, Erlotinib, Esomeprazol, Eszopiclone, Etanercept, Everolimus, Exemestan, Everolimus, Ezetimibe, Ezetimibe/Simvastatin, Fenofibrat, Filgrastim, Fingolimod, Fluticasonpropionat, Fluticason/Salmeterol, Fulvestrant, Gefitinib, Glatiramer, Goserelinacetat, Imatinib, Ibritumomab tiuxetan, Ibrutinib, Idelalisib, Infliximab, Insulin aspart, Insulin detemir, Insulin glargine, Insulin lispro, Interferon beta 1a, Interferon beta 1b, Lapatinib, Ipilimumab, Ipratropiumbromid/Salbutamol, Lanreotidacetat, Lenaliomid, Lenvatinib-Mesylat, Letrozol, Levothyroxin, Levothyroxin, Lidocain, Linezolid, Liraglutid, Lisdexamfetamin, MEDI4736, Memantine, Methylphenidat, Metoprolol, Modafinil, Mometason, Nilotinib, Nivolumab, Ofatumumab, Obinutuzumab, Olaparib, Olmesartan, Olmesartan/Hydrochlorothiazid, Omalizumab, Omega-3-Fettsäureethylester, Oseltamivir, Oxycodon, Palbociclib, Palivizumab, Panitumumab, Panobinostat, Pazopanib, Pembrolizumab, Pemetrexed, Pertuzumab, Pneumokokken-Konjugatimpfstoff, Pomalidomid, Pregabalin, Quetiapin, Rabeprazol, Radiumchlorid 223, Raloxifen, Raltegravir, Ramucirumab, Ranibizumab, Regorafenib, Rituximab, Rivaroxaban, Romidepsin, Rosuvastatin, Ruxolitinib Phosphat, Salbutamol, Sevelamer, Sildenafil, Siltuximab, Sitagliptin, Sitagliptin/Metformin, Solifenacin, Sorafenib, Sunitinib, Tadalafil, Tamoxifen, Telaprevir, Temsirolimus, Tenofovir/Emtricitabin, Testosteron-Gel, Thalidomid (Immunoprin, Talidex), Tiotropiumbromid, Toremifen, Trametinib, Trastuzumab, Tretinoin, Ustekinumab, Valsartan, Vandetanib, Vemurafenib, Vorinostat, Ziv-Aflibercept, Zostavax und ihre pharmazeutisch akzeptablen Salze, Träger, Verdünnungsmittel oder Hilfsstoffe dafür oder eine Kombination davon.

## Revendications

1. Produit qui est :
(A) un conjugué molécule de liaison cellulaire-lieu de pontage-médicament représenté par 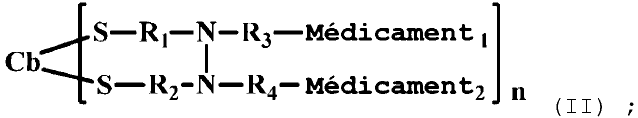 ou
(B) un composé de formule (IV) 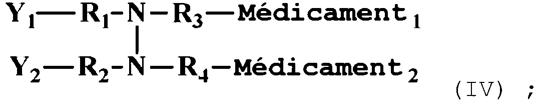 dans lequel :
Y₁ et Y₂ sont
sont choisis parmi les structures présentées ci-dessous : 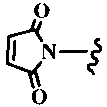 maléimide ; 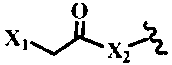 halogénoacétyle ; et 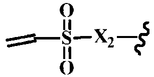 éthènesulfonyle ; dans lequel X₁ est F, Cl, Br, I ou Lv ; X₂ est O, NH, N(R₁), ou CH₂ ; Lv est choisi parmi le nitrophénol ; le N-hydroxysuccinimide (NHS) ; le phénol ; le dinitrophénol ; le pentafluorophénol ; le tétrafluorophénol ; le difluorophénol ; le monofluorophénol ; le pentachlorophénol ; le triflate ; l'imidazole ; le dichlorophénol ; le tétrachlorophénol ; le 1-hydroxybenzotriazole ; le tosylate ; le mésylate ; le 2-éthyl-5-phénylisoxazolium-3'-sulfonate, ou des anhydrides ; ou une molécule intermédiaire générée par un réactif de condensation pour des réactions de couplage peptidique ou de Mitsobonu,
R₁, R₂, R₃ et R₄ sont identiques ou différents et sont absents, alkyle linéaire ayant de 1 à 8 atomes de carbone, alkyle ramifié ou cyclique ayant de 3 à 8 atomes de carbone, alcényle ou alcynyle linéaire, ramifié ou cyclique, ou 1~8 atomes de carbone d'esters, d'éther, d'amide, ou d'unité polyéthylèneoxy de formule (OCH₂CH₂)ₚ, dans lequel p est un entier de 0 à 1000, ou une combinaison de ceux-ci ; et
de plus, R₁, R₂, R₃ et R₄ sont respectivement une chaîne d'atomes choisis parmi C, N, O, S, Si et P, qui se connecte de manière covalente à Y₁ ou Y₂ et Z₁ ou Z₂, dans lequel les atomes utilisés pour former R₁, R₂, R₃ et R₄ peuvent être combinés de toutes les manières chimiquement pertinentes ;
Cb représente un agent de liaison cellulaire ;
Médicament₁ et Médicament₁ représentent des médicaments/agents cytotoxiques identiques ou différents, liés à l'agent de liaison cellulaire via le lieu de pontage par une liaison alkylène, alcénylène, alcynylène, éther, polyoxyalkylène, ester, amine, imine, polyamine, hydrazine, hydrazone, amide, urée, semicarbazide, carbazide, alcoxyamine, uréthanes, acide aminé, peptide, acyloxylamine, acide hydroxamique, disulfure, thioéther, thioester, carbamate, carbonate, cycle hétérocyclique, hétéroaromatique, ou alcoxime ; et
n est un entier compris entre 1 et 20.

2. Produit selon la revendication 1, dans lequel « Médicament₁ » et « Médicament₁ » sont choisis parmi les tubulysines, les calichéamicines, les auristatines, les maytansinoïdes, les analogues de CC-1065, les composés de daunorubicine et de doxorubicine, les taxanoïdes (taxanes), les cryptophycines, les épothilones, les dimères de benzodiazépine (par exemple, les dimères de pyrrolobenzodiazépine (PBD), de tomaymycine, d'anthramycine, d'indolinobenzodiazépines, d'imidazobenzothiadiazépines, ou d'oxazolidinobenzodiazépines), les calichéamicines et les antibiotiques énédiynes, l'actinomycine, les azasérines, les bléomycines, l'épirubicine, le tamoxifène, l'idarubicine, les dolastatines/auristatines (par exemple, la monométhyl auristatine E, le MMAE, le MMAF, l'auristatine PYE, l'auristatine TP, Auristatines 2-AQ, 6-AQ, EB (AEB), et EFP (AEFP)), les duocarmycines, la thiotépa, la vincristine, les hémiasterlines, les nazumamides, les microginines, les radiosumines, les alterobactines, les microsclérodermines, les théonellamides, les espéramycines, un siARN, les enzymes nucléolytiques, et/ou des sels pharmaceutiquement acceptables, et/ou des acides de l'une quelconque des molécules ci-dessus.

3. Produit selon la revendication 1 dans lequel l'agent/molécule de liaison cellulaire est choisi parmi un anticorps, une protéine, une vitamine (par exemple, le folate), des peptides, une micelle polymère, un liposome, un vecteur de médicament à base de lipoprotéine, un vecteur de médicament à nanoparticules, un dendrimère, et une molécule ci-dessus revêtue de ligands de liaison cellulaire, ou une combinaison de ceux-ci.

4. Produit selon la revendication 1, dans lequel l'agent ou le composé de liaison cellulaire est capable de cibler une cellule tumorale, une cellule infectée par un virus, une cellule infectée par un micro-organisme, une cellule infectée par un parasite, une cellule de maladie auto-immune, une cellule tumorale activée, une cellule myéloïde, une cellule T activée, une cellule B affectant, ou un mélanocyte.

5. Produit selon la revendication 4, dans lequel la cellule tumorale est choisie parmi les cellules de lymphome, les cellules de myélome, les cellules rénales, les cellules du cancer du sein, les cellules du cancer de la prostate, les cellules du cancer de l'ovaire, les cellules du cancer colorectal, les cellules du cancer gastrique, les cellules du cancer squameux, les cellules du cancer du poumon à petites cellules, les cellules du cancer du poumon non à petites cellules, les cellules du cancer des testicules, ou toutes cellules qui se développent et se divisent à un rythme accéléré et non régulé pour provoquer des cancers.

6. Produit selon la revendication 1, dans lequel les composants de liaison R₁ R₂, R₃ et/ou R₄ sont composés d'un ou plusieurs composants de liaison parmi : le 6-maléimidocaproyle (MC), le maléimido propanoyle (MP), la valine-citrulline (val-cit), l'alanine-phénylalanine (ala-phe), la lysine-phénylalanine (lys-phe), le p-aminobenzyloxycarbonyle (PAB), le 4-thio-pentanoate (SPP), le 4-(N-maléimidométhyl)-cyclohexane-1-carboxylate (MCC), le 4-thio-butyrate (SPDB), maléimidoéthyle (ME), le 4-thio-2-hydroxysulfonyl-butyrate (2-sulfo-SPDB), le pyridinyldithiol (PySS), l'alcoxy amino (AOA), l'éthylèneoxy (EO), le 4-méthyl-4-dithio-pentanoïque (MPDP), l'azido (N₃), l'alcynyle, le dithio, le peptide, et/ou le (4-acétyl)aminobenzoate (SIAB).

7. Produit selon la revendication 1, dans lequel « Médicament₁ » et « Médicament₂ » sont :
(A) un analogue de la tubulysine ;
(B) un analogue de la calichéamicine ;
(C) un analogue du maytansinoïde ;
(D) un analogue du taxane ;
(E) un analogue du CC-1065 et/ou un analogue de la doucarmycine ;
(F) un analogue de la daunorubicine ou de la doxorubicine ;
(G) un analogue d'auristatine et de dolastatine ; ou
(H) un analogue du dimère de benzodiazépine.

8. Produit selon la revendication 1, dans lequel le composé conjugué de formule (II) est :
(A) la structure de T01, T02, T03, T04, T05, T06 ou T07 comme suit : 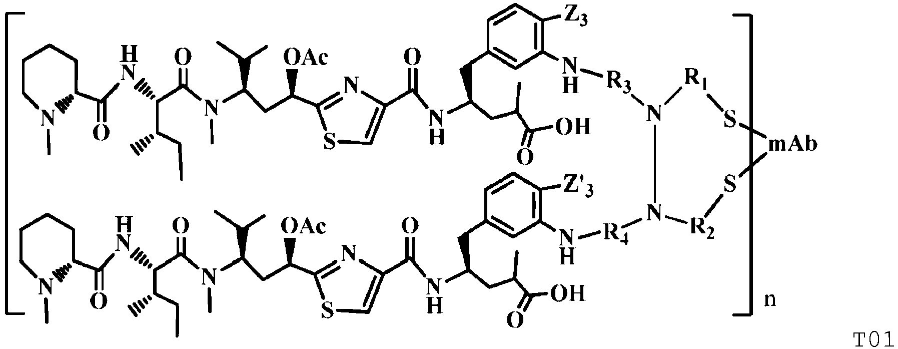 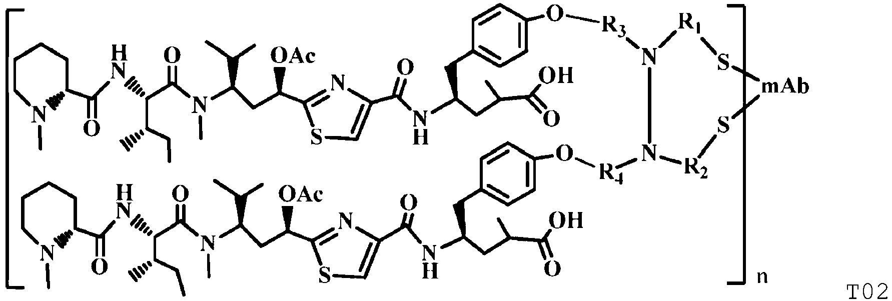 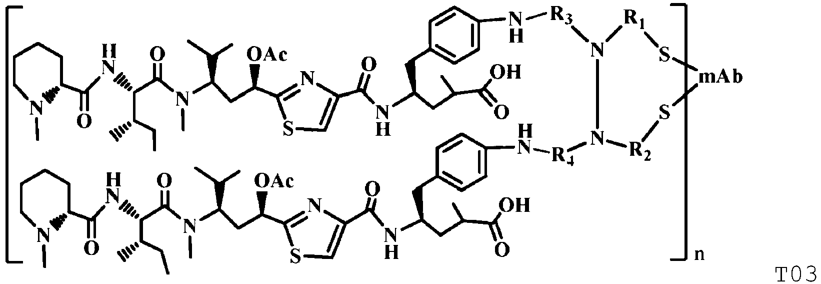 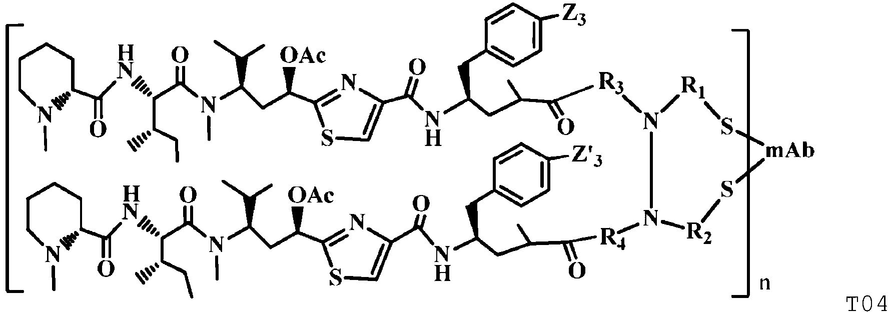 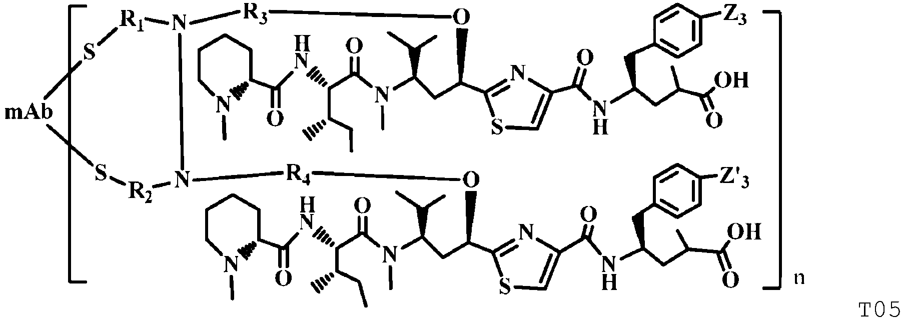 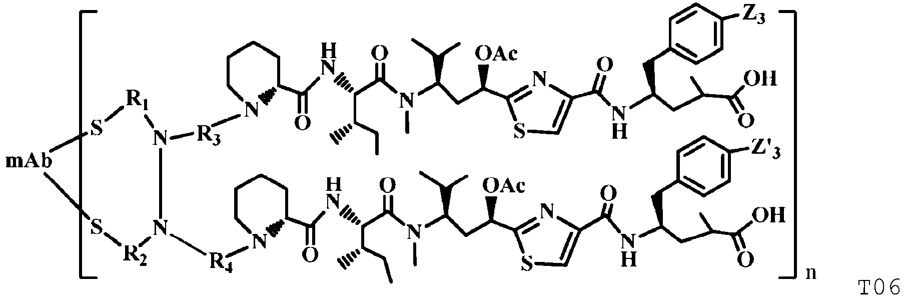 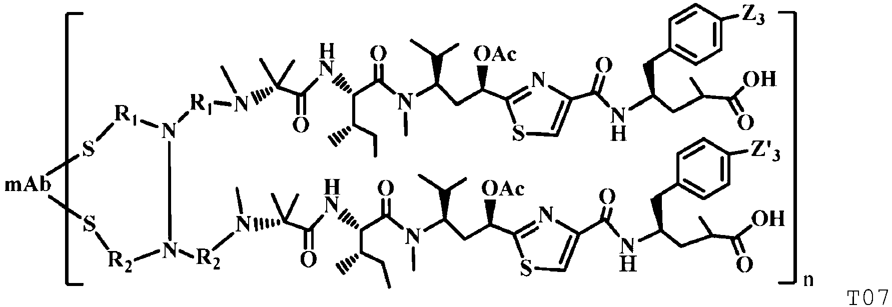 dans lequel mAb est un anticorps ; Z₃ est H, R₁, OP(O)(OM₁)(OM₂), OCH₂OP(O)(OM₁)(OM₂), OSO₃M₁, ou O-glycoside (glucoside, galactoside, mannoside, glucuronoside, alloside, fructoside, etc.), NH-glycoside, S-glycoside, ou CH₂-glycoside ; M₁ et M₂ sont indépendamment H, Na, K, Ca, Mg, NH₄, ou NR₁R₂R₃R₄ ; n est 1~20 ; R₁, R₂, R₃ et R₄ sont tels que définis dans la revendication **1 ;** ou
(B) la structure de C01 comme suit : 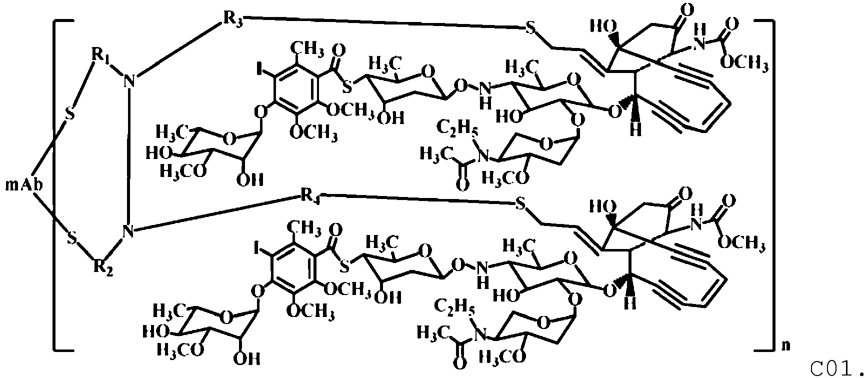 dans lequel mAb est un anticorps ; n est 1~20 ; R₁, R₂, R₃ et R₄ sont tels que définis dans la revendication **1** ; ou
(C) la structure de M01 comme suit : 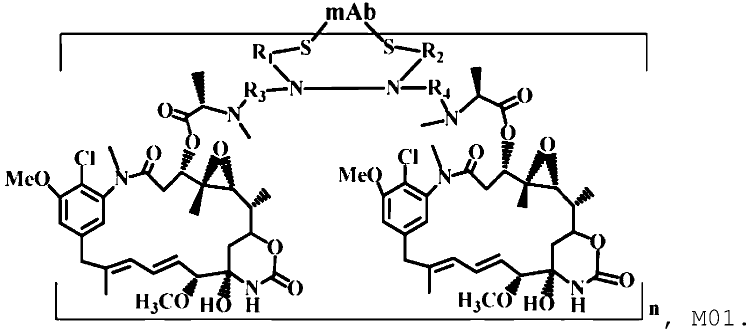 dans lequel mAb est un anticorps ; n est 1~20 ; R₁, R₂, R₃ et R₄ sont tels que définis dans la revendication **1** ; ou
(D) la structure de Tx01, Tx02 ou Tx03 : 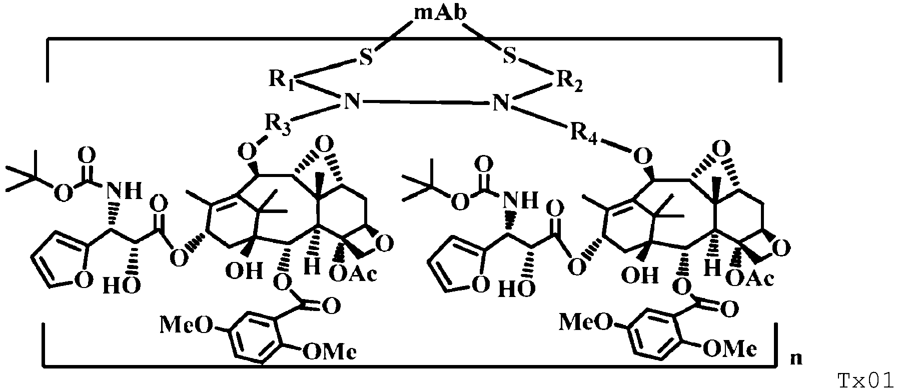 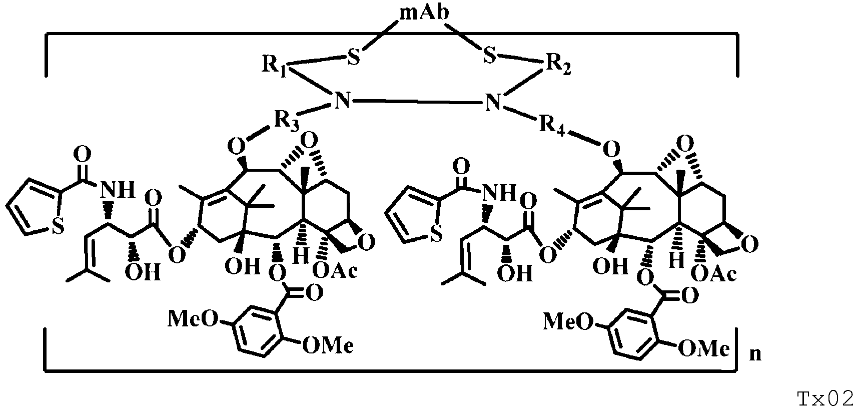 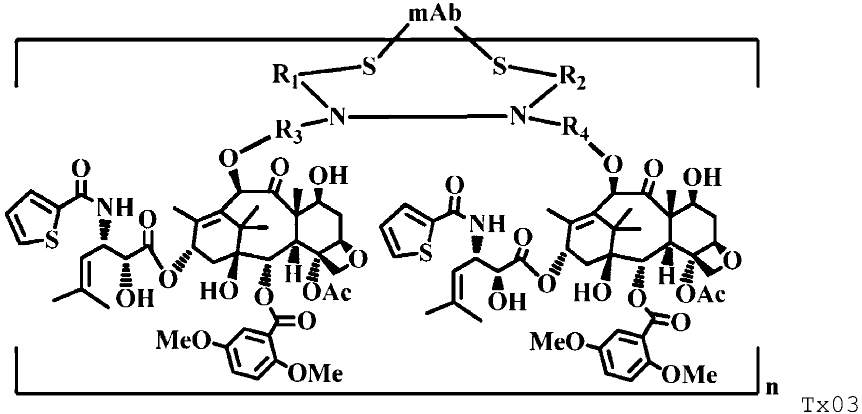 dans lequel mAb est un anticorps ; n est 1~20 ; R₁, R₂, R₃ et R₄ sont tels que définis dans la revendication **1** ; ou
(E) la structure de CC01, CC02, et CC03 comme suit : 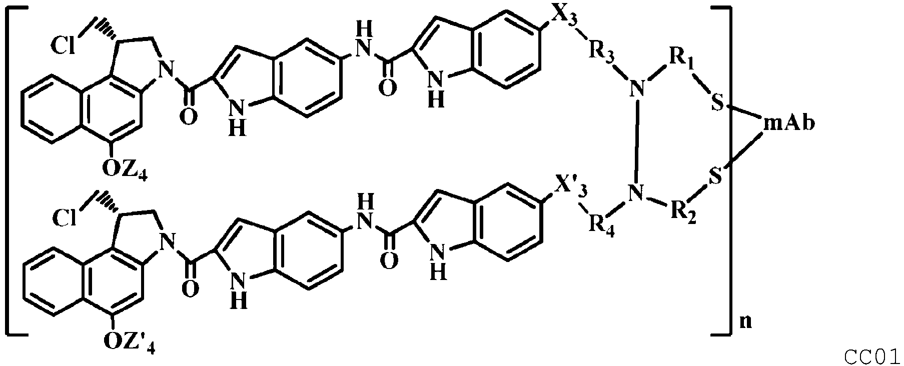 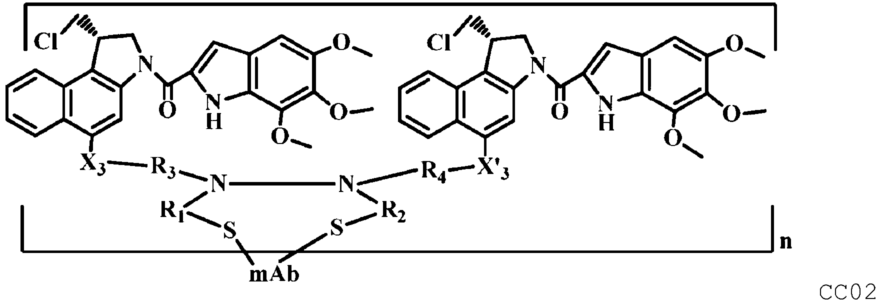 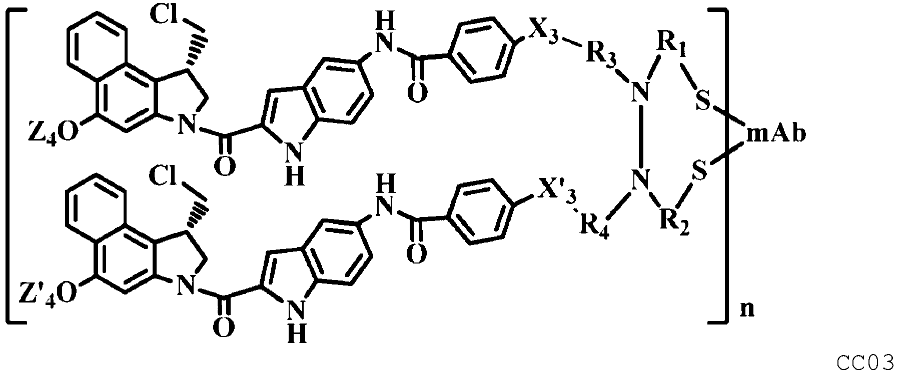 dans lequel mAb est un anticorps ; n est 1~20 ; Z₄ est H, PO(OM₁)(OM₂), SO₃M₁, CH₂PO(OM₁)(OM₂), CH₃N(CH₂CH₂)₂NC(O)-, O(CH₂CH₂)₂NC(O)-, ou un glycoside ; X₃ est O, NH, NR₁, NHC(O), OC(O), CO, R₁, ou absent ; M₁ et M₂ sont indépendamment Na, K, H, NH₄, NR₁R₂R₃R₄ ; R₁, R₂, R₃, et R₄ sont tels que définis dans la revendication **1** ; ou
(F) la structure de Da01, Da02, Da03 ou Da04 comme suit : 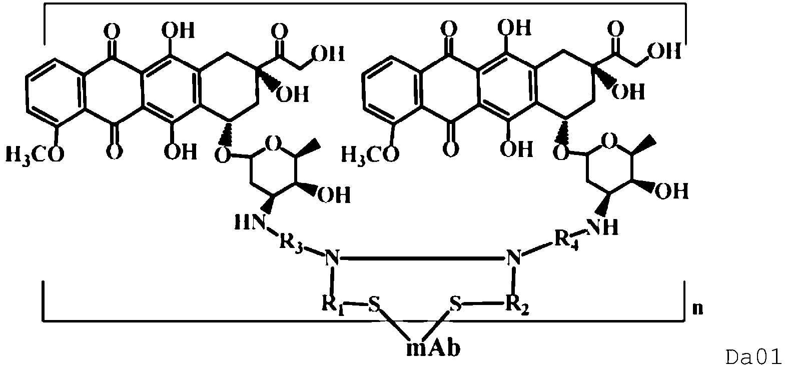 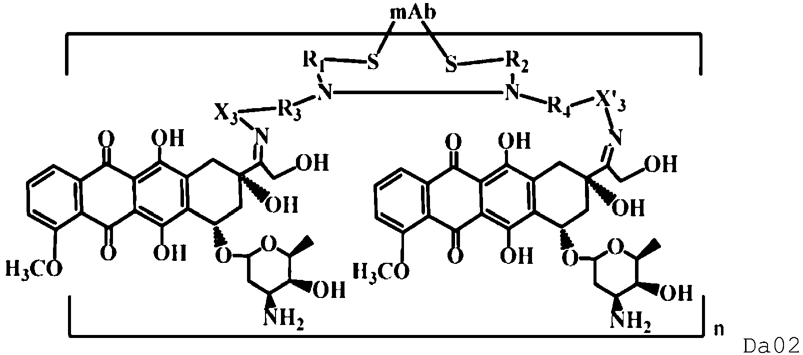 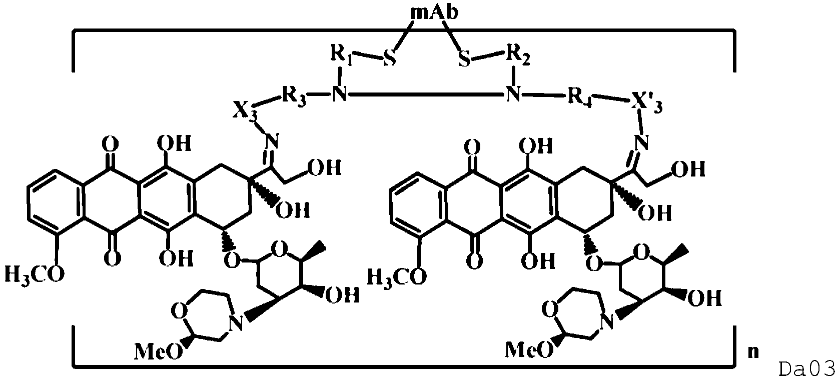 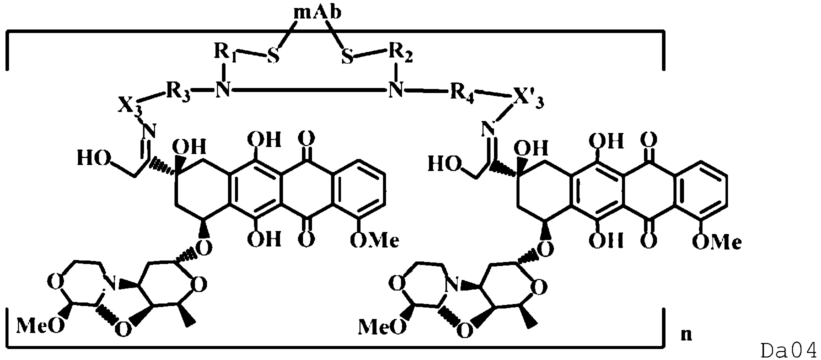 dans lequel mAb est un anticorps ; n est 1~20 ; X₃ est O, NH, NR₁, NHC(O), OC(O), CO, R₁, ou absent ; R₁, R₂, R₃, et R₄ sont tels que définis dans la revendication **1** ; ou
(G) la structure de Au01, Au02, Au03, Au04, ou Au05 comme suit : 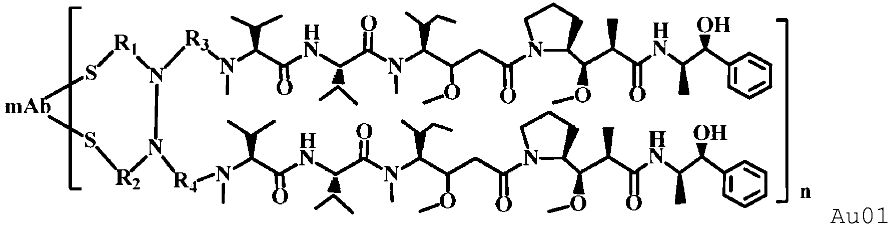 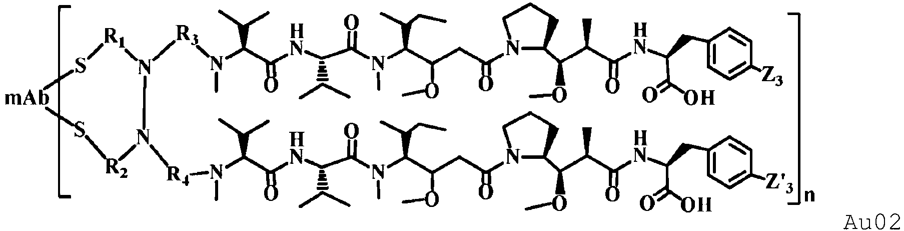 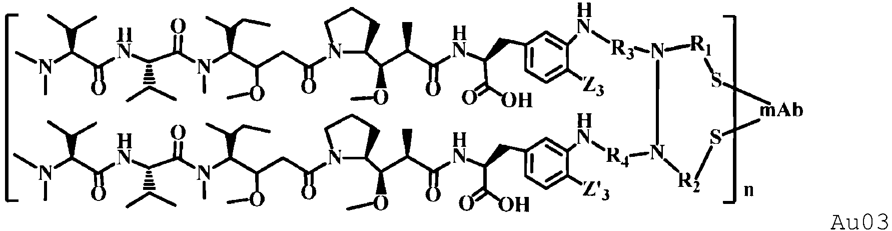 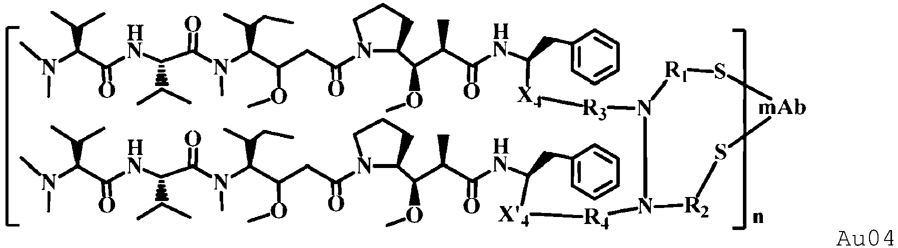 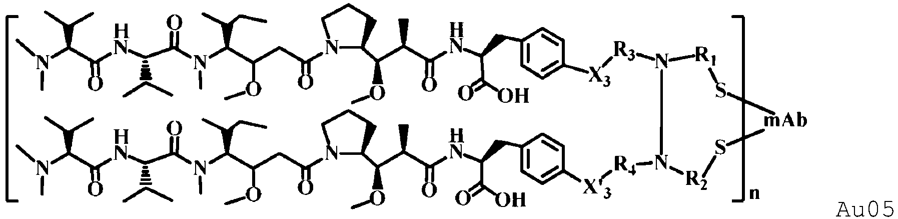 dans lequel mAb est un anticorps ; n est 1~20 ; X₃ est CH₂, O, NH, NR₁, NHC(O), NHC(O)NH, C(O), OC(O), R₁, ou absent ; X₄ est CH₂, C(O), C(O)NH, C(O)N(R₁), R₁, ou C(O)O ; R₁, R₂, R₃, et R₄ sont tels que définis dans la revendication **1** ; ou
(H) la structure de PB01, PB02, PB03, PB04, PB05, PB06, PB07, PB08 et PB09 comme suit : 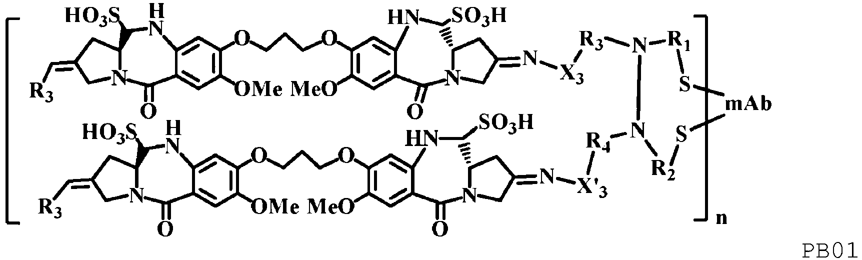 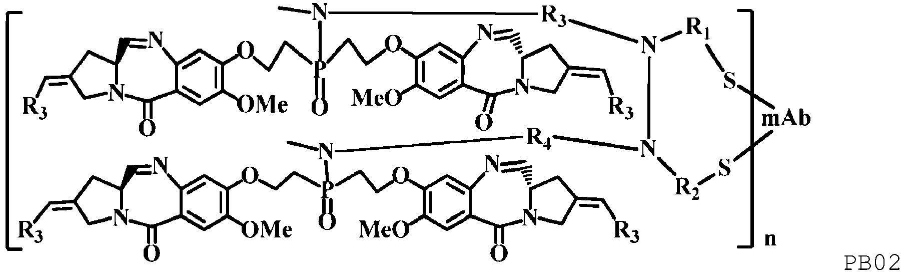 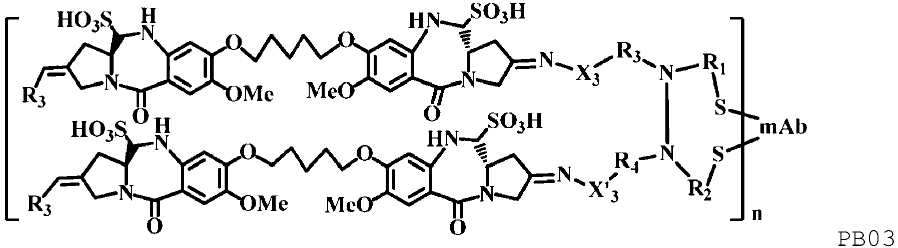 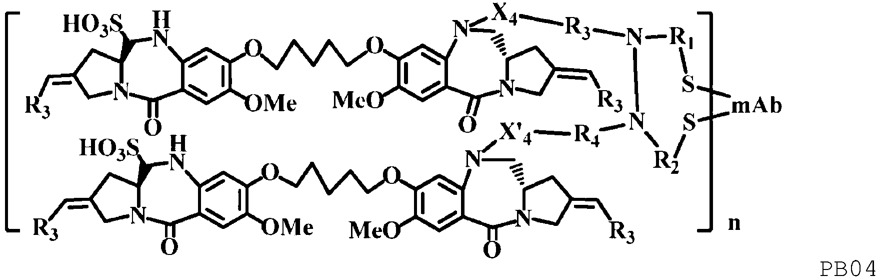 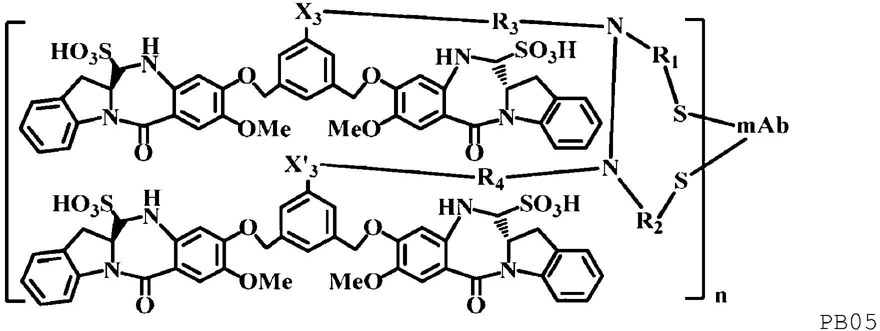 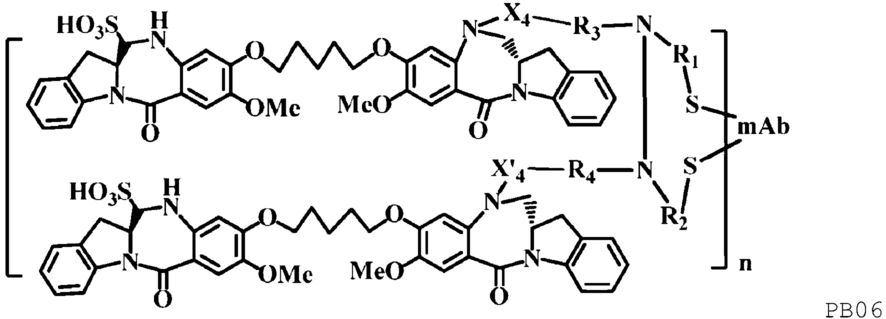 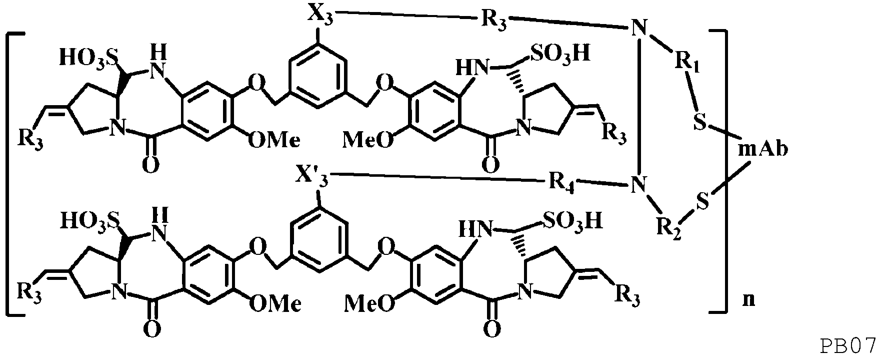 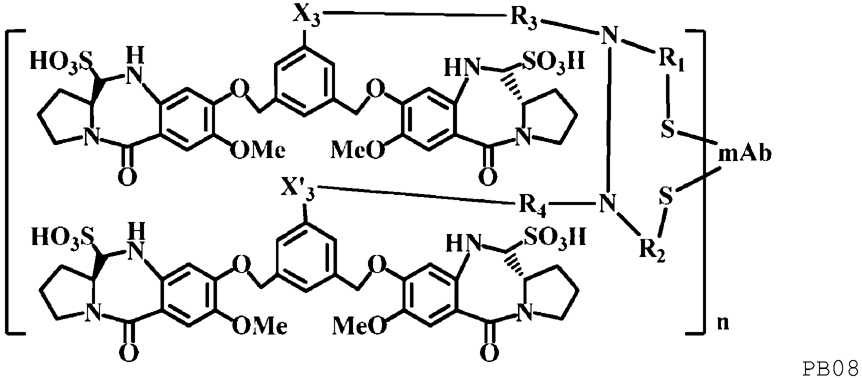 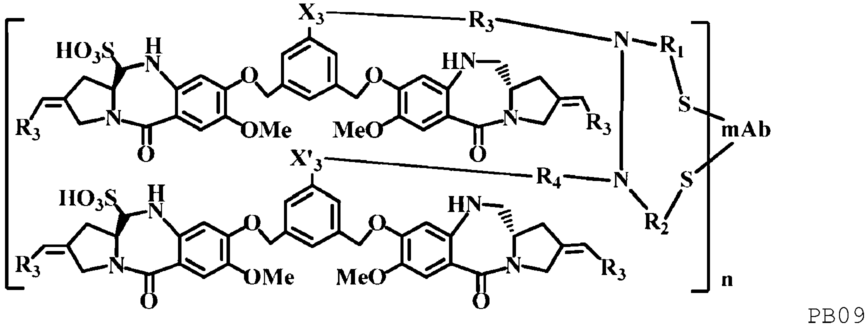 dans lequel mAb est un anticorps ; n est 1~20 ; X₃ est CH₂, O, NH, NR₁, NHC(O), NHC(O)NH, C(O), OC(O), R₁, ou absent ; X₄ est CH₂, C(O), C(O)NH, C(O)N(R₁), C(O)O, R₁, ou absent ; R₁, R₂, R₃, et R₄ sont tels que définis dans la revendication 1 ; et R₃ et R₄ peuvent être absents.

9. Produit selon la revendication 1 ou 7 ou 8, dans lequel le composé conjugué comprend soit un peptide de 1~20 unités d'acides aminés naturels ou non naturels, soit une unité p-aminobenzyle, soit une unité 6-maléimidocaproyle, soit une unité disulfure, soit une unité thioéther, soit une unité hydrozone, une unité triazole, soit une unité alcoxime, parmi les composants de liaison R₁, R₂, R₃ et/ou R₄.

10. Produit selon la revendication 1 ou 7 ou 8, dans lequel les composants de liaison R₁, R₂, R₃ et/ou R₄ sont clivables par une protéase.

11. Produit selon la revendication 1 ou 7 ou 8, dans lequel les paires liées d'atomes de soufre proviennent des liaisons disulfure interchaînes d'un anticorps, qui sont réduites par des molécules de TCEP et/ou de DTT pour générer les paires de thiols comme sites de conjugaison.

12. Produit selon l'une quelconque des revendications 1 à 11, dans lequel R₁, R₂, R₃ et R₄ sont respectivement une chaîne d'atomes choisis parmi C, N, O, S, Si, et P, qui se connecte de manière covalente à Y₁ ou Y₂ et Z₁ ou Z₂, dans lequel les atomes utilisés dans la formation de R₁, R₂, R₃ et R₄ peuvent être combinés pour former un alkylate, un alkylène, un alcénylène, et un alcynylène, des éthers, un polyoxyalkylène, des esters, des amines, des imines, des polyamines, des hydrazines, des hydrazones, des amides, des urées, des semicarbazotures, des carbazides, des alcoxyamines, des alcoxylamines, des uréthanes, des acides aminés, des peptides, des acyloxylamines, des acides hydroxamiques, ou une combinaison de ceux-ci.

13. Composition pharmaceutique comprenant une quantité thérapeutiquement efficace des composés conjugués selon la revendication 1 ou 7 ou 8, et un sel, un support, un diluant, ou un excipient pharmaceutiquement acceptable de ceux-ci, ou une combinaison de ceux-ci, pour le traitement ou la prévention d'un cancer, ou d'une maladie auto-immune, ou d'une maladie infectieuse.

14. Composition pharmaceutique pour une utilisation selon la revendication 13, dans laquelle la composition pharmaceutique est administrée simultanément avec un autre agent thérapeutique tel qu'un agent chimiothérapeutique, une radiothérapie, un agent d'immunothérapie, un agent de maladie auto-immune, un agent anti-infectieux ou les autres conjugués pour un traitement ou une prévention synergiquement efficace d'un cancer, ou d'une maladie auto-immune, ou d'une maladie infectieuse.

15. Composition pharmaceutique pour une utilisation selon la revendication 14, dans laquelle les agents thérapeutiques sont choisis parmi un ou plusieurs des médicaments suivants : abatacept, acétate d'abiratérone, acétaminophène/hydrocodone, adalimumab, dimaléate d'afatinib, alemtuzumab, alitrétinoïne, ado-trastuzumab emtansine, sels mixtes d'amphétamine (amphétamine/dextroamphétamine), anastrozole, aripiprazole, atazanavir, atézolizumab, atorvastatine, axitinib, belinostat, bévacizumab, cabazitaxel, cabozantinib, bexarotène, blinatumomab, bortézomib, bosutinib, brentuximab vedotin, budésonide, budésonide/formotérol, buprénorphine, capécitabine, carfilzomib, célécoxib, céritinib, cétuximab, Ciclosporine, Cinacalcet, crizotinib, Dabigatran, dabrafénib, Darbépoétine alfa, Darunavir, mésylate d'imatinib, dasatinib, dénileukine diftitox, Dénosumab, Dépakote, Dexlansoprazole, Dexméthylphénidate, Dinutuximab, Doxycycline, Duloxétine, Emtricitabine/Rilpivirine/Fumarate de ténofovir disoproxil, Emtricitabine/ténofovir/éfavirenz, Énoxaparine, Enzalutamide, Époétine alfa, erlotinib, Ésoméprazole, Eszopiclone, Étanercept, Évérolimus, exémestane, évérolimus, Ézétimibe, Ézétimibe/simvastatine, Fénofibrate, Filgrastim, fingolimod, Propionate de fluticasone, Fluticasone/salmétérol, fulvestrant, géfitinib, glatiramère, acétate de goséréline, imatinib, ibritumomab tiuxétan, ibrutinib, idelalisib, infliximab, insuline asparte, insuline détémir, insuline glargine, insuline lispro, interféron bêta-1a, interféron bêta-1b, lapatinib, ipilimumab, bromure d'ipratropium/salbutamol, acétate de lanréotide, lénaliomide, mésylate de lenvatinib, létrozole, lévothyroxine, lévothyroxine, lidocaïne, linézolide, liraglutide, lisdexamfétamine, MEDI4736, mémantine, méthylphénidate, métoprolol, modafinil, mométasone, nilotinib, nivolumab, ofatumumab, obinutuzumab, olaparib, Olmésartan, Olmésartan/hydrochlorothiazide, Omalizumab, Esters éthyliques d'acides gras oméga-3, Oseltamivir, Oxycodone, palbociclib, Palivizumab, panitumumab, panobinostat, pazopanib, pembrolizumab, Pemetrexed, pertuzumab, Vaccin conjugué contre le pneumocoque, pomalidomide, Prégabaline, Quétiapine, Rabéprazole, Chlorure de radium 223, Raloxifène, Raltégravir, Ramucirumab, Ranibizumab, Régorafénib, Rituximab, Rivaroxaban, Romidepsine, Rosuvastatine, Phosphate de ruxolitinib, Salbutamol, Sevelamer, Sildénafil, Siltuximab, Sitagliptine, Sitagliptine/metformine, Solifénacine, Sorafénib, Sunitinib, Tadalafil, tamoxifène, télaprévir, temsirolimus, ténofovir/emtricitabine, gel de testostérone, thalidomide (immunoprine, Talidex), bromure de tiotropium, torémifène, trametinib, trastuzumab, trétinoïne, ustékinumab, valsartan, vandétanib, vémurafénib, vorinostat, ziv-aflibercept, Zostavax, et leurs sels, supports, diluants, ou excipients pharmaceutiquement acceptables, ou une combinaison de ceux-ci.
